# EUROPEAN PATENT SPECIFICATION

(11) **EP 1 666 052 B1**
(45) Date of publication and mention of the grant of the patent: **08.06.2011**
(21) Application number: 05025025.7
(22) Date of filing: 28.11.2000
(51) Int. Cl.: A61K 39/395

(54) **Anti-APRIL monoclonal antibody and its use for the treatment of an immune related disease or cancer**
Anti-APRIL monoklonaler Antikörper und deren verwendung zur Behandlung von Immunerkrankungen oder Krebs
Anticorps monoclonal anti-APRIL et son utilisation pour le traitement de maladies immunitaires ou du cancer

(30) Priority: 16.02.2000 US 182938 P; 22.08.2000 US 226986 P
(43) Date of publication of application: 07.06.2006
(62) Divisional of application: 00983773.3
(73) Proprietor: Genentech, Inc., South San Francisco CA 94080-4990 (US)
(72) Inventor: Ashkenazi, Avi, J., San Mateo, CA 94402 (US); Dodge, Kelly, H., San Mateo, CA 94402 (US); Grewal, Iqbal, Fremont, CA 95555 (US); Kim, Kyung, Jin, Cupertino, CA 95014 (US); Marsters, Scot, A., San Carlos, CA 94070 (US); Pitti, Robert, M., El Cerrito, CA 94530 (US); Yan, Minhong, Burlingame, CA 94010 (US)
(74) Representative: Kiddle, Simon John

(56) References cited:
- WO-A-98/39361
- WO-A-99/12965
- P. MOORE ET AL.: "BLyS: member of the tumor necrosis factor family and B cell stimulator." SCIENCE, vol. 285, 9 July 1999 (1999-07-09), pages 260-263, XP002142252 New York, NY, USA
- H. SHU ET AL.: "TALL-1 is a novel member of the TNF family that is down-regulated by mitogens." JOURNAL OF LEUKOCYTE BIOLOGY, vol. 65, May 1999 (1999-05), pages 680-683, XP000939092 New York, NY, USA
- M. HAHNE ET AL.: "APRIL, a new ligand of the tumor necrosis factor family, stimulates tumor cell growth." THE JOURNAL OF EXPERIMENTAL MEDICINE, vol. 188, no. 6, 21 September 1998 (1998-09-21), pages 1185-1190, XP002093077 New York, NY, USA
- A. MUKHOPADHYAY ET AL.: "Identification and characterization of a novel cytokine, THANK, a TNF homologue that activates apoptoss, nuclear factor-kB and c-jun NH2-terminal kinase." THE JOURNAL OF BIOLOGICAL CHEMISTRY, vol. 274, no. 23, 4 June 1999 (1999-06-04), pages 15978-15981, XP002142251 Baltimore, MD, USA
- G. VON BÜLOW ET AL.: "NF-AT activation induced by a CAML-interacting member of the tumor necrosis factor receptor superfamily." SCIENCE, vol. 278, no. 5335, 3 October 1997 (1997-10-03), pages 138-141, XP002165404 New York, NY, USA
- S. MARSTERS ET AL.: "Interaction of the TNF homologues BLyS and APRIL with the TNF receptor homologues BCMA and TACI." CURRENT BIOLOGY, vol. 10, no. 13, 29 June 2000 (2000-06-29), pages 785-788, XP000992744 London, GB
- G. YU ET AL.: "APRIL and TALL-1 and receptors BCMA and TACI: System for regulating humoral immunity." NATURE IMMUNOLOGY, vol. 1, no. 3, September 2000 (2000-09), pages 252-256, XP000992743 New York, NY, USA

### Sequence Listing

<110> GENENTECH, INC.
<120> USES OF AGONISTS AND ANTAGONISTS TO MODULATE ACTIVITY OF TNF-RELATED MOLECULES
<130> P1805R1 PCT
<141> 2000-11-28
<150> US 60/182,938
   <151> 2000-02-16
<150> US 60/226,986
   <151> 2000-08-22
<160> 14
<210> 1
   <211> 1377
   <212> DNA
   <213> Homo sapiens
<900> 1
<210> 2
   <211> 293
   <212> PRT
   <213> Homo sapiens
<400> 2
<210> 3
   <211> 995
   <212> DNA
   <213> Homo sapiens
<400> 3
   aagactcaaa cttagaaact tgaattagat gtggtattca aatccttacg 50
   tgccgcgaag acacagacag cccccgtaag aacccacgaa gcaggcgaag 100
   ttcattgttc tcaacattct agctgctctt gctgcatttg ctctggaatt 150
   cttgtagaga tattacttgt ccttccaggc tgttctttct gtagctccct 200
   tgttttcttt ttgtgatcat gttgcagatg gctgggcagt gctcccaaaa 250
   tgaatatttt gacagtttgt tgcatgcttg cataccttgt caacttcgat 300
   gttcttctaa tactcctcct ctaacatgtc agcgttattg taatgcaagt 350
   gtgaccaatt cagtgaaagg aacgaatgcg attctctgga cctgtttggg 400
   actgagctta ataatttctt tggcagtttt cgtgctaatg tttttgctaa 450
   ggaagataag ctctgaacca ttaaaggacg agtttaaaaa cacaggatca 500
   ggtctcctgg gcatggctaa cattgacctg gaaaagagca ggactggtga 550
   tgaaattatt cttccgagag gcctcgagta cacggtggaa gaatgcacct 600
   gtgaagactg catcaagagc aaaccgaagg tcgactctga ccattgcttt 650
   ccactcccag ctatggagga aggcgcaacc attcttgtca ccacgaaaac 700
   gaatgactat tgcaagagcc tgccagctgc tttgagtgct acggagatag 750
   agaaatcaat ttctgctagg taattaacca tttcgactcg agcagtgcca 800
   ctttaaaaat cttttgtcag aatagatgat gtgtcagatc tctttaggat 850
   gactgtattt ttcagttgcc gatacagctt tttgtcctct aactgtggaa 900
   actctttatg ttagatatat ttctctaggt tactgttggg agcttaatgg 950
   tagaaacttc cttggtttca tgattaaagt cttttttttt cctga 995
<210> 4
   <211> 184
   <212> PRT
   <213> Homo sapiens
<400> 4
<210> 5
   <211> 858
   <212> DNA
   <213> Homo sapiens
<400> 5
   atggatgact ccacagaaag ggagcagtca cgccttactt cttgccttaa 50
   gaaaagagaa gaaatgaaac tgaaggagtg tgtttccatc ctcccacgga 100
   aggaaagccc ctctgtccga tcctccaaag acggaaagct gctggctgca 150
   accttgctgc tggcactgct gtcttgctgc ctcacggtgg tgtctttcta 200
   ccaggtggcc gccctgcaag gggacctggc cagcctccgg gcagagctgc 250
   agggccacca cgcggagaag ctgccagcag gagcaggagc ccccaaggcc 300
   ggcttggagg aagctccagc tgtcaccgcg ggactgaaaa tctttgaacc 350
   accagctcca ggagaaggca actccagtca gaacagcaga aataagcgtg 400
   ccgttcaggg tccagaagaa acagtcactc aagactgctt gcaactgatt 450
   gcagacagtg aaacaccaac tatacaaaaa ggatcttaca catttgttcc 500
   atggcttctc agctttaaaa ggggaagtgc cctagaagaa aaagagaata 550
   aaatattggt caaagaaact ggttactttt ttatatatgg tcaggtttta 600
   tatactgata agacctacgc catgggacat ctaattcaga ggaagaaggt 650
   ccatgtcttt ggggatgaat tgagtctggt gactttgttt cgatgtattc 700
   aaaatatgcc tgaaacacta cccaataatt cctgctattc agctggcatt 750
   gcaaaactgg aagaaggaga tgaactccaa cttgcaatac caagagaaaa 800
   tgcacaaata tcactggatg gagatgtcac attttttggt gcattgaaac 850
   tgctgtga 858
<210> 6
   <211> 285
   <212> PRT
   <213> Homo sapiens
<400> 6
<210> 7
   <211> 1348
   <212> DNA
   <213> Homo sapiens
<400> 7
   ggtacgaggc ttcctagagg gactggaacc taattctcct gaggctgagg 50
   gagggtggag ggtctcaagg caacgctggc cccacgacgg agtgccagga 100
   gcactaacag tacccttagc ttgctttcct cctccctcct ttttattttc 150
   aagttccttt ttatttctcc ttgcgtaaca accttcttcc cttctgcacc 200
   actgcccgta cccttacccg ccccgccacc tccttgctac cccactcttg 250
   aaaccacagc tgttggcagg gtccccagct catgccagcc tcatctcctt 300
   tcttgctagc ccccaaaggg cctccaggca acatgggggg cccagtcaga 350
   gagccggcac tctcagttgc cctctggttg agttgggggg cagctctggg 400
   ggccgtggct tgtgccatgg ctctgctgac ccaacaaaca gagctgcaga 450
   gcctcaggag agaggtgagc cggctgcagg ggacaggagg cccctcccag 500
   aatggggaag ggtatccctg gcagagtctc ccggagcaga gttccgatgc 550
   cctggaagcc tgggagaatg gggagagatc ccggaaaagg agagcagtgc 600
   tcacccaaaa acagaagaag cagcactctg tcctgcacct ggttcccatt 650
   aacgccacct ccaaggatga ctccgatgtg acagaggtga tgtggcaacc 700
   agctcttagg cgtgggagag gcctacaggc ccaaggatat ggtgtccgaa 750
   tccaggatgc tggagtttat ctgctgtata gccaggtcct gtttcaagac 800
   gtgactttca ccatgggtca ggtggtgtct cgagaaggcc aaggaaggca 850
   ggagactcta ttccgatgta taagaagtat gccctcccac ccggaccggg 900
   cctacaacag ctgctatagc gcaggtgtct tccatttaca ccaaggggat 950
   attctgagtg tcataattcc ccgggcaagg gcgaaactta acctctctcc 1000
   acatggaacc ttcctggggt ttgtgaaact gtgattgtgt tataaaaagt 1050
   ggctcccagc ttggaagacc agggtgggta catactggag acagccaaga 1100
   gctgagtata taaaggagag ggaatgtgca ggaacagagg catcttcctg 1150
   ggtttggctc cccgttcctc acttttccct tttcattccc accccctaga 1200
   ctttgatttt acggatatct tgcttctgtt ccccatggag ctccgaattc 1250
   ttgcgtgtgt gtagatgagg ggcgggggac gggcgccagg cattgttcag 1300
   acctggtcgg ggcccactgg aagcatccag aacagcacca ccatctta 1348
<210> 8
   <211> 250
   <212> PRT
   <213> Homo sapiens
<400> 8
<210> 9
   <211> 265
   <212> PRT
   <213> Homo sapiens
<400> 9
   Met Ser Gly Leu Gly Arg Ser Arg Arg Gly Gly Arg Ser Arg Val
   1               5                   10                  15
   Asp Gln Glu Glu Arg Phe Pro Gln Gly Leu Trp Thr Gly Val Ala
                   20                  25                  30
   Met Arg Ser Cys Pro Glu Glu Gln Tyr Trp Asp Pro Leu Leu Gly
                   35                  40                  45
   Thr Cys Met Ser Cys Lys Thr Ile Cys Asn His Gln Ser Gln Arg
                   50                  55                  60
   Thr Cys Ala Ala Phe Cys Arg Ser Leu Ser Cys Arg Lys Glu Gln
                   65                  70                  75
   Gly Lys Phe Tyr Asp His Leu Leu Arg Asp Cys Ile Ser Cys Ala
                   80                  85                  90
   Ser Ile Cys Gly Gln His Pro Lys Gln Cys Ala Tyr Phe Cys Glu
                   95                  100                 105
   Asn Lys Leu Arg Ser Pro Val Asn Leu Pro Pro Glu Leu Arg Arg
                   110                 115                 120
   Gln Arg Ser Gly Glu Val Glu Asn Asn Ser Asp Asn Ser Gly Arg
                   125                 130                 135
   Tyr Gln Gly Leu Glu His Arg Gly Ser Glu Ala Ser Pro Ala Leu
                   140                 145                 150
   Pro Gly Leu Lys Leu Ser Ala Asp Gln Val Ala Leu Val Tyr Ser
                   155                 160                 165
   Thr Leu Gly Leu Cys Leu Cys Ala Val Leu Cys Cys Phe Leu Val
                   170                 175                 180
   Ala Val Ala Cys Phe Leu Lys Lys Arg Gly Asp Pro Cys Ser Cys
                   185                 190                 195
   Gln Pro Arg Ser Arg Pro Arg Gln Ser Pro Ala Lys Ser Ser Gln
   200 205 210
   Asp His Ala Met Glu Ala Gly Ser Pro Val Ser Thr Ser Pro Glu
   215 220 225
   Pro Val Glu Thr Cys Ser Phe Cys Phe Pro Glu Cys Arg Ala Pro
   230 235 240
   Thr Gln Glu Ser Ala Val Thr Pro Gly Thr Pro Asp Pro Thr Cys
   245 250 255
   Ala Gly Arg Thr Ala Pro Pro Arg Glu Gly
   260 265
<210> 10
   <211> 11
   <212> PRT
   <213> Artificial sequence
<220>
   <223> Sequence is synthesized.
<400> 10
   Ala Ser Gln Lys Arg Pro Ser Gln Arg Ser Lys
   1 5 10
<210> 11
   <211> 1377
   <212> DNA
   <213> Homo sapiens
<400> 11
   tttttttttt tttttttttt gaggaagggc agctgccaaa ggttttattc 50
   tccaggagga ggggctctcg ggggctgtga gcagcaggca cgaggacttt 100
   attgcacgtg tggttaggtg atgacgacct acagctgcac tgggaactgg 150
   gactcagagt gccccgacct cctgctctat ctctctctgt ccctctctcc 200
   ctctctgtct ctctgcctct ctccctctct gcctctctcc ctctctgcct 250
   ctctccttct ctgcctgtct ctttccttct ctgcctcttt ccctctctgc 300
   ctctcttccc ctctgtctct ctctccctct gtctctctct ccctctctgt 350
   ctcctctgtt tctctccctt tctgcctcct ctgtctctct ctcctcatat 400
   ctctctcccc tctccccacc tctctttctc tctcccctcc tctccatctc 450
   tctccctcct cctttccctc cctgaccccc atttatgcac ctgggccccc 500
   ctcctgggca ggcacacaca caatgccaag gccactgtct gggatgtgtg 550
   ggcaaggctg caggactgtg gtcctggtgt ggcaccccca ccttccagca 600
   caagtggggt cgggggtccc aggcgtgact gcgctctcct gcgtgggcgc 650
   cctgcactca gggaagcaga agctgcaggt ctccactggc tcgggggatg 700
   tgctcacagg gctgccggct tccatcgcgt gatcctggga agacttggcc 750
   ggactttgac ggggccttga gcggggctgg caggagcagg gatcccccct 800
   cttcttgagg aagcaggcca ccgccaccag gaagcagcag aggacggcac 850
   acaggcagag ccccagcgtg ctgtagacca gggccacctg atctgcactc 900
   agcttcagcc ccgggagagc tggacttgct tctgagcctc tgtgctccaa 950
   tccttggtac cttcccgagt tgtctgaatt gttttcaact tctccactcc 1000
   gctgtctcct gagctctggt ggaaggttca ctgggctcct gagcttgttc 1050
   tcacagaagt atgcacattg cttagggtgc tgtccacaga tggaggcaca 1100
   gctgatgcag tccctcagga gatggtcata gaacttgcct tgctccttgc 1150
   ggcagctgag tgacctgcag aaggctgcac aggtgcgctg gctctgatgg 1200
   ttgcaaatgg ttttgcagga catgcaggta cccagcagag gatcccagta 1250
   ctgctcttcg gggcaggatc tcatagccac ccccgtccac aggccctgtg 1300
   gaaagcgctc ctcctggtcc acacggctcc ggccacctcg cctgctccgg 1350
   cccaggccac tcattactca ggatgct 1377
<210> 12
   <211> 995
   <212> DNA
   <213> Homo sapiens
<400> 12
   tcaggaaaaa aaaagacttt aatcatgaaa ccaaggaagt ttctaccatt 50
   aagctcccaa cagtaaccta gagaaatata tctaacataa agagtttcca 100
   cagttagagg acaaaaagct gtatcggcaa ctgaaaaata cagtcatcct 150
   aaagagatct gacacatcat ctattctgac aaaagatttt taaagtggca 200
   ctgctcgagt cgaaatggtt aattacctag cagaaattga tttctctatc 250
   tccgtagcac tcaaagcagc tggcaggctc ttgcaatagt cattcgtttt 300
   cgtggtgaca agaatggttg cgccttcctc catagctggg agtggaaagc 350
   aatggtcaga gtcgaccttc ggtttgctct tgatgcagtc ttcacaggtg 400
   cattcttcca ccgtgtactc gaggcctctc ggaagaataa tttcatcacc 450
   agtcctgctc ttttccaggt caatgttagc catgcccagg agacctgatc 500
   ctgtgttttt aaactcgtcc tttaatggtt cagagcttat cttccttagc 550
   aaaaacatta gcacgaaaac tgccaaagaa attattaagc tcagtcccaa 600
   acaggtccag agaatcgcat tcgttccttt cactgaattg gtcacacttg 650
   cattacaata acgctgacat gttagaggag gagtattaga agaacatcga 700
   agttgacaag gtatgcaagc atgcaacaaa ctgtcaaaat attcattttg 750
   ggagcactgc ccagccatct gcaacatgat cacaaaaaga aaacaaggga 800
   gctacagaaa gaacagcctg gaaggacaag taatatctct acaagaattc 850
   cagagcaaat gcagcaagag cagctagaat gttgagaaca atgaacttcg 900
   cctgcttcgt gggttcttac gggggctgtc tgtgtcttcg cggcacgtaa 950
   ggatttgaat accacatcta attcaagttt ctaagtttga gtctt 995
<210> 13
   <211> 858
   <212> DNA
   <213> Homo sapiens
<400> 13
   tcacagcagt ttcaatgcac caaaaaatgt gacatctcca tccagtgata 50
   tttgtgcatt ttctcttggt attgcaagtt ggagttcatc tccttcttcc 100
   agttttgcaa tgccagctga atagcaggaa ttattgggta gtgtttcagg 150
   catattttga atacatcgaa acaaagtcac cagactcaat tcatccccaa 200
   agacatggac cttcttcctc tgaattagat gtcccatggc gtaggtctta 250
   tcagtatata aaacctgacc atatataaaa aagtaaccag tttctttgac 300
   caatatttta ttctcttttt cttctagggc acttcccctt ttaaagctga 350
   gaagccatgg aacaaatgtg taagatcctt tttgtatagt tggtgtttca 400
   ctgtctgcaa tcagttgcaa gcagtcttga gtgactgttt cttctggacc 450
   ctgaacggca cgcttatttc tgctgttctg actggagttg ccttctcctg 500
   gagctggtgg ttcaaagatt ttcagtcccg cggtgacagc tggagcttcc 550
   tccaagccgg ccttgggggc tcctgctcct gctggcagct tctccgcgtg 600
   gtggccctgc agctctgccc ggaggctggc caggtcccct tgcagggcgg 650
   ccacctggta gaaagacacc accgtgaggc agcaagacag cagtgccagc 700
   agcaaggttg cagccagcag ctttccgtct ttggaggatc ggacagaggg 750
   gctttccttc cgtgggagga tggaaacaca ctccttcagt ttcatttctt 800
   ctcttttctt aaggcaagaa gtaaggcgtg actgctccct ttctgtggag 850
   tcatccat 858
<210> 14
   <211> 1348
   <212> DNA
   <213> Homo sapiens
<400> 14
   taagatggtg gtgctgttct ggatgcttcc agtgggcccc gaccaggtct 50
   gaacaatgcc tggcgcccgt cccccgcccc tcatctacac acacgcaaga 100
   attcggagct ccatggggaa cagaagcaag atatccgtaa aatcaaagtc 150
   tagggggtgg gaatgaaaag ggaaaagtga ggaacgggga gccaaaccca 200
   ggaagatgcc tctgttcctg cacattccct ctcctttata tactcagctc 250
   ttggctgtct ccagtatgta cccaccctgg tcttccaagc tgggagccac 300
   tttttataac acaatcacag tttcacaaac cccaggaagg ttccatgtgg 350
   agagaggtta agtttcgccc ttgcccgggg aattatgaca ctcagaatat 400
   ccccttggtg taaatggaag acacctgcgc tatagcagct gttgtaggcc 450
   cggtccgggt gggagggcat acttcttata catcggaata gagtctcctg 500
   ccttccttgg ccttctcgag acaccacctg acccatggtg aaagtcacgt 550
   cttgaaacag gacctggcta tacagcagat aaactccagc atcctggatt 600
   cggacaccat atccttgggc ctgtaggcct ctcccacgcc taagagctgg 650
   ttgccacatc acctctgtca catcggagtc atccttggag gtggcgttaa 700
   tgggaaccag gtgcaggaca gagtgctgct tcttctgttt ttgggtgagc 750
   actgctctcc ttttccggga tctctcccca ttctcccagg cttccagggc 800
   atcggaactc tgctccggga gactctgcca gggataccct tccccattct 850
   gggaggggcc tcctgtcccc tgcagccggc tcacctctct cctgaggctc 900
   tgcagctctg tttgttgggt cagcagagcc atggcacaag ccacggcccc 950
   cagagctgcc ccccaactca accagagggc aactgagagt gccggctctc 1000
   tgactgggcc ccccatgttg cctggaggcc ctttgggggc tagcaagaaa 1050
   ggagatgagg ctggcatgag ctggggaccc tgccaacagc tgtggtttca 1100
   agagtggggt agcaaggagg tggcggggcg ggtaagggta cgggcagtgg 1150
   tgcagaaggg aagaaggttg ttacgcaagg agaaataaaa aggaacttga 1200
   aaataaaaag gagggaggag gaaagcaagc taagggtact gttagtgctc 1250
   ctggcactcc gtcgtggggc cagcgttgcc ttgagaccct ccaccctccc 1300
   tcagcctcag gagaattagg ttccagtccc tctaggaagc ctcgtacc 1348

## Description

### FIELD OF THE INVENTION

This invention relates generally to the use of one or more agonists or antagonists to modulate activity of tumor necrosis factor (TNF) and TNF receptor (TNFR)-related molecules, and more specifically, the members of the TNF and TNFR families referred to as TALL-1, APRIL, TACI, and BCMA. The invention also relates to the *in vitro, in situ*, and/or *in vivo* diagnosis and/or treatment of mammalian cells or pathological conditions associated with TALL-1, APRIL, TACI, or BCMA, using one or more agonist or antagonist molecules.

### BACKGROUND OF THE INVENTION

Various molecules, such as tumor necrosis factor-α ("TNF-α"), tumor necrosis factor-β ("TNF-β" or "lymphotoxin-α"), lymphotoxin-β ("LT-β"), CD30 ligand, CD27 ligand, CD40 ligand, OX-40 ligand, 4-1BB ligand, Apo-1 ligand (also referred to as Fas ligand or CD95 ligand), Apo-2 ligand (also referred to as TRAIL), Apo-3 ligand (also referred to as TWEAK), osteoprotegerin (OPG), APRIL, RANK ligand (also referred to as TRANCE), and TALL-1 (also referred to as BlyS, BAFF or THANK) have been identified as members of the tumor necrosis factor ("TNF") family of cytokines [See, e.g., Gruss and Dower, Blood, 85:3378-3404 (1995); Pitti et al., J. Biol. Chem., 271:12687-12690 (1996); Wiley et al., Immunity, 3:673-682 (1995); Browning et al., Cell, 72:847-856 (1993); Armitage et al. Nature, 357:80-82 (1992), WO 97/01633 published January 16, 1997; WO 97/25428 published July 17, 1997; Marsters et al., Curr. Biol., 8:525-528 (1998); Simonet et al., Cell, 89:309-319 (1997); Chicheportiche et al., Biol. Chem., 272:32901-32910 (1997); Hahne et al., J. Exp. Med., 188:1185-1190 (1998); WO98/2842 published July 2, 1998; WO98/46751 published October 22, 1998; WO/98/18921 published May 7, 1998; Moore et al., Science, 285:260-263 (1999); Shu et al., J. Leukocyte Biol., 65:680 (1999); Schneider et al., J. Exp. Med., 189:1747-1756 (1999); Mukhopadhyay et al., J. Biol. Chem., 274:15978-15981 (1999)]. Among these molecules, TNF-α, TNF-β, CD30 ligand, 4-1BB ligand, Apo-1 ligand, Apo-2 ligand (Apo2L/TRAIL) and Apo-3 ligand (TWEAK) have been reported to be involved in apoptotic cell death. Both TNF-α and TNF-β have been reported to induce apoptotic death in susceptible tumor cells [Schmid et al., Proc. Natl. Acad. Sci., 83:1881 (1986); Dealtry et al., Eur. J. Immunol., 17:689 (1987)].

Various molecules in the TNF family also have purported role(s) in the function or development of the immune system [Gruss et al., Blood, 85:3378 (1995)]. Zheng et al. have reported that TNF-α is involved in post-stimulation apoptosis of CD8-positive T cells [Zheng et al., Nature, 377:348-351 (1995)]. Other investigators have reported that CD30 ligand may be involved in deletion of self-reactive T cells in the thymus [Amakawa et al., Cold Spring Harbor Laboratory Symposium on Programmed Cell Death, Abstr. No. 10, (1995)]. CD40 ligand activates many functions of B cells, including proliferation, immunoglobulin secretion, and survival [Renshaw et al., J. Exp. Med., 180:1889 (1994)]. Another recently identified TNF family cytokine, TALL-1 (BlyS), has been reported, under certain conditions, to induce B cell proliferation and immunoglobulin secretion. [Moore et al., supra; Schneider et al., supra; Mackay et al., J. Exp. Med., 190:1697 (1999)].

Mutations in the mouse Fas/Apo-1 receptor or ligand genes (*called lpr and gld*, respectively) have been associated with some autoimmune disorders, indicating that Apo-1 ligand may play a role in regulating the clonal deletion of self-reactive lymphocytes in the periphery [Krammer et al., Curr. Op. Immunol., 6:279-289 (1994); Nagata et al., Science, 267:1449-1456 (1995)]. Apo-1 ligand is also reported to induce post-stimulation apoptosis in CD4-positive T lymphocytes and in B lymphocytes, and may be involved in the elimination of activated lymphocytes when their function is no longer needed [Krammer et al., supra; Nagata et al., supra]. Agonist mouse monoclonal antibodies specifically binding to the Apo-1 receptor have been reported to exhibit cell killing activity that is comparable to or similar to that of TNF-α [Yonehara et al., J. Exp. Med., 169:1747-1756 (1989)].

Induction of various cellular responses mediated by such TNF family cytokines is believed to be initiated by their binding to specific cell receptors. Previously, two distinct TNF receptors of approximately 55-kDa (TNFR1) and 75-kDa (TNFR2) were identified [Hohman et al., J. Biol. Chem., 264:14927-14934 (1989); Brockhaus et al., Proc. Natl. Acad. Sci., 87:3127-3131 (1990); EP 417,563, published March 20, 1991; Loetscher et al., Cell, 61:351 (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026 (1991)]. Those TNFRs were found to share the typical structure of cell surface receptors including extracellular, transmembrane and intracellular regions. The extracellular portions of both receptors were found naturally also as soluble TNF-binding proteins [Nophar, Y. et al., EMBO J., 9:3269 (1990); and Kohno, T. et al., Proc. Natl. Acad. Sci. U.S.A., 87:8331 (1990); Hale et al., J. Cell. Biochem. Supplement 15F, 1991, p. 113 (P424)].

The extracellular portion of type 1 and type 2 TNFRs (TNFR1 and TNFR2) contains a repetitive amino acid sequence pattern of four cysteine-rich domains (CRDs) designated 1 through 4, starting from the NH₂-terminus. [Schall et al., supra; Loetscher et al., supra; Smith et al., supra; Nophar et al., supra; Kohno et al., supra; Banner et al., Cell, 73: 431-435 (1993)]. A similar repetitive pattern of CRDs exists in several other cell-surface proteins, including the p75 nerve growth factor receptor (NGFR) [Johnson et al., Cell, 47:545 (1986); Radeke et al., Nature, 325:593 (1987)], the B cell antigen CD40 [Stamenkovic et al., EMBO J., 8:1403 (1989)], the T cell antigen OX40 [Mallet et al., EMBO J., 9:1063 (1990)] and the Fas antigen [Yonehara et al., supra and Itoh et al., Cell, 66:233-243 (1991)]. CRDs are also found in the soluble TNFR (sTNFR)-like T2 proteins of the Shope and myxoma poxviruses [Upton et al., Virology, 160:20-29 (1987); Smith et al., Biochem. Biophys. Res. Commun., 176:335 (1991); Upton et al., Virology, 189:370 (1991)]. Optimal alignment of these sequences indicates that the positions of the cysteine residues are well conserved. These receptors are sometimes collectively referred to as members of the TNF/NGF receptor superfamily.

The TNF family ligands identified to date, with the exception of lymphotoxin-α, are type II transmembrane proteins, whose C-terminus is extracellular. In contrast, most receptors in the TNF receptor (TNFR) family identified to date are type I transmembrane proteins. In both the TNF ligand and receptor families, however, homology identified between family members has been found mainly in the extracellular domain ("ECD"). Several of the TNF family cytokines, including TNF-α, Apo-1 ligand and CD40 ligand, are cleaved proteolytically at the cell surface; the resulting protein in each case typically forms a homotrimeric molecule that functions as a soluble cytokine. TNF receptor family proteins are also usually cleaved proteolytically to release soluble receptor ECDs that can function as inhibitors of the cognate cytokines.

More recently, other members of the TNFR family have been identified. In von Bulow et al., Science, 278:138-141 (1997), investigators describe a plasma membrane receptor referred to as Transmembrane Activator and CAML-Interactor or "TACI". The TACI receptor is reported to contain a cysteine-rich motif characteristic of the TNFR family and to be present on both B cells and activated T cells. In an *in vitro* assay, cross linking of TACI on the surface of transfected Jurkat cells with TACI-specific antibodies led to activation of NF-KB. [see also, WO 98/39361 published September 18, 1998].

Laabi et al., EMBO J., 11:3897-3904 (1992) reported identifying a new gene called "BCM" whose expression was found to coincide with B cell terminal maturation. The open reading frame of the BCM normal cDNA predicted a 184 amino acid long polypeptide with a single transmembrane domain. These investigators later termed this gene "BCMA." [Laabi et al., Nucleic Acids Res., 22:1147-1154 (1994)]. BCMA mRNA expression was reported to be absent in human malignant B cell lines which represent the pro-B lymphocyte stage, and thus, is believed to be linked to the stage of differentiation of lymphocytes [Gras et al., Int. Immunology, 7:1093-1106 (1995)]. In Madry et al., Int. Immunology, 10:1693-1702 (1998), the cloning of murine BCMA cDNA was described. The murine BCMA cDNA is reported to encode a 185 amino acid long polypeptide having 62% identity to the human BCMA polypeptide. Alignment of the murine and human BCMA protein sequences revealed a conserved motif of six cysteines in the N-terminal region, suggesting that the BCMA protein belongs to the TNFR superfamily [Madry et al., supra].

In Marsters et al., Curr. Biol., 6:750 (1996), investigators describe a full length native sequence human polypeptide, called Apo-3, which exhibits similarity to the TNFR family in its extracellular cysteine-rich repeats and resembles TNFR1 and CD95 in that it contains a cytoplasmic death domain sequence [see also Marsters et al., Curr. Biol., 6:1669 (1996)]. Apo-3 has also been referred to by other investigators as DR3, ws1-1, TRAMP, and LARD [Chinnaiyan et al., Science, 274:990 (1996); Kitson et al., Nature, 384:372 (1996); Bodmer et al., Immunity, 6:79 (1997); Screaton et al., Proc. Natl. Acad. Sci., 94:4615-4619 (1997)].

Pan et al. have disclosed another TNF receptor family member referred to as "DR4" [Pan et al., Science, 276:111-113 (1997); see also WO98/32856 published July 30, 1998]. The DR4 was reported to contain a cytoplasmic death domain capable of engaging the cell suicide apparatus. Pan et al. disclose that DR4 is believed to be a receptor for the ligand known as Apo2L/TRAIL.

In Sheridan et al., Science, 277:818-821 (1997) and Pan et al., Science, 277:815-818 (1997), another molecule believed to be a receptor for Apo2L/TRAIL is described [see also, WO98/51793 published November 19, 1998; WO98/4162 published September 24, 1998]. That molecule is referred to as DR5 (it has also been alternatively referred to as Apo-2; TRAIL-R, TR6, Tango-63, hAPO8, TRICK2 or KILLER [Screaton et al., Curr. Biol., 7:693-696 (1997); Walczak et al., EMBO J., 16:5386-5387 (1997); Wu et al., Nature Genetics, 17: 141-143 (1997); WO98/35986 published August 20, 1998; EP 870,827 published October 14, 1998; WO98/46643 published October 22, 1998; WO99/02653 published January 21, 1999; WO99/09165 published February 25, 1999; WO99/11791 published March 11, 1999]. Like DR4, DR5 is reported to contain a cytoplasmic death domain and be capable of signaling apoptosis. The crystal structure of the complex formed between Apo-2L/TRAIL and DR5 is described in Hymowitz et al., Molecular Cell, 4:563-571 (1999).

Yet another death domain-containing receptor, DR6, was recently identified [Pan et al., FEBS Letters, 431:351-356 (1998)]. Aside from containing four putative extracellular cysteine rich domains and a cytoplasmic death domain, DR6 is believed to contain a putative leucine-zipper sequence that overlaps with a proline-rich motif in the cytoplasmic region. The proline-rich motif resembles sequences that bind to src-homology-3 domains, which are found in many intracellular signal-transducing molecules.

A further group of recently identified receptors are referred to as "decoy receptors," which are believed to function as inhibitors, rather than transducers of signaling. This group includes DCR1 (also referred to as TRID, LIT or TRAIL-R3) [Pan et al., Science, 276:111-113 (1997); Sheridan et al., Science, 277:818-821 (1997); McFarlane et al., J. Biol. Chem., 272:25417-25420 (1997); Schneider et al., FEBS Letters, 416:329-334 (1997); Degli-Esposti et al., J. Exp. Med., 186:1165-1170 (1997); and Mongkolsapaya et al., J. Immunol., 160:3-6 (1998)] and DCR2 (also called TRUNDD or TRAIL-R4) [Marsters et al., Curr. Biol., 7:1003-1006 (1997); Pan et al., FEBS Letters, 424:41-45 (1998); Degli-Esposti et al., Immunity, 7:813-820 (1997)], both cell surface molecules, as well as OPG [Simonet et al., supra; Emery et al., infra] and DCR3 [Pitti et al., Nature, 396:699-703 (1998)], both of which are secreted, soluble proteins.

Additional newly identified members of the TNFR family include CAR1, HVEM, GITR, ZTNFR-5, NTR-1, and TNFL1 [Brojatsch et al., Cell, 87:845-855 (1996); Montgomery et al., Cell, 87:427-436 (1996); Marsters et al., J. Biol. Chem., 272:14029-14032 (1997); Nocentini et al., Proc. Natl. Acad. Sci. USA 94:6216-6221 (1997); Emery et al., J. Biol. Chem., 273:14363-14367 (1998); WO99/04001 published January 28, 1999; WO99/07738 published February 18, 1999; WO99/33980 published July 8, 1999].

As reviewed recently by Tewari et al., TNFR1, TNFR2 and CD40 modulate the expression of proinflammatory and costimulatory cytokines, cytokine receptors, and cell adhesion molecules through activation of the transcription factor, NF-κB [Tewari et al., Curr. Op. Genet. Develop., 6:39-44 (1996)]. NF-κB is the prototype of a family of dimeric transcription factors whose subunits contain conserved Rel regions [Verma et al., Genes Develop., 9:2723-2735 (1996); Baldwin, Ann. Rev. Immunol., 14:649-681 (1996)]. In its latent form, NF-κB is complexed with members of the IκB inhibitor family; upon inactivation of the IκB in response to certain stimuli, released NF-κB translocates to the nucleus where it binds to specific DNA sequences and activates gene transcription. As described above, the TNFR members identified to date either include or lack an intracellular death domain region. Some TNFR molecules lacking a death domain, such as TNFR2, CD40, HVEM, and GITR, are capable of modulating NF-κB activity. [see, e.g., Lotz et al., J. Leukocyte Biol., 60:1-7 (1996)].

For a review of the TNF family of cytokines and their receptors, see Ashkenazi and Dixit, Science, 281:1305-1308 (1998); Golstein, Curr. Biol., 7:750-753 (1997); Gruss and Dower, supra, and Nagata, Cell, 88:355-365 (1997).

### SUMMARY OF THE INVENTION

The invention is as defined in the claims.

Applicants have surprisingly found that the TNF family ligand referred to as TALL-1 binds to the TACI receptor and to the BCMA receptor. Applicants have also surprisingly found that the TNF family ligand referred to as APRIL binds to both the TACI and BCMA receptors. Although certain TALL-1 and APRIL ligands, and certain TACI and BCMA receptors, have been described previously, it was not appreciated in the art that TALL-1 and APRIL bind and activate the TACI and BCMA receptors. The present invention thus provides for novel medical use of antibody antagonists of these TNF-related APRIL ligands according to the claims. The antagonists described herein find utility for, among other things, *in vitro*, *in* situ, or *in vivo* 10 diagnosis or treatment of mammalian cells or pathological conditions associated with the presence (or absence) of TALL-1, APRIL, TACI, or BCMA.

The medical use relates to methods to treat pathological conditions or diseases in mammals associated with or resulting from 15 increased or enhanced TALL-1 or APRIL expression and/or activity. In the methods of treatment, APRIL antagonists may be administered to the mammal suffering from such pathological condition or disease. The APRIL antagonists contemplated for use are anti-ARRIL antibodies which are capable of blocking or reducing binding of the respective ligand to the TACI or TACI and BCMA receptors. Optionally, the antagonist molecule(s) employed in the methods will be capable of blocking or neutralizing the activity of both TALL-1 and APRIL, e.g., a dual antagonist which blocks or neutralizes activity of both TALL-1 and APRIL. The methods contemplate the use of a single type of antagonist molecule or a combination of two or more types of antagonist.

The invention thus provides medical use of APRIL antagonists in methods to block or neutralize the interaction between APRIL and TACI or TACI and BCMA according to the claims. For example, a method may comprise exposing a mammalian cell, such as a white blood cell (preferably a B cell), to one or more APRIL antagonists in an amount effective to decrease, neutralize or block activity of the APRIL ligand. The cell may be in cell culture or in a mammal, e.g. a mammal suffering from, for instance, an immune related disease or cancer. Thus, the invention relates to a method for treating a mammal suffering from a pathological condition such as an immune related disease or cancer comprising administering an effective amount of one or more APRIL antagonists, as disclosed herein.

The invention also provides compositions which comprise one or more APRIL antagonists according to the claims. Optionally, the compositions of the invention will include pharmaceutically acceptable carriers or diluents. Preferably, the compositions will include one or more TALL-1 antagonists or APRIL antagonists in an amount which is therapeutically effective to treat a pathological condition or disease.

The invention also provides particular antagonist molecules comprising monoclonal anti-APRIL antibodies according to the claims. Such antibodies may include chimeric antibodies, humanized antibodies or human antibodies. Hybridomas secreting various anti-APRIL monoclonal antibodies are further described herein.

Articles of manufacture and kits which include one or more TALL-1 antagonists or APRIL antagonists are also described herein.

The invention also provides methods of conducting screening assays to identify candidate molecules, such as small molecule compounds, polypeptides or antibodies, which act as agonists or antagonists with respect to the interaction between APRIL and TACI or BCMA according to the claims.

### BRIEF DESCRIPTION OF THE DRAWING

Figures 1A-1B show a polynucleotide sequence encoding a native sequence human TACI (SEQ ID NO:1) (reverse complimentary sequence is provided in SEQ ID NO:11) and its putative amino acid sequence (SEQ ID NO:2).
Figure 2 shows a polynucleotide sequence encoding a native sequence human BCMA (SEQ ID NO:3) (reverse complimentary sequence is provided in SEQ ID NO:12) and its putative amino acid sequence (SEQ ID NO:4).
Figure 3 shows a polynucleotide sequence encoding a native sequence human TALL-1 (SEQ ID NO:5) (reverse complimentary sequence is provided in SEQ ID NO:13) and its putative amino acid sequence (SEQ ID NO:6).
Figures 4A-4B show a polynucleotide sequence encoding a native sequence human APRIL (SEQ ID NO:7) (reverse complimentary sequence is provided in SEQ ID NO:14) and its putative amino acid sequence (SEQ ID NO:8).
Figures 5A-5B show exemplary methods for calculating the % amino acid sequence identity of the amino acid sequence designated "Comparison Protein" to the amino acid sequence designated "PRO". For purposes herein, the "PRO" sequence may be the TACI, BCMA, TALL-1, or APRIL sequences referred to in Figures 1, 2, 3, or 4 herein, respectively.
Figure 6 shows an alignment of two amino acid sequences for the TACI receptor, referred to as "hTACI (265)" (SEQ ID NO:9), believed to be a spliced variant, and "hTACI", also referred to in Figures 1A-1B (SEQ ID NO:2).
Figures 7A-7D show the results of an in situ assay, staining for AP activity. COS 7 cells were transfected with TACI (Fig. 7A-7C) or vector plasmid (Fig. 7D) and incubated with AP-TALL-1 (Fig. 7A, 7D); AP-TNF-alpha (Fig. 7B); or AP-EDA (Fig. 7C).
Figures 8A-8H show the results, by Western blot analysis, of various co-immunoprecipitation assays of TALL-1 or APRIL with TACI or BCMA immunoadhesins, as described in detail in Example 2. In Figures 8A-8H, TALL-1 is referred to as "Blys/TALL-1".

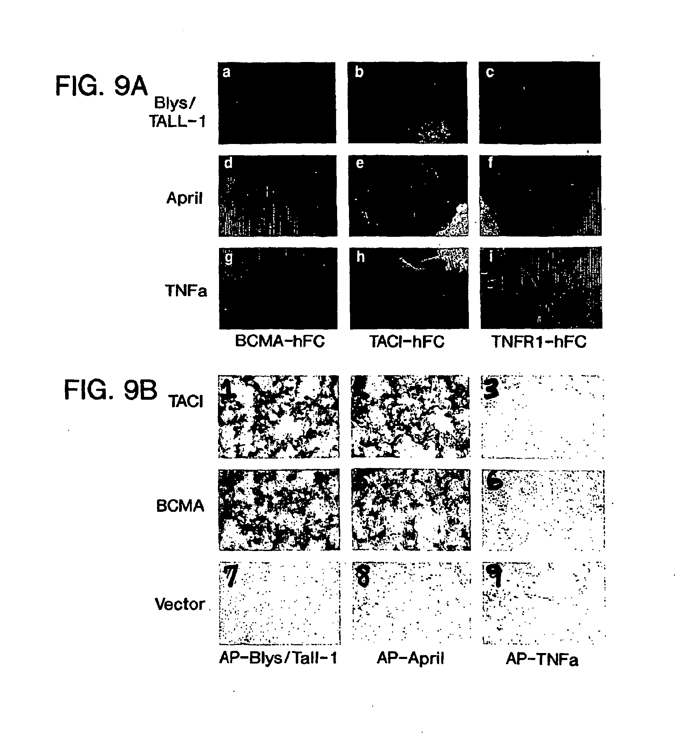

Figures 9A-9B show the results of further binding assays to demonstrate TALL-1 and APRIL are ligands for TACI and BCMA. In Figure 9A, COS 7 cells were transfected with TALL-1 (9a-9c); APRIL (9d-9f); or TNF-alpha (9g-9i) and incubated with BCMA-Fc (9a, 9d, and 9g); TACI-Fc (9b, 9e, and 9h); or TNFR1-Fc (9c, 9f, and 9i). Cells were then washed, fixed and the binding of Fc protein was detected by biotinylated goat anti-human antibody followed by Cy3-streptavidin. In Figure 9B, COS 7 cells were transfected with TACI (1, 2, and 3): BCMA (4, 5, and 6); or vector (7, 8, and 9) and incubated with conditioned medium containing AP-TALL-1 (1, 4, and 7), AP-APRIL (2, 5, and 8) or AP-TNF-alpha (3, 6, and 9). Cells were then washed, fixed and stained for AP activity in situ. In Figures 9A-9B, TALL-1 is referred to as "BlyS/TALL-1".
Figure 10 shows a bar diagram illustrating the results of an IgM ELISA, testing the effects of the indicated cytokines, ligands and receptors on IgM production in target PBLs.
Figures 11A-11D shows the results of assays demonstrating that interaction between TALL-1 or APRIL and TACI or BCMA results in activation of NF-KB. In Figure 11A, stimulation of TACI/BCMA mediated NF-KB activation by TALL-1 or APRIL. In Figures 11B and 11C, stimulation of TACI/BCMA mediated NF-KB activation by co-transfection of full-length TALL-1 or APRIL. In Figure 11D, treatment of untransfected IM-9 cells with Flag-TALL-1 also resulted in activation of NF-KB, as measured in an electrophoretic mobility shift assay.
Figure 12 shows a bar diagram illustrating the results of an ELISA testing the effects of blocking TALL-1/TACI and TALL-1/BCMA interactions on NP-specific IgM, low affinity IgG1, and high affinity IgG1 production.
Figures 13-1 (panels A, B and C) and 13-2 (panels A, B, and C) show immunohistochemical analysis of spleen sections from immunized mice treated with TACI-Fc or BCMA-Fc, and described in further detail in Example 5.
Figure 14A shows the results of an ELISA testing binding of various anti-APRIL antibodies to Flag-APRIL.
Figure 14B shows a table indicating the various results of assays of monoclonal antibodies 3C6.4.2; 5E8.7.4; 5E11.1.2; and 5G8.2.2, including isotype analysis.
Figures 15A-15D show bar diagrams illustrating the results of competitive binding ELISAs of anti-APRIL antibodies 3C6.4.2 ("3C6") (Fig. 15A); 5E8.7.4 ("5E8") (Fig. 15B); 5E11. 1.2 ("5E11") (Fig. 15C); and 5G8.2.2 ("5G8") (Fig. 15D), and described in further detail in Example 9.
Figures 16A and 16B shows the results of assays demonstrating inhibition of collagen-induced arthritis by TACI-Fc treatment in an *in vivo* murine model. The arthritic score observations are described in Example 10.
Figures 17A-17F show histo-chemical and radiological profiles of joints of CIA mice treated with TACI-Fc or Controls.
Figures 18A-18E shows the results of assays demonstrating TACI-Fc inhibited anti-collagen immune responses in the CIA murine model. Figs. 18A and 18B show serum levels of anti-BCII IgG1 and IgG2a isotype antibodies; Fig. 18C illustrates the effects of TACI-Fc on proliferative T cell responses; Fig. 18D illustrates the effects of TACI-Fc on IL-2 production by T cells; Fig. 18E illustrates the effects of TACI-Fc on Interferon-gamma production by T cells.
Figures 19A-19B show the inhibitory effects of TACI-Fc on anti-CD3 induced proliferation of naïve T cells ((Fig. 19A) and on anti-CD3 induced IL-2 production by naïve T cells, as measured in in vitro assays.
Figure 20 shows the results of an assay demonstrating inhibition of experimental allergic encephalomyelitis (EAE) by TACI-Fc treatment in an *in vivo* murine model. The clinical EAE score observations are described in Example 11.

### DETAILED DESCRIPTION OF THE INVENTION

### I. Definitions

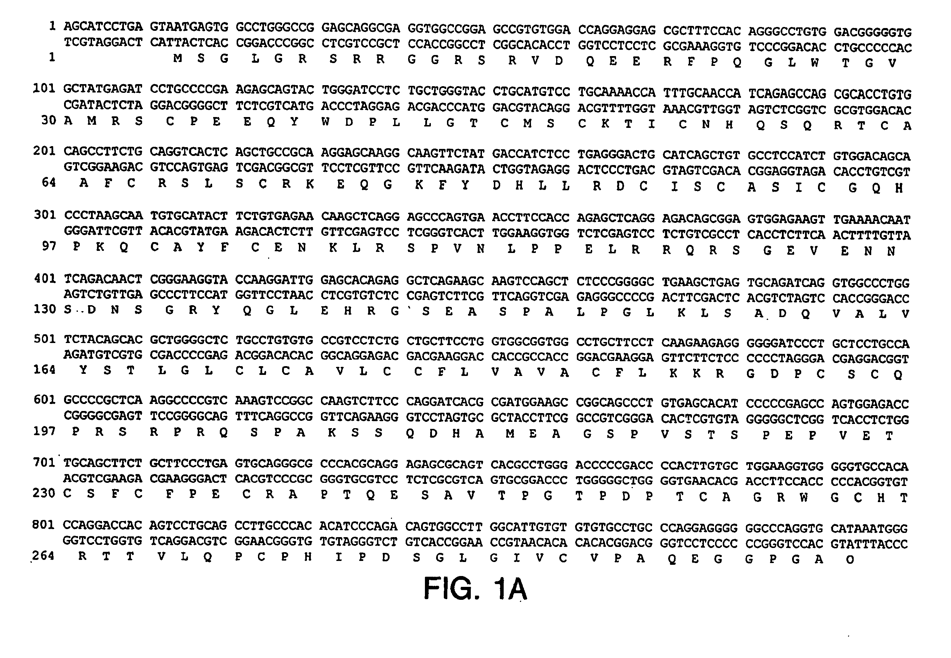

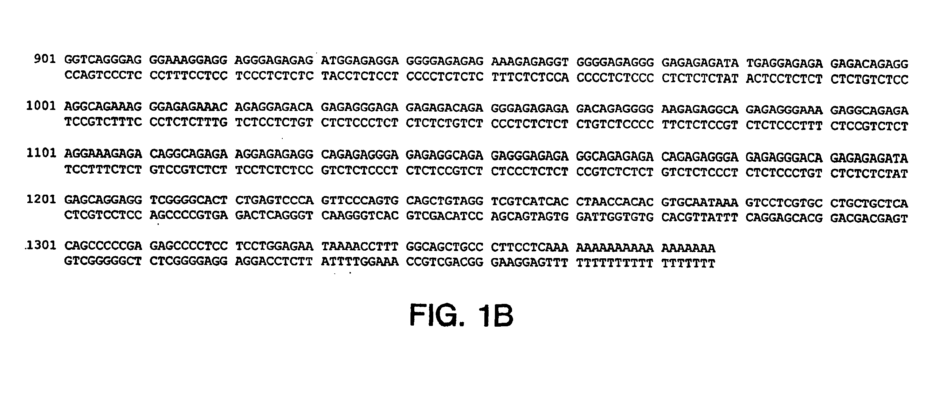

The terms "TACI" or "TACI polypeptide" or "TACI preceptor" when used herein encompass "native sequence TACI polypeptides" and "TACI variants" (which are further defined herein). "TACI" is a designation given to those polypeptides which are encoded by the nucleic acid molecules comprising the polynucleotide sequences shown in Figure 1 and variants or fragments thereof, nucleic acid molecules comprising the sequence shown in the Figure 1 and variants thereof as well as fragments of the above. The TACI polypeptides may be isolated from a variety of sources, such as from human tissue types or from another source, or prepared by recombinant and/or synthetic methods.

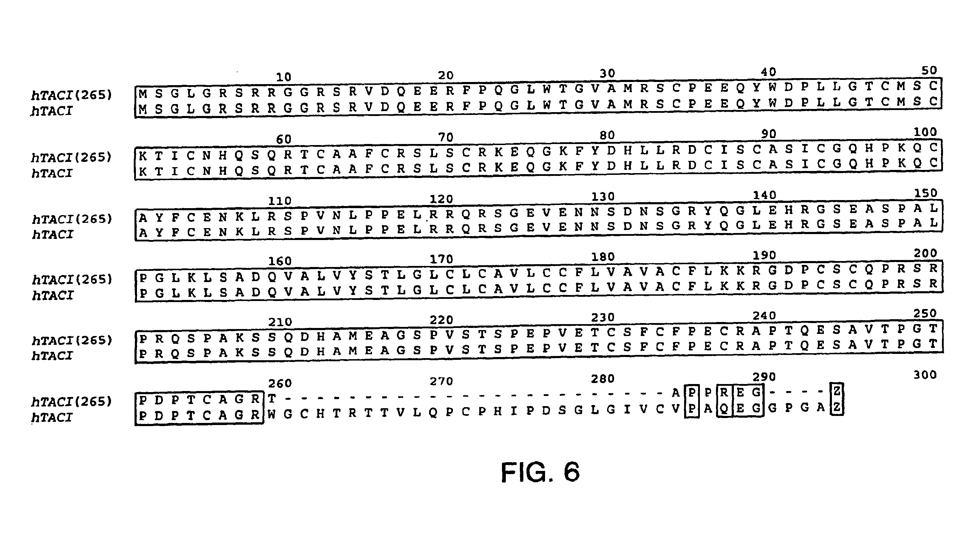

A "native sequence" TACI polypeptide comprises a polypeptide having the same amino acid sequence as the corresponding TACI polypeptide derived from nature. Such native sequence TACI polypeptides can be isolated from nature or can be produced by recombinant and/or synthetic means. The term "native sequence TACI polypeptide" specifically encompasses naturally-occurring truncated or secreted forms (e.g., an extracellular domain sequence), naturally-occurring variant forms (e.g., alternatively spliced forms) and naturally-occurring allelic variants of the polypeptide. The TACI polypeptides include but are not limited to the polypeptides described in von Bulow et al., supra and WO98/39361 published September 11, 1998, the spliced variant (referred to as "hTACI(265)" above and shown in Fig. 6 (SEQ ID NO:9)), and the TACI polypeptide comprising the contiguous sequence of amino acid residues 1-293 of Figs. 1A-1B (SEQ ID NO:2).

A TACI "extracellular domain" or "ECD" refers to a form of the TACI polypeptide which is essentially free of the transmembrane and cytoplasmic domains. Ordinarily, a TACI polypeptide ECD will have less than about 1% of such transmembrane and/or cytoplasmic domains and preferably, will have less than about 0.5% of such domains. It will be understood that any transmembrane domain(s) identified for the TACI polypeptides are identified pursuant to criteria routinely employed in the art for identifying that type of hydrophobic domain. The exact boundaries of a transmembrane domain may vary but most likely by no more than about 5 amino acids at either end of the domain as initially identified. ECD forms of TACI include those described in von Bulow et al., supra and WO98/39361.

"TACI variant" means a TACI polypeptide having at least about 80% amino acid sequence identity with the amino acid sequence of a native sequence full length TACI or TACI ECD. Preferably such TACI variant acts as a TALL-1 antagonist or APRIL antagonist as defined below. Such TACI variant polypeptides include, for instance, TACI polypeptides wherein one or more amino acid residues are added, or deleted, at the N- and/or C-terminus, as well as within one or more internal domains, of the full-length amino acid sequence. Fragments of the TACI ECD are also contemplated. Ordinarily, a TACI variant polypeptide will have at least about 80% amino acid sequence identity, more preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with a TACI polypeptide encoded by a nucleic acid molecule shown in Figure 1 or a specified fragment thereof. TACI variant polypeptides do not encompass the native TACI polypeptide sequence. Ordinarily, TACI variant polypeptides are at least about 10 amino acids in length, often at least about 20 amino acids in length, more often at least about 30 amino acids in length, more often at least about 40 amino acids in length, more often at least about 50 amino acids in length, more often at least about 60 amino acids in length, more often at least about 70 amino acids in length, more often at least about 80 amino acids in length, more often at least about 90 amino acids in length, more often at least about 100 amino acids in length, more often at least about 150 amino acids in length, more often at least about 200 amino acids in length, more often at least about 250 amino acids in length, more often at least about 300 amino acids in length, or more.

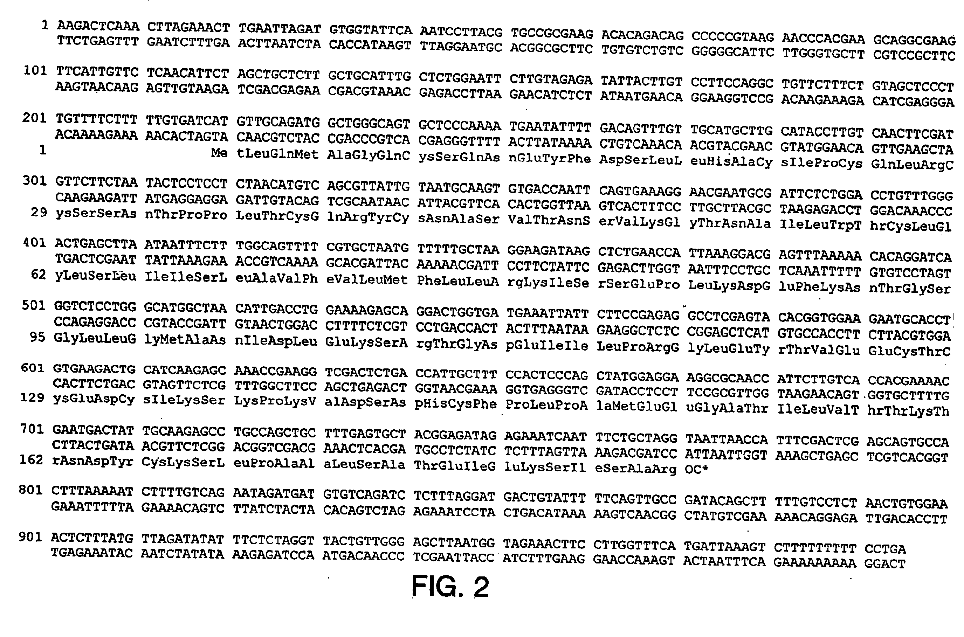

The terms "BCMA" or "BCMA polypeptide" or "BCMA receptor" when used herein encompass "native sequence BCMA polypeptides" and "BCMA variants" (which are further defined herein). "BCMA" is a designation given to those polypeptides which are encoded by the nucleic acid molecules comprising the polynucleotide sequences shown in Figure 2 and variants thereof, nucleic acid molecules comprising the sequence shown in the Figure 2 and variants thereof as well as fragments of the above. The BCMA polypeptides may be isolated from a variety of sources, such as from human tissue types or from another source, or prepared by recombinant and/or synthetic methods.

A "native sequence" BCMA polypeptide comprises a polypeptide having the same amino acid sequence as the corresponding BCMA polypeptide derived from nature. Such native sequence BCMA polypeptides can be isolated from nature or can be produced by recombinant and/or synthetic means. The term "native sequence BCMA polypeptide" specifically encompasses naturally-occurring truncated or secreted forms (e.g., an extracellular domain sequence), naturally-occurring variant forms (e.g., alternatively spliced forms) and naturally-occurring allelic variants of the polypeptide. The BCMA polypeptides include the polypeptides described in Laabi et al., EMBO J., 11:3897-3904 (1992), Laabi et al., Nucleic Acids Res., 22:1147-1154 (1994), Gras et al., Int. Immunology, 7:1093-1106 (1995); Madry et al., Int. Immunology, 10:1693-1702 (1998); and the BCMA polypeptide comprising the contiguous sequence of amino acid residues 1-184 of Fig. 2 (SEQ ID NO:4).

A BCMA "extracellular domain" or "ECD" refers to a form of the BCMA polypeptide which is essentially free of the transmembrane anc cytoplasmic domains. Ordinarily, a BCMA polypeptide ECD will have less than about 1% of such transmembrane and/or cytoplasmic domains and preferably, will have less than about 0.5% of such domains. It will be understood that any transmembrane domain(s) identified for the BCMA polypeptides are identified pursuant to criteria routinely employed in the art for identifying that type of hydrophobic domain. The exact boundaries of a transmembrane domain may vary but most likely by no more than about 5 amino acids at either end of the domain as initially identified. ECD forms of TACI include those described in Laabi et al., EMBO J., 11:3897-3904 (1992); Laabi et al., Nucleic Acids Res., 22:1147-1154 (1994); Gras et al., Int. Immunology, 7:1093-1106 (1995); Madry et al., Int. Immunology, 10:1693-1702 (1998).

"BCMA variant" means a BCMA polypeptide having at least about 80% amino acid sequence identity with the amino acid sequence of a native sequence BCMA or BCMA ECD. Preferably such a BCMA variant acts as a TALL-1 antagonist or APRIL antagonist as defined below. Such BCMA variant polypeptides include, for instance, BCMA polypeptides wherein one or more amino acid residues are added, or deleted, at the N- and/or C-terminus, as well as within one or more internal domains, of the full-length amino acid sequence. Fragments of the BCMA ECD are also contemplated. Ordinarily, a BCMA variant polypeptide will have at least about 80% amino acid sequence identity, more preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with a BCMA polypeptide encoded by a nucleic acid molecule shown in Figure 2 or a specified fragment thereof. BCMA variant polypeptides do not encompass the native BCMA polypeptide sequence. Ordinarily, BCMA variant polypeptides are at least about 10 amino acids in length, often at least about 20 amino acids in length, more often at least about 30 amino acids in length, more often at least about 40 amino acids in length, more often at least about 50 amino acids in length, more often at least about 60 amino acids in length, more often at least about 70 amino acids in length, more often at least about 80 amino acids in length, more often at least about 90 amino acids in length, more often at least about 100 amino acids in length, more often at least about 150 amino acids in length, more often at least about 200 amino acids in length, more often at least about 250 amino acids in length, more often at least about 300 amino acids in length, or more.

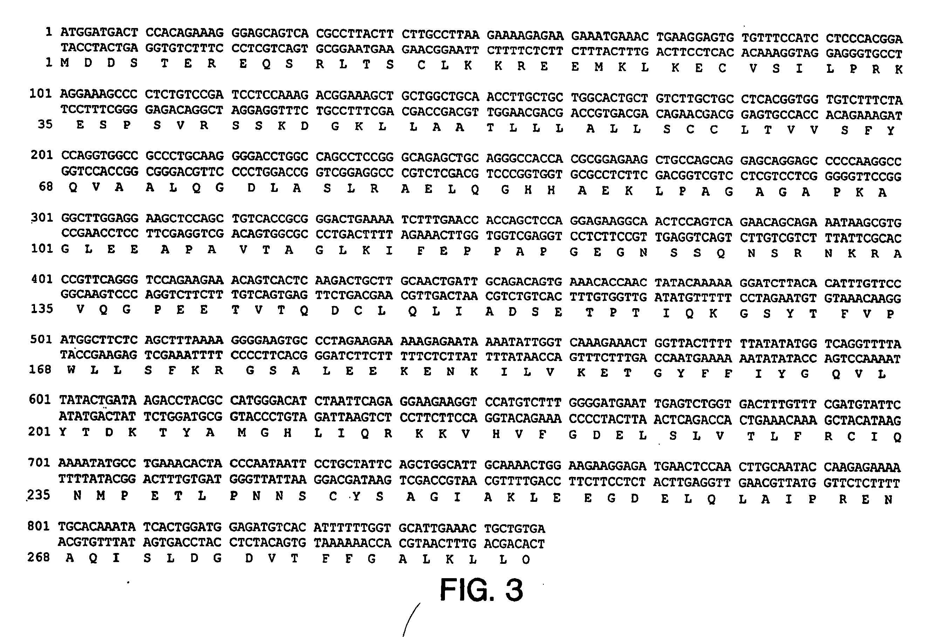

The terms "TALL-1" or "TALL-1 polypeptide" when used herein encompass "native sequence TALL-1 polypeptides" and "TALL-1 variants". "TALL-1" is a designation given to those polypeptides which are encoded by the nucleic acid molecules comprising the polynucleotide sequences shown in Figure 3 and variants thereof, nucleic acid molecules comprising the sequence shown in the Figure 3, and variants thereof as well as fragments of the above which have the biological activity of the native sequence TALL-1. Variants of TALL-1 will preferably have at least 80%, more preferably, at least 90%, and even more preferably, at least 95% amino acid sequence identity with the native sequence TALL-1 polypeptide shown in Figure 3. A "native sequence" TALL-1 polypeptide comprises a polypeptide having the same amino acid sequence as the corresponding TALL-1 polypeptide derived from nature. Such native sequence TALL-1 polypeptides can be isolated from nature or can be produced by recombinant and/or synthetic means. The term "native sequence TALL-1 polypeptide" specifically encompasses naturally-occurring truncated or secreted forms (e.g., an extracellular domain sequence), naturally-occurring variant forms (e.g., alternatively spliced forms) and naturally-occurring allelic variants of the polypeptide. The term "TALL-1" includes those polypeptides described in Shu et al., GenBank Accession No. AF136293; WO98/1892 published May 7, 1998; EP 869,180 published October 7, 1998; WO98/27114 published June 25, 1998; WO99/12964 published March 18, 1999; WO99/33980 published July 8, 1999; Moore et al., supra; Schneider et al., supra; and Mukhopadhyay et al., supra.

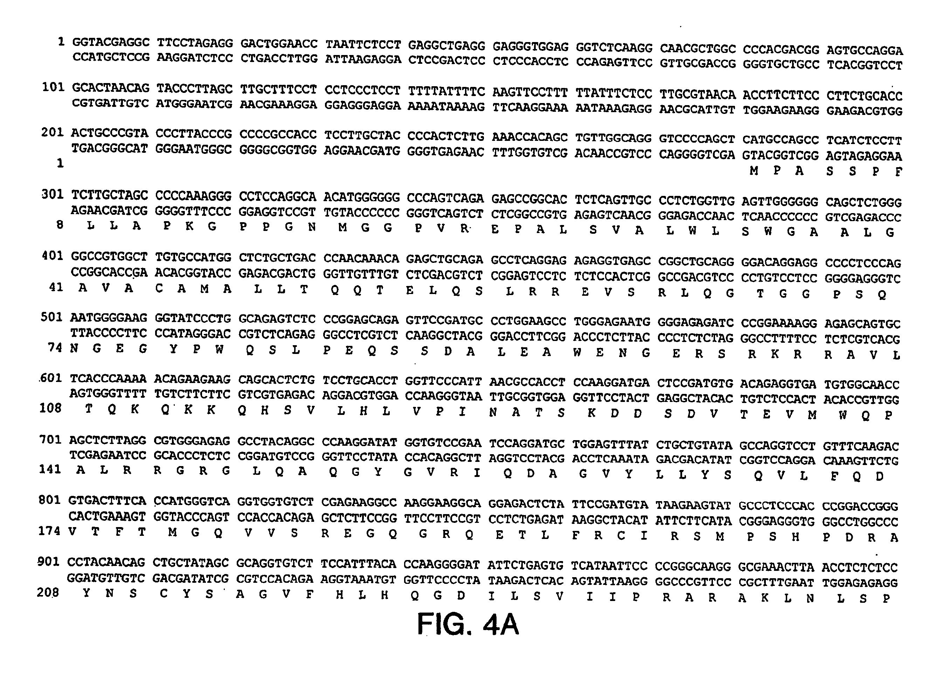

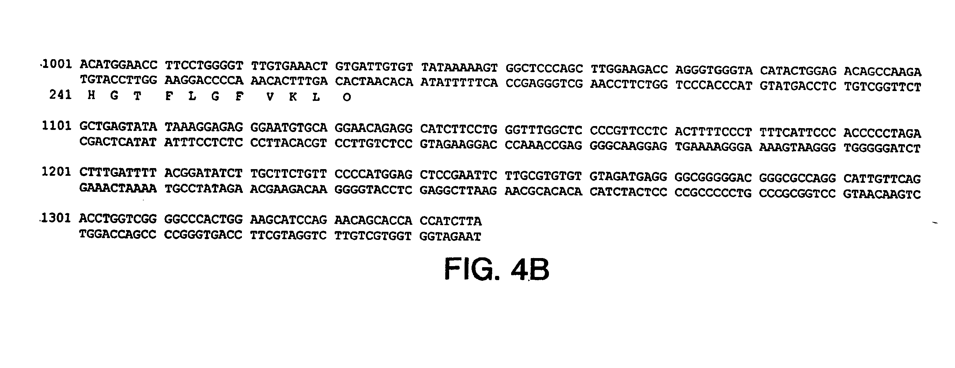

The terms "APRIL" or "APRIL polypeptide" when used herein encompass "native sequence APRIL polypeptides" and "APRIL variants". "APRIL" is a designation given to those polypeptides which are encoded by the nucleic acid molecules comprising the polynucleotide sequences shown in Figure 4 and variants thereof, nucleic acid molecules comprising the sequence shown in the Figure 4, and variants thereof as well as fragments of the above which have the biological activity of the native sequence APRIL. Variants of APRIL will preferably have at least 80%, more preferably, at least 90%, and even more preferably, at least 95% amino acid sequence identity with the native sequence APRIL polypeptide shown in Figure 4. A "native sequence" APRIL polypeptide comprises a polypeptide having the same amino acid sequence as the corresponding APRIL polypeptide derived from nature. Such native sequence APRIL polypeptides can be isolated from nature or can be produced by recombinant and/or synthetic means. The term "native sequence APRIL polypeptide" specifically encompasses naturally-occurring truncated or secreted forms (e.g., an extracellular domain sequence), naturally-occurring variant forms (e.g., alternatively spliced forms) and naturally-occurring allelic variants of the polypeptide. The term "APRIL" includes those polypeptides described in Hahne et al., J. Exp. Med., 188:1185-1190 (1998); GenBank Accession No. AF046888; WO 99/00518 published January 7, 1999; WO 99/12965 published March 18, 1999; WO 99/33980 published July 8, 1999; WO 97/33902 published September 18, 1997; WO 99/11791 published March 11, 1999; EP 911,633 published March 28, 1999; and WO99/5041 published October 7, 1999.

"Percent (%) amino acid sequence identity" with respect to the ligand or receptor polypeptide sequences identified herein is defined as the percentage of amino acid residues in a candidate sequence that are identical with the amino acid residues in such a ligand or receptor sequence identified herein, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are obtained as described below by using the sequence comparison computer program ALIGN-2, wherein the complete source code for the ALIGN-2 program is provided in the table below. The ALIGN-2 sequence comparison computer program was authored by Genentech, Inc. and the source code shown in the table below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in the table below. The ALIGN-2 program should be compiled for use on a UNIX operating system, preferably digital UNIX V4.0D. All sequence comparison parameters are set by the ALIGN-2 program and do not vary.

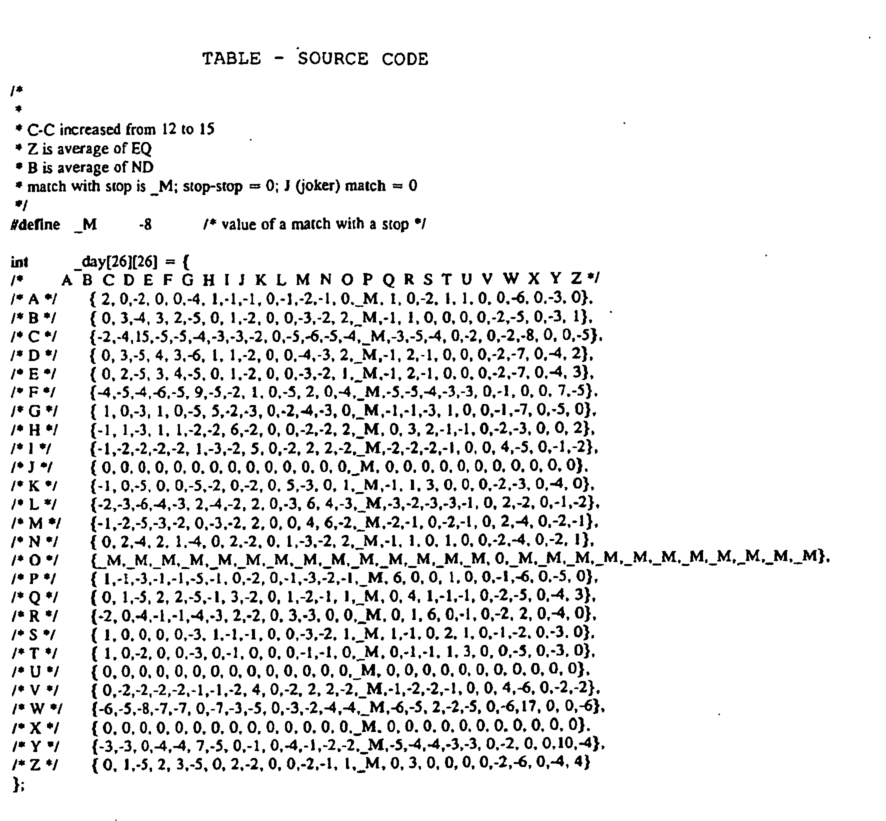

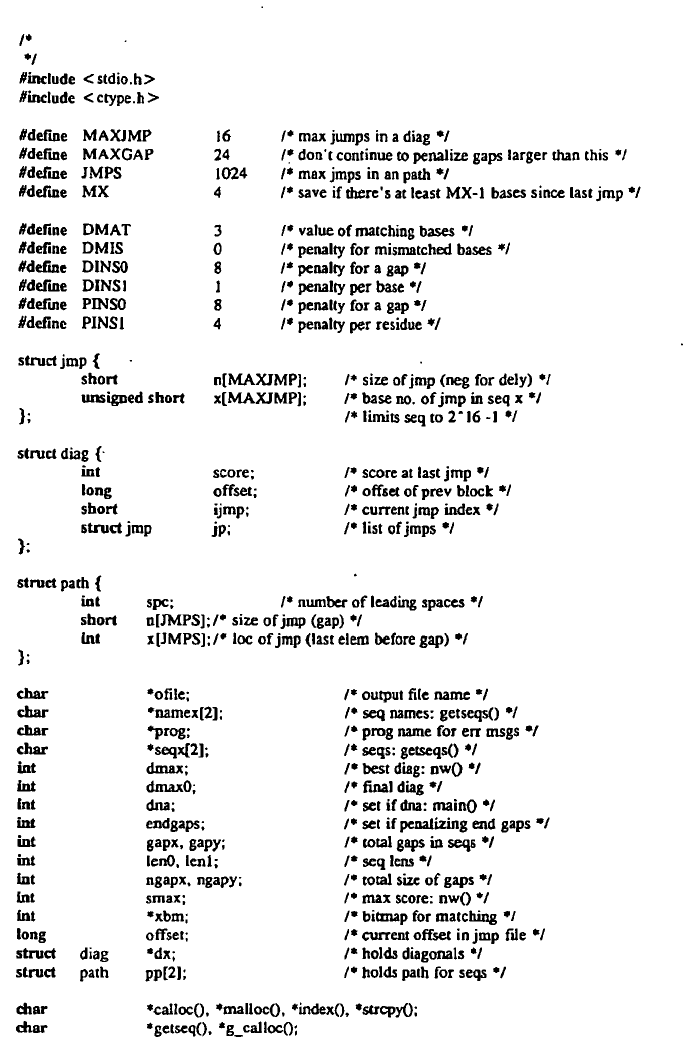

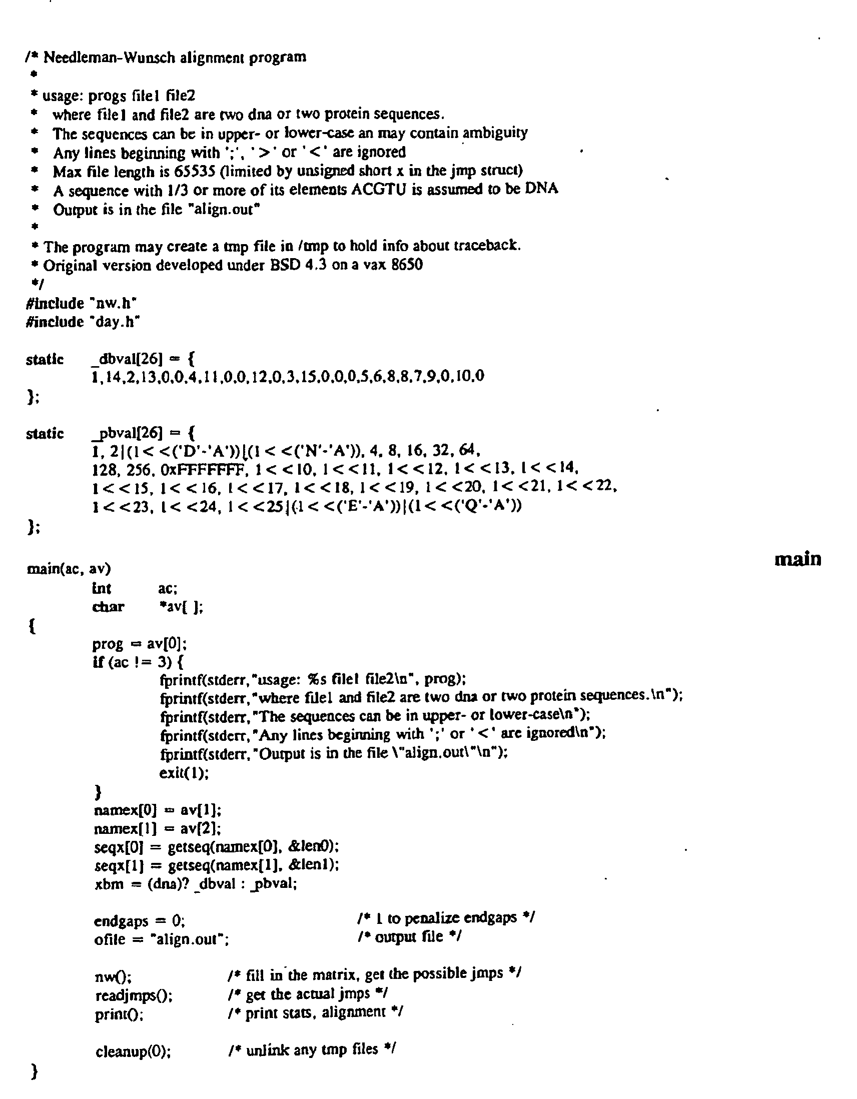

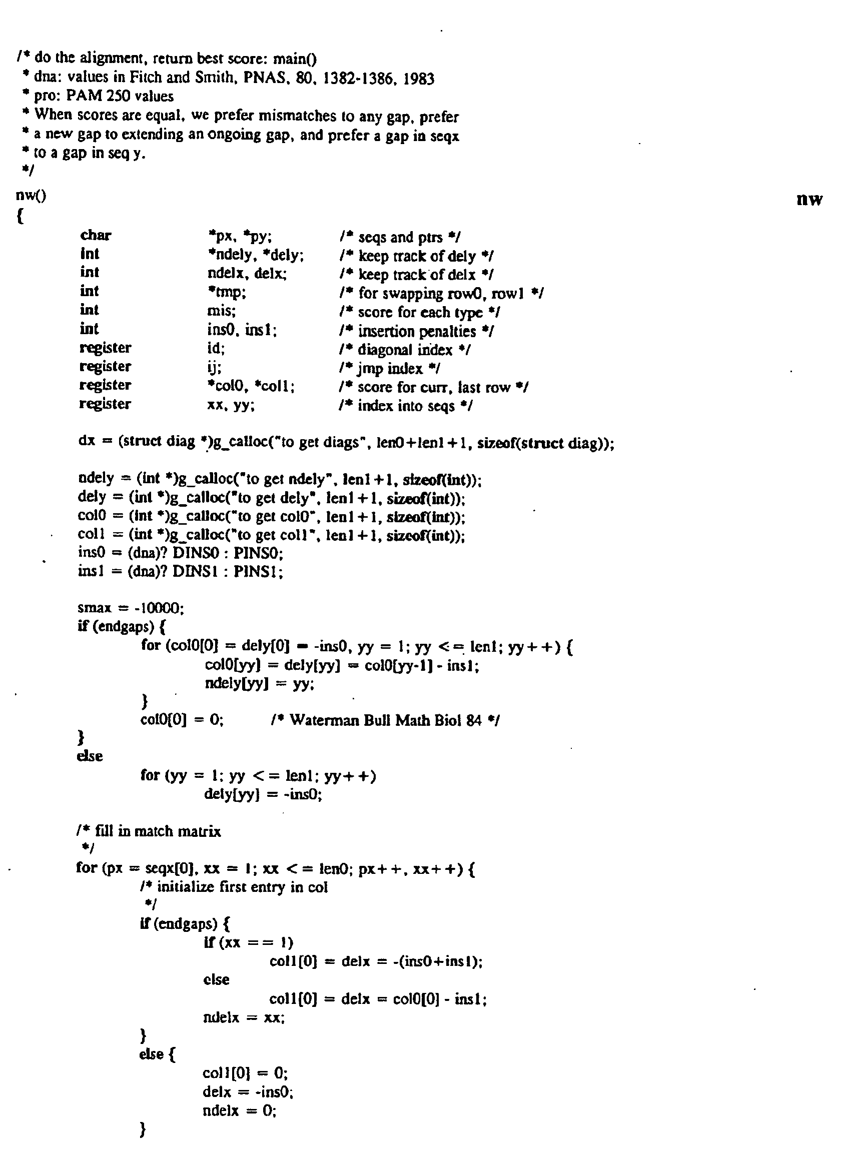

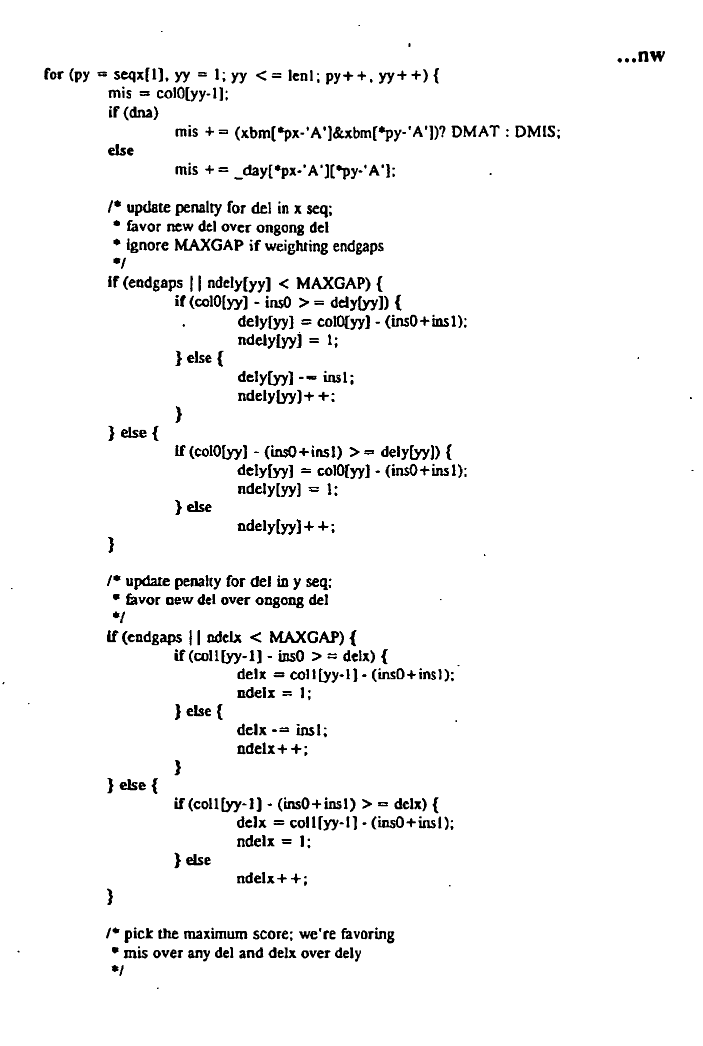

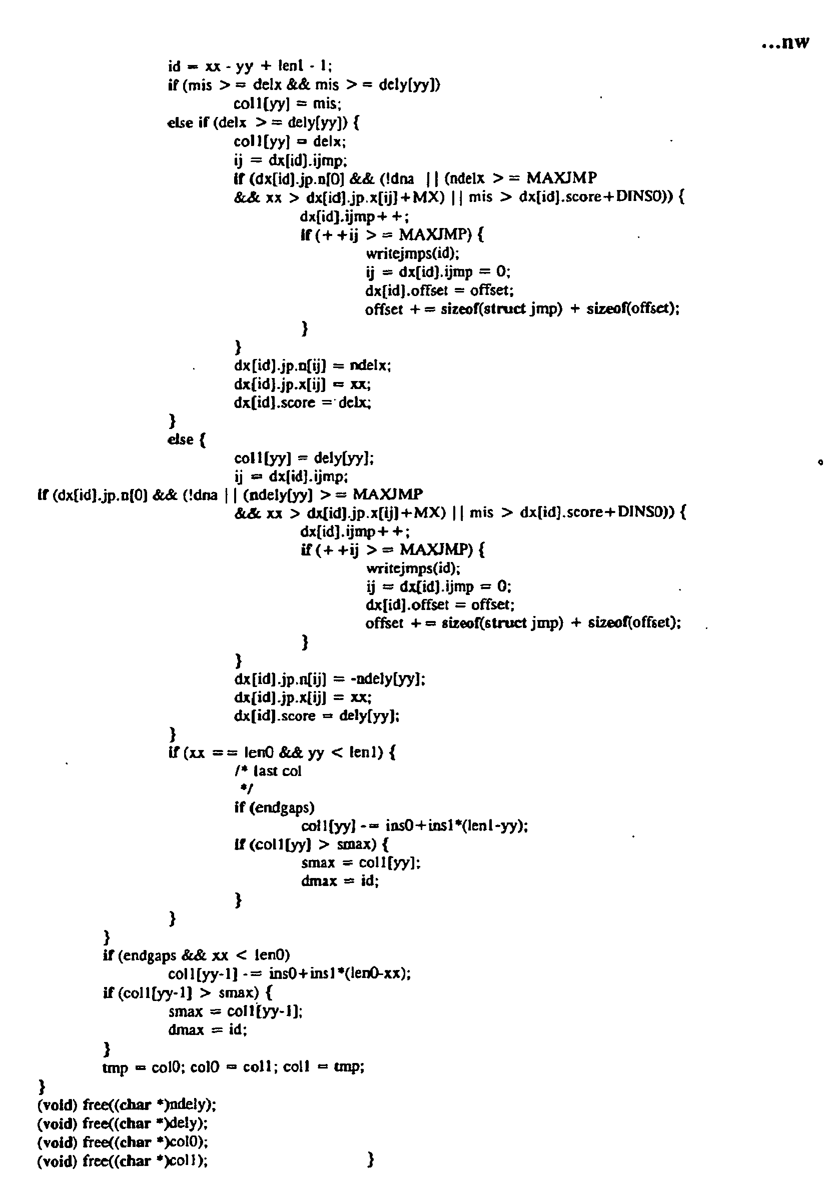

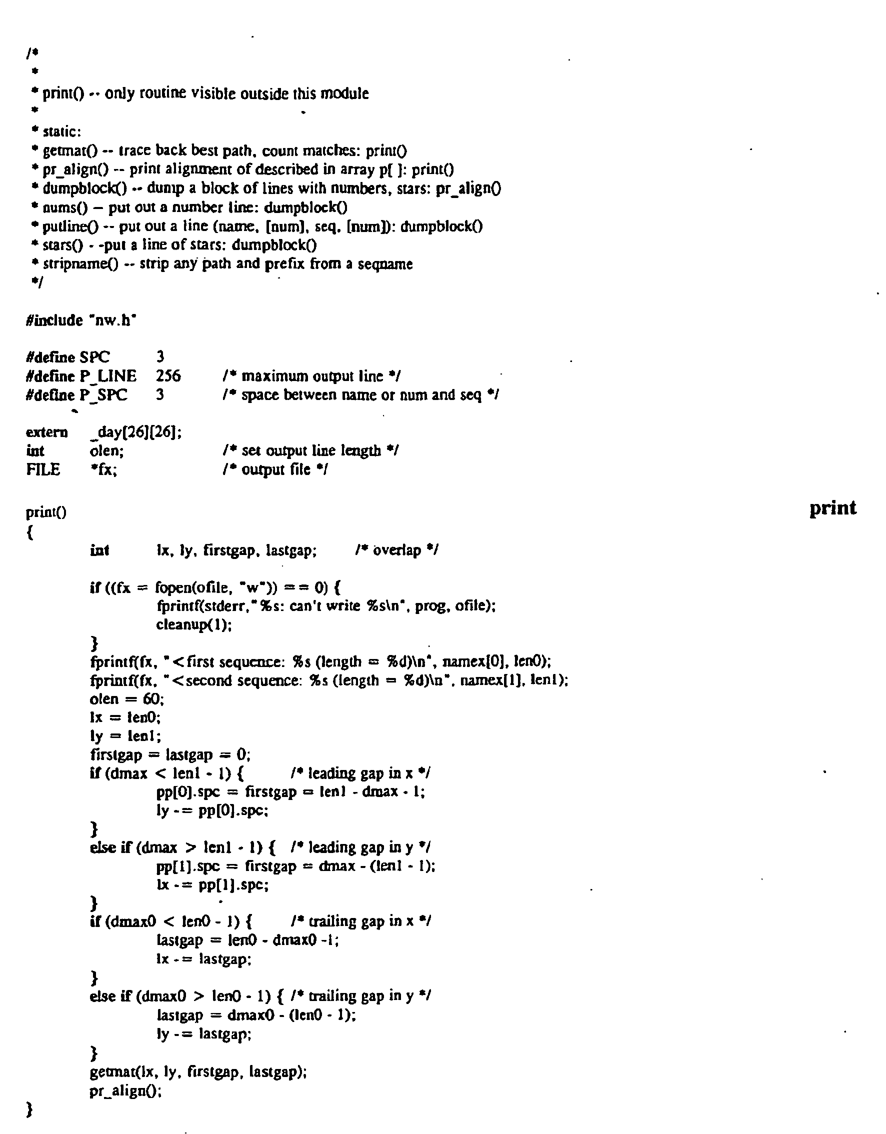

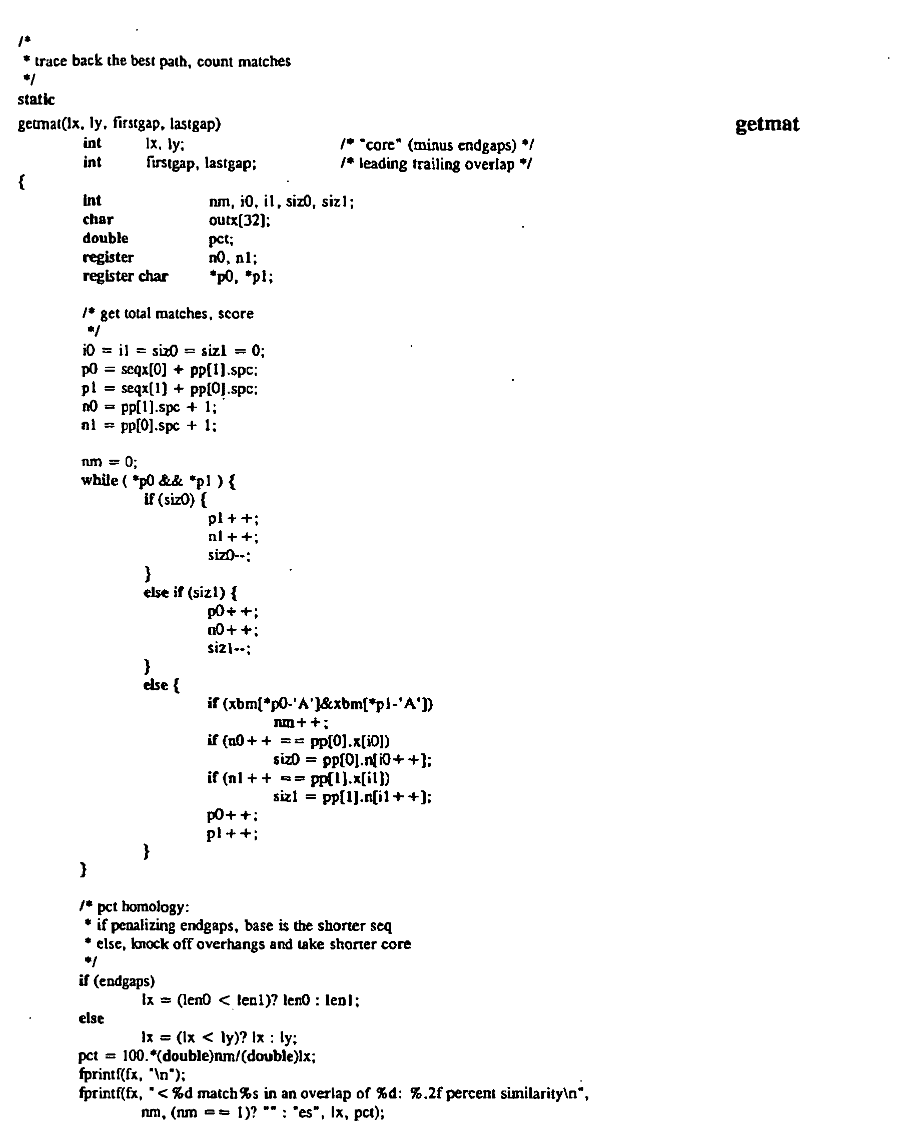

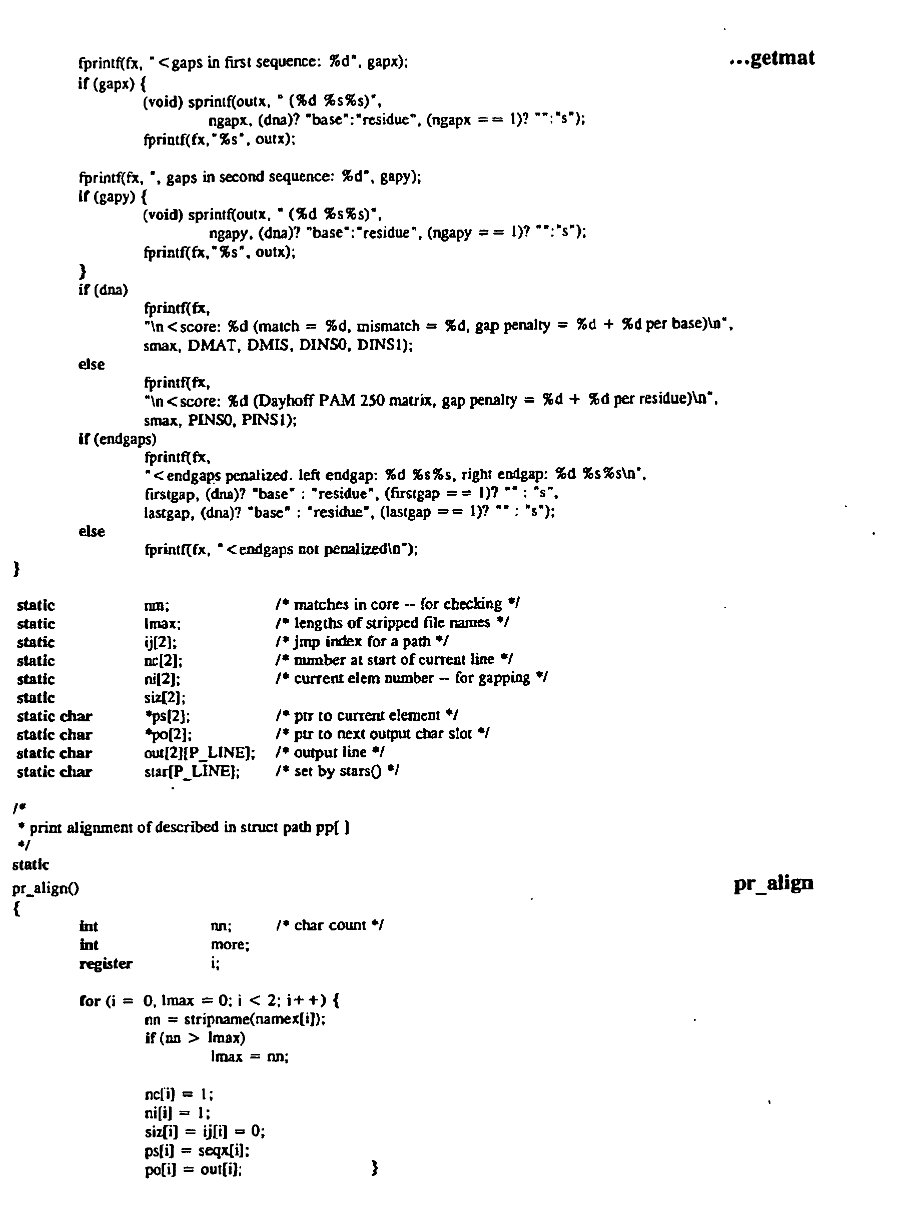

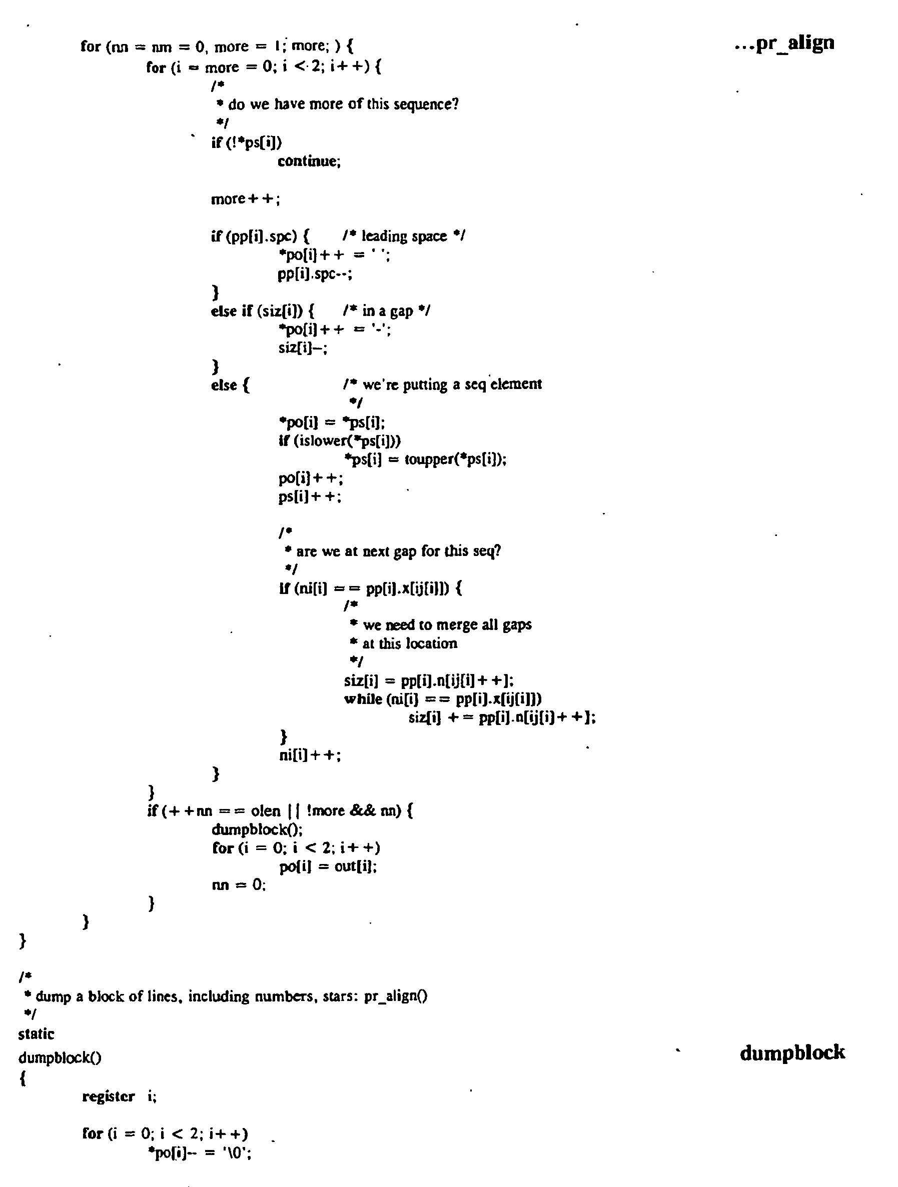

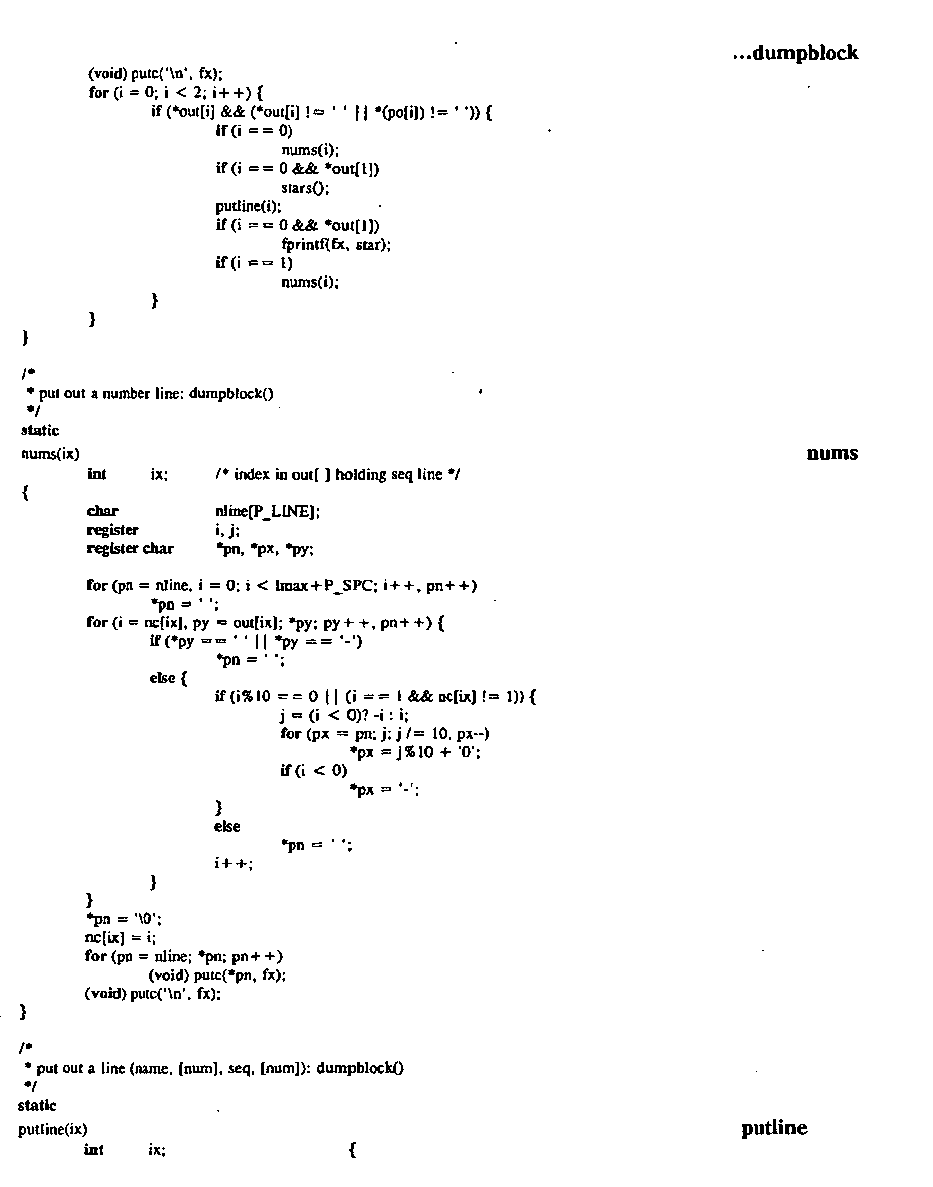

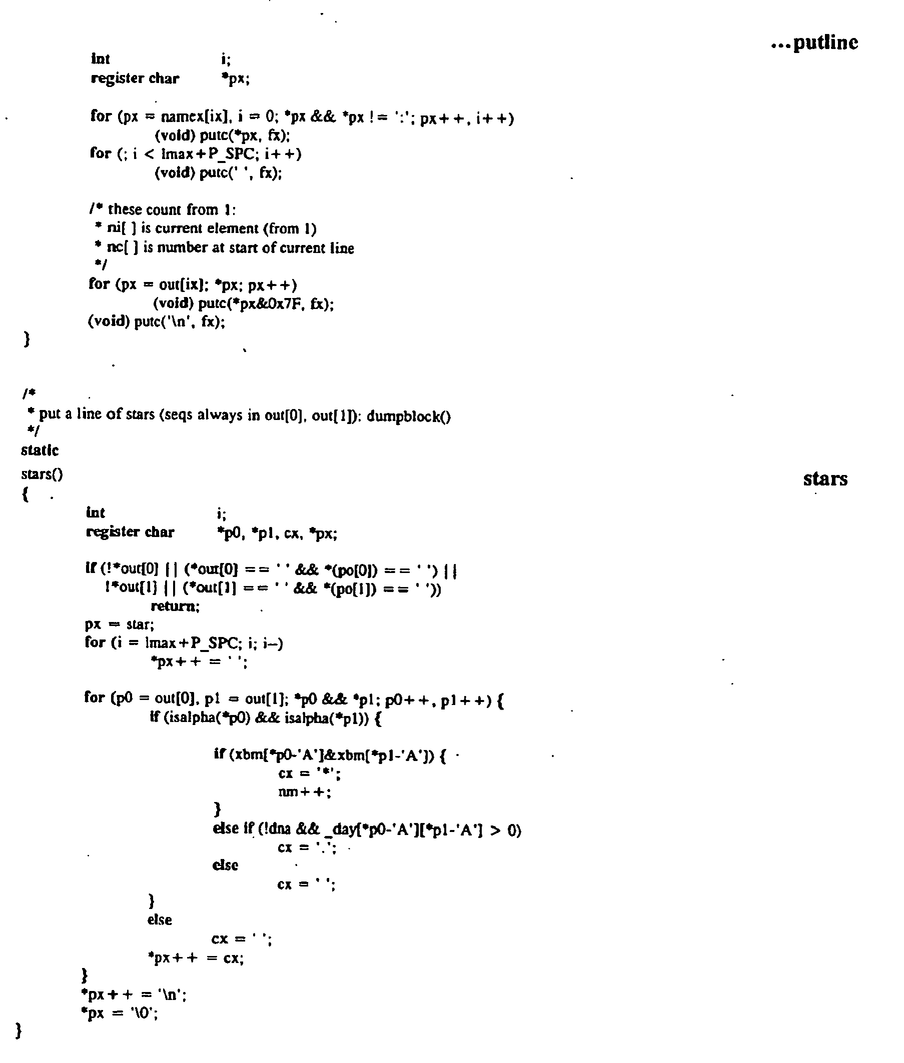

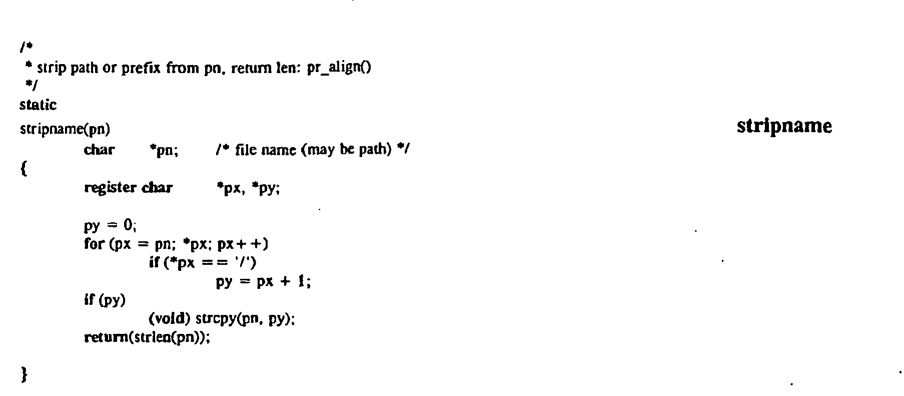

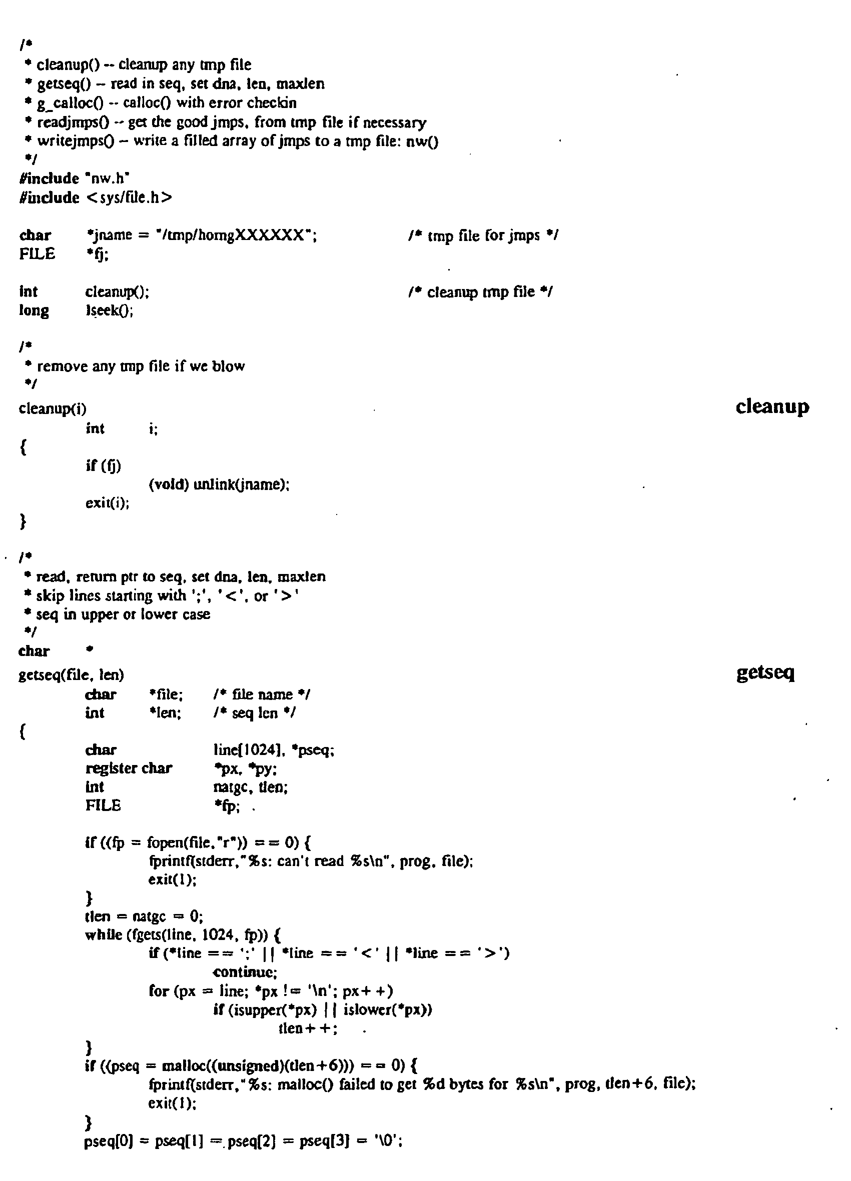

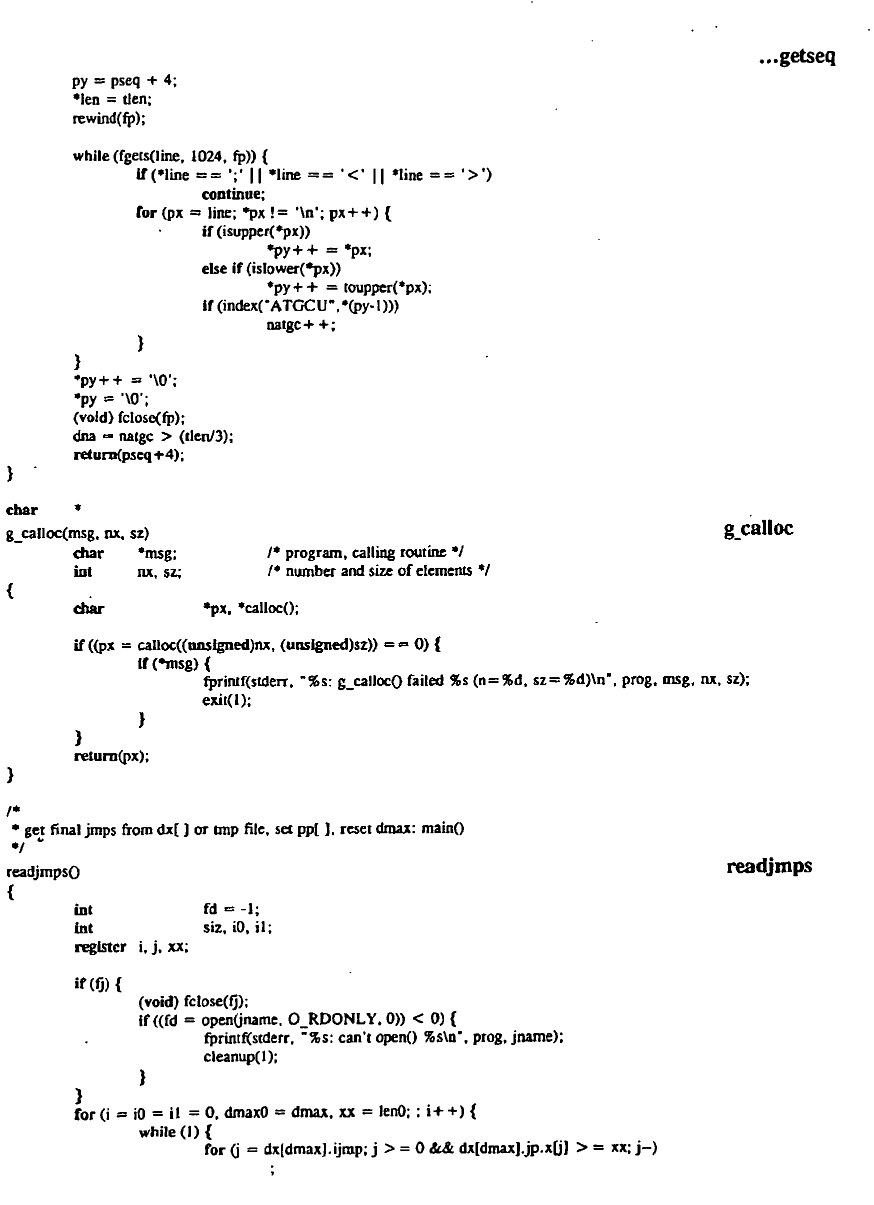

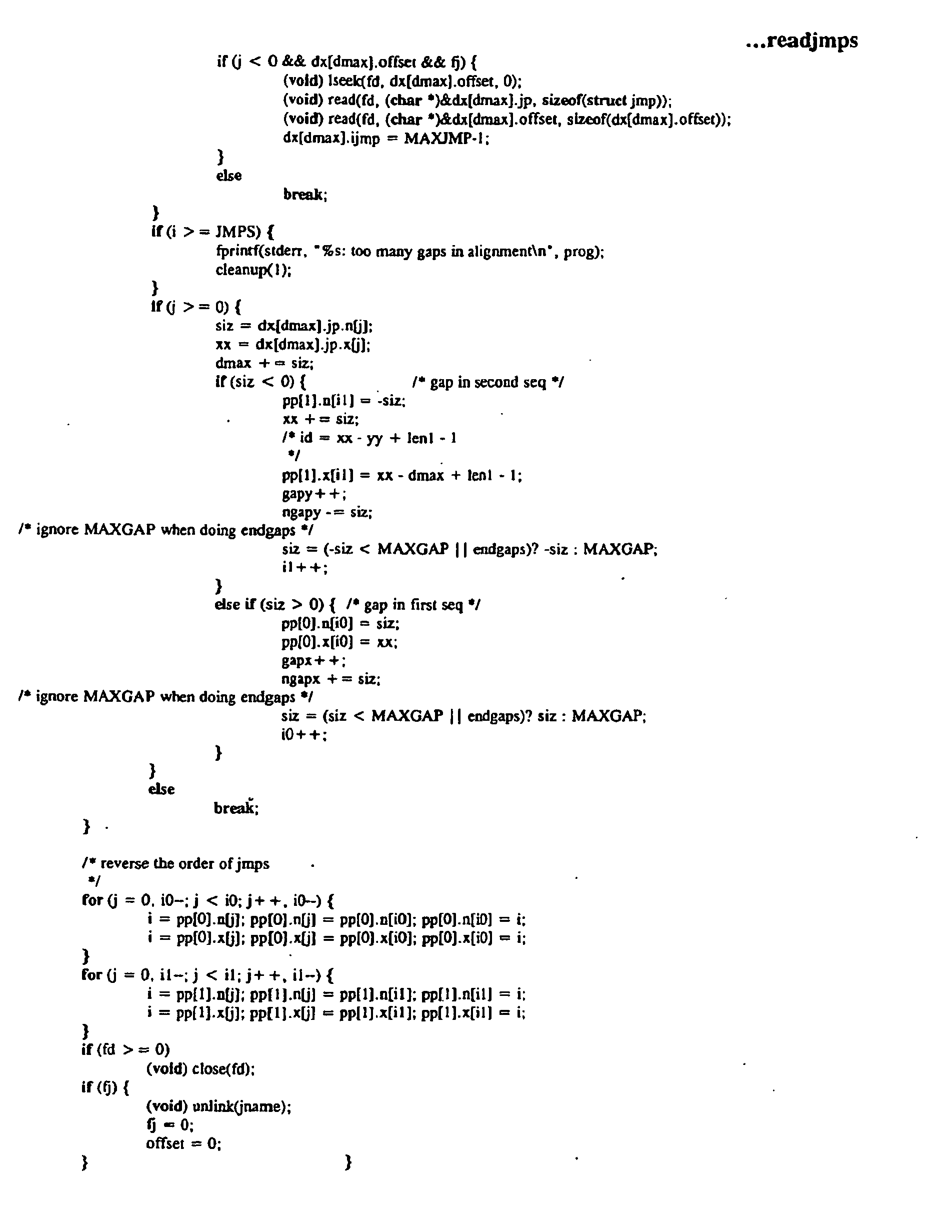

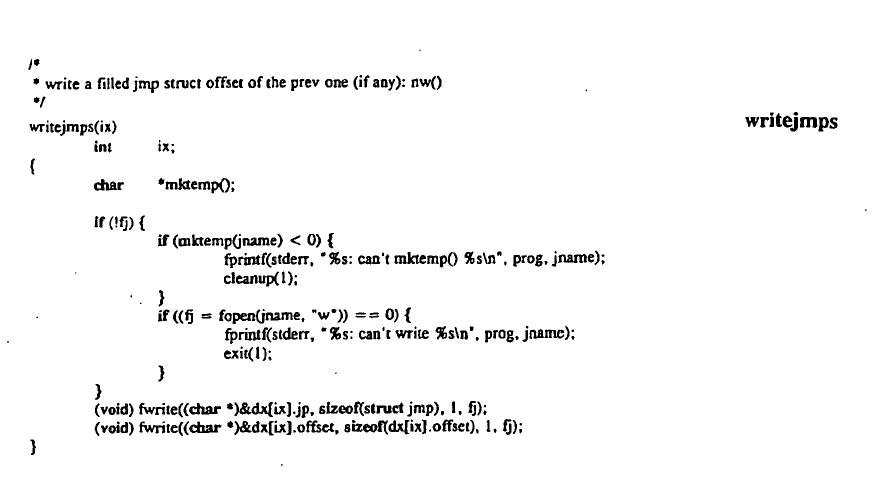

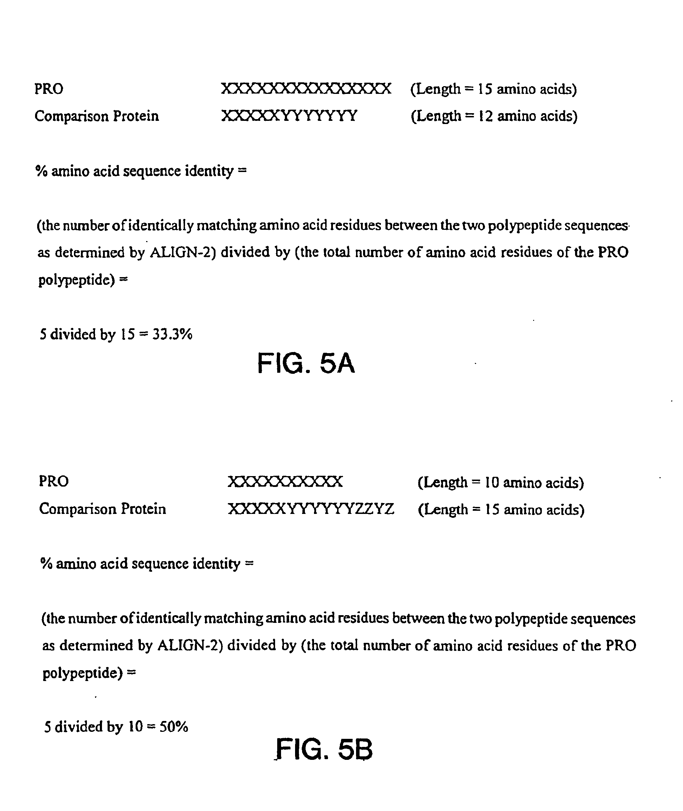

For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations, Figures 5A-5B demonstrate how to calculate the % amino acid sequence identity of the amino acid sequence designated "Comparison Protein" to the amino acid sequence designated "PRO".

Unless specifically stated otherwise, all % amino acid sequence identity values used herein are obtained as described above using the ALIGN-2 sequence comparison computer program. However, % amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from the NCBI internet web site. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

The term "epitope tagged" when used herein refers to a chimeric polypeptide comprising a polypeptide fused to a "tag polypeptide". The tag polypeptide has enough residues to provide an epitope against which an antibody can be made. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

The term "antagonist" is used in the broadest sense, and includes any molecule that partially or fully blocks, inhibits, or neutralizes one or more biological activities of TALL-1 polypeptide, APRIL polypeptide, or both TALL-1 and APRIL, *in vitro, in situ*, or *in vivo*. Examples of such biological activities of TALL-1 and APRIL polypeptides include binding of TALL-1 or APRIL to TACI or BCMA, activation of NF-KB and activation of proliferation and of Ig secretion by B cells, immune-related conditions such as rheumatoid arthritis, as well as those further reported in the literature. An antagonist may function in a direct or indirect manner. For instance, the antagonist may function to partially or fully block, inhibit or neutralize one or more biological activities of TALL-1 polypeptide, APRIL polypeptide, or both TALL-1 and APRIL, *in vitro, in situ*, or *in vivo* as a result of its direct binding to TALL-1, APRIL, TACI or BCMA. The antagonist may also function indirectly to partially or fully block, inhibit or neutralize one or more biological activities of TALL-1 polypeptide, APRIL polypeptide, or both TALL-1 and APRIL, *in vitro*, *in situ*, or *in vivo* as a result of, e.g., blocking or inhibiting another effector molecule. The antagonist molecule may comprise a "dual" antagonist activity wherein the molecule is capable of partially or fully blocking, inhibiting or neutralizing a biological activity of both TALL-1 and APRIL.

The term "agonist" is used in the broadest sense, and includes any molecule that partially or fully enhances, stimulates or activates one or more biological activities of TACI polypeptide, BCMA polypeptide, or both TACI and BCMA, *in vitro*, *in situ*, or *in vivo.* Examples of such biological activities of TACI and BCMA include activation of NF-KB, induction of immunoglobulin production and secretion, and cell proliferation, as well as those further reported in the literature. An agonist may function in a direct or indirect manner. For instance, the agonist may function to partially or fully enhance, stimulate or activate one or more biological activities of TACI polypeptide, BCMA polypeptide, or both TACI and BCMA, *in vitro*, *in situ*, or *in vivo* as a result of its direct binding to TACI or BCMA, which causes receptor activation or signal transduction. The agonist may also function indirectly to partially or fully enhance, stimulate or activate one or more biological activities of TACI polypeptide, BCMA polypeptide, or both TACI and BCMA, *in vitro, in situ,* or *in vivo* as a result of, e.g., stimulating another effector molecule which then causes TACI or BCMA receptor activation or signal transduction. It is contemplated that an agonist may act as an enhancer molecule which functions indirectly to enhance or increase TACI or BCMA activation or activity. For instance, the agonist may enhance activity of endogenous TALL-1 or APRIL in a mammal. This could be accomplished, for example, by pre-complexing TACI or BCMA or by stabilizing complexes of the respective ligand with the TACI or BCMA receptor (such as stabilizing native complex formed between TALL-1 and TACI or APRIL and TACI).

The term "TALL-1 antagonist," or "APRIL antagonist" refers to any molecule that partially or fully blocks, inhibits, or neutralizes a biological activity of TALL-1 or APRIL, respectively, or both TALL-1 and APRIL, and include, but are not limited to, soluble forms of TACI receptor or BCMA receptor such as an extracellular domain sequence of TACI or BCMA, TACI receptor immunoadhesins, BCMA receptor immnoadhesins, TACI receptor fusion proteins, BCMA receptor fusion proteins, covalently modified forms of TACI receptor, covalently modified forms of BCMA receptor, TACI variants, BCMA variants, TACI receptor antibodies, BCMA receptor antibodies, TALL-1 antibodies, and APRIL antibodies. To determine whether a TALL-1 antagonist molecule partially or fully blocks, inhibits or neutralizes a biological activity of TALL-1 or APRIL, assays may be conducted to assess the effect(s) of the antagonist molecule on, for example, binding of TALL-1 or APRIL to TACI or to BCMA, or NF-KB activation by the respective ligand. Such assays may be conducted in known *in vitro* or *in vivo* assay formats, for instance, in cells expressing BCMA and/or TACI. Preferably, the TALL-1 antagonist employed in the methods described herein will be capable of blocking or neutralizing at least one type of TALL-1 activity, which may optionally be determined in assays such as described herein. To determine whether an APRIL antagonist molecule partially or fully blocks, inhibits or neutralizes a biological activity of TALL-1 or APRIL, assays may be conducted to assess the effect(s) of the antagonist molecule on, for example, binding of TALL-1 or APRIL to TACI or to BCMA, or NF-KB activation by the ligand. Such assays may be conducted in known *in vitro* or *in vivo* formats, for instance, using cells transfected with TACI or BCMA (or both TACI and BCMA) (preferably transfected at relatively low levels). Preferably, the APRIL antagonist employed in the methods described herein will be capable of blocking or neutralizing at least one type of APRIL activity, which may optionally be determined in a binding assay or an IgM-production assay. Optionally, a TALL-1 antagonist or APRIL antagonist will be capable of reducing or inhibiting binding of either TALL-1 or APRIL (or both TALL-1 and APRIL) to TACI or to BCMA by at least 50%, preferably, by at least 90%, more preferably by at least 99%, and most preferably, by 100%, as compared to a negative control molecule, in a binding assay. The TALL-1 antagonist or APRIL antagonist may comprise antibodies which will competitively inhibit the binding of another ligand or antibody to TACI or BCMA. Methods for determining antibody specificity and affinity by competitive inhibition are known in the art [see, e.g., Harlow et al., Antibodies:A Laboratory Manual, Cold Spring Harbor Laboratory Press, Cold Spring Harbor, NY (1998); Colligan et al., Current Protocols in Immunology, Green Publishing Assoc., NY (1992; 1993); Muller, Meth. Enzym., 92:589-601 (1983).

The term "TACI agonist" or "BCMA agonist" refers to any molecule that partially or fully enhances, stimulates or activates a biological activity of TACI or BCMA, respectively, or both TACI and BCMA, and include, but are not limited to, anti-TACI receptor antibodies and anti-BCMA receptor antibodies. To determine whether a TACI agonist molecule partially or fully enhances, stimulates, or activates a biological activity of TACI or BCMA, assays may be conducted to assess the effect(s) of the agonist molecule on, for example, PBLs or TACI or BCMA-transfected cells. Such assays may be conducted in known *in vitro* or *in vivo* assay formats. Preferably, the TACI agonist employed in the methods described herein will be capable of enhancing or activating at least one type of TACI activity, which may optionally be determined in assays such as described herein. To determine whether a BCMA agonist molecule partially or fully enhances, stimulates, or activates a biological activity of TACI or BCMA, assays may be conducted to assess the effect(s) of the antagonist molecule on, for example, an activity of APRIL or TACI. Such assays may be conducted in *in vitro* or *in vivo* formats, for instance, using PBLs or TACI or BCMA-transfected cells. Preferably, the TACI agonist or BCMA agonist will be capable of stimulating or activating TACI or BCMA, respectively, to the extent of that accomplished by the native ligand for the TACI or BCMA receptors.

The term "antibody" is used in the broadest sense and specifically covers, for example, single monoclonal antibodies against TALL-1, APRIL, TACI, or BCMA, antibody compositions with polyepitopic specificity, single chain antibodies, and fragments of antibodies.

The term "monoclonal antibody" as used herein refers to an antibody obtained from a population of substantially homogeneous antibodies, *i.e*., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Methods of making chimeric antibodies are known in the art.

"Humanized" forms of non-human (*e.g*., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementarity-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones et al., Nature, 321:522-525 (1986); Reichmann et al., Nature, 332:323-329 (1988); and Presta, Curr. Op. Struct. Biol., 2:593-596 (1992). The humanized antibody includes a PRIMATIZED^{™} antibody wherein the antigen-binding region of the antibody is derived from an antibody produced by immunizing macaque monkeys with the antigen of interest. Methods of making humanized antibodies are known in the art.

Human antibodies can also be produced using various techniques known in the art, including phage-display libraries. Hoogenboom and Winter, J. Mol. Biol., 227:381 (1991); Marks et al., J. Mol. Biol., 222:581 (1991). The techniques of Cole et al. and Boerner *et al*. are also available for the preparation of human monoclonal antibodies. Cole et al., Monoclonal Antibodies and Cancer Therapy, Alan R. Liss, p. 77 (1985); Boerner et al., J. Immunol., 147(1):86-95 (1991).

"Isolated," when used to describe the various proteins disclosed herein, means protein that has been identified and separated and/or recovered from a component of its natural environment. Contaminant components of its natural environment are materials that would typically interfere with diagnostic or therapeutic uses for the protein, and may include enzymes, hormones, and other proteinaceous or non-proteinaceous solutes. In preferred embodiments, the protein will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated protein includes protein *in situ* within recombinant cells, since at least one component of the protein natural environment will not be present. Ordinarily, however, isolated protein will be prepared by at least one purification step.

"Treatment" or "therapy" refer to both therapeutic treatment and prophylactic or preventative measures.

"Mammal" for purposes of treatment or therapy refers to any animal classified as a mammal, including humans, domestic and farm animals, and zoo, sports, or pet animals, such as dogs, horses, cats, cows, etc. Preferably, the mammal is human.

"TALL-1 -related pathological condition" and "APRIL-related pathological condition" refer to pathologies or conditions associated with abnormal levels of expression or activity of TALL-1 or APRIL, respectively, in excess of, or less than, levels of expression or activity in normal healthy mammals, where such excess or diminished levels occur in a systemic, localized, or particular tissue or cell type or location in the body. TALL-1 -related pathological conditions and APRIL-related pathological conditions include acute and chronic immune related diseases and cancer.

The terms "cancer", "cancerous", and "malignant" refer to or describe the physiological condition in mammals that is typically characterized by unregulated cell growth. Examples of cancer include but are not limited to, carcinoma including adenocarcinoma, lymphoma, blastoma, melanoma, sarcoma, and leukemia. More particular examples of such cancers include squamous cell cancer, small-cell lung cancer, non-small cell lung cancer, gastrointestinal cancer, Hodgkin's and non-Hodgkin's lymphoma, pancreatic cancer, glioblastoma, cervical cancer, ovarian cancer, liver cancer such as hepatic carcinoma and hepatoma, bladder cancer, breast cancer, colon cancer, colorectal cancer, endometrial carcinoma, salivary gland carcinoma, kidney cancer such as renal cell carcinoma and Wilms' tumors, basal cell carcinoma, melanoma, prostate cancer, vulval cancer, thyroid cancer, testicular cancer, esophageal cancer, and various types of head and neck cancer. Optionally, the cancer will express, or have associated with the cancer cell, TALL-1, APRIL, TACI or BCMA. By way of example, colon, lung and melanoma cancers have been reported in the literature to express APRIL. The preferred cancers for treatment herein include lymphoma, leukemia and myeloma, and subtypes thereof, such as Burkitt's lymphoma, multiple myeloma, acute lymphoblastic or lymphocytic leukemia, non-Hodgkin's and Hodgkin's lymphoma, and acute myeloid leukemia.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are autoimmune diseases, immune-mediated inflammatory diseases, non-immune-mediated inflammatory diseases, infectious diseases, and immunodeficiency diseases. Examples of immune-related and inflammatory diseases, some of which are immune or T cell mediated, which can be treated according to the invention include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjogren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory and fibrotic lung diseases such as inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft-versus-host-disease. Infectious diseases include AIDS (HIV infection), hepatitis A, B, C, D, and E, bacterial infections, fungal infections, protozoal infections and parasitic infections.

"Autoimmune disease" is used herein in a broad, general sense to refer to disorders or conditions in mammals in which destruction of normal or healthy tissue arises from humoral or cellular immune responses of the individual mammal to his or her own tissue constituents. Examples include, but are not limited to, lupus erythematous, thyroiditis, rheumatoid arthritis, psoriasis, multiple sclerosis, autoimmune diabetes, and inflammatory bowel disease (IBD).

The term "cytotoxic agent" as used herein refers to a substance that inhibits or prevents the function of cells and/or causes destruction of cells. The term is intended to include radioactive isotopes (e.g. I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of disease. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, e.g. paclitaxel (Taxol, Bristol-Myers Squibb Oncology, Princeton, NJ), and doxetaxel (Taxotere, Rhône-Poulenc Rorer, Antony, Rnace), toxotere, methotrexate, cisplatin, melphalan, CPT-11, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), melphalan and other related nitrogen mustards. Also included in this definition are hormonal agents that act to regulate or inhibit hormone action such as tamoxifen and onapristone.

A "growth inhibitory agent" when used herein refers to a compound or composition which inhibits growth of a cell, either *in vitro* or *in vivo*. Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Mutakami et al. (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors; platelet-growth factor; transforming growth factors (TGFs) such as TGF-α and TGF-β; insulin-like growth factor-I and -II; erythropoietin (EPO); osteoinductive factors; interferons such as interferon-α, -β, and -gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-2, IL-3, IL-4, IL-5, IL-6, IL-7, IL-8, IL-9, IL-11, IL-12; and other polypeptide factors including LIF and kit ligand (KL). As used herein, the term cytokine includes proteins from natural sources or from recombinant cell culture and biologically active equivalents of the native sequence cytokines.

### II. Methods and Materials

Generally, methods for modulating TALL-1, APRIL, TACI, and/or BCMA activity in mammalian cells comprise exposing the cells to a desired amount of antagonist or agonist which affects TALL-1 or APRIL interaction with TACI or BCMA. Preferably, the amount of antagonist or agonist employed will be an amount effective to affect the binding and/or activity of the respective ligand or respective receptor to achieve a therapeutic effect. This can be accomplished *in vivo* or *ex vivo* in accordance, for instance, with the methods described below and in the Examples. Exemplary conditions or disorders to be treated with such TALL-1 antagonists or APRIL antagonists include conditions in mammals clinically referred to as autoimmune diseases, including but not limited to rheumatoid arthritis, multiple sclerosis, psoriasis, and lupus or other pathological conditions in which B cell response(s) in mammals is abnormally upregulated such as cancer. As shown in the Examples below, TACI immunoadhesin molecules substantially inhibited arthritic disease induced by immunization with type-II collagen, reduced joint inflammation and formation of anti-collagen antibodies, prevented bone and cartilage destruction and blocked stimulation of autoreactive T cells. These results implicate TACI in T cell-mediated autoimmunity, and suggest that blocking or inhibiting TACI interactions with TALL-1 and/or APRIL may have therapeutic utility for autoimmune diseases such as RA. Exemplary conditions or disorders to be treated with TACI agonists or BCMA agonists include immunodeficiency and cancer.

Diagnostic methods are also described herein. For instance, the antagonists or agonists may be employed to detect the respective ligands (TALL-1 or APRIL) or receptors (TACI or BCMA) in mammals known to be or suspected of having a TALL-1 - related pathological condition or APRIL-related pathological condition. The antagonist or agonist molecule may be used, e.g., in immunoassays to detect or quantitate TALL-1 or APRIL in a sample. A sample, such as cells obtained from a mammal, can be incubated in the presence of a labeled antagonist or agonist molecule, and detection of the labeled antagonist or agonist bound in the sample can be performed. Such assays, including various clinical assay procedures, are known in the art, for instance as described in voller et al., Immunoassays, University Park, 1981.

### A. MATERIALS

The antagonists and agonists which can be employed in the methods include, but are not limited to, soluble forms of TACI and BCMA receptors, TACI receptor immunoadhesins and BCMA receptor immunoadhesins, fusion-proteins comprising TACI or BCMA, covalently modified forms of TACI or BCMA, TACI receptor variants and BCMA receptor variants, TACI or BCMA receptor antibodies, and TALK-1 or APRIL antibodies. Various techniques that can be employed for making the antagonists and agonists are described below.

Generally, the compositions of the invention may be prepared using recombinant techniques known in the art. The description below relates to methods of producing such polypeptides by culturing host cells transformed or transfected with a vector containing the encoding nucleic acid and recovering the polypeptide from the cell culture. (See, e.g., Sambrook et al., Molecular Cloning: A Laboratory Manual (New York: Cold Spring Harbor Laboratory Press, 1989); Dieffenbach et al., PCR Primer:A Laboratory Manual (Cold Spring Harbor Laboratory Press, 1995)).

The nucleic acid (e.g., cDNA or genomic DNA) encoding the desired polypeptide may be inserted into a replicable vector for further cloning (amplification of the DNA) or for expression. Various vectors are publicly available. The vector components generally include, but are not limited to, one or more of the following: a signal sequence, an origin of replication, one or more marker genes, an enhancer element, a promoter, and a transcription termination sequence, each of which is described below. Optional signal sequences, origins of replication, marker genes, enhancer elements and transcription terminator sequences that may be employed are known in the art and described in further detail in WO97/25428.

Expression and cloning vectors usually contain a promoter that is recognized by the host organism and is operably linked to the encoding nucleic acid sequence. Promoters are untranslated sequences located upstream (5') to the start codon of a structural gene (generally within about 100 to 1000 bp) that control the transcription and translation of a particular nucleic acid sequence, to which they are operably linked. Such promoters typically fall into two classes, inducible and constitutive. Inducible promoters are promoters that initiate increased levels of transcription from DNA under their control in response to some change in culture conditions, e.g., the presence or absence of a nutrient or a change in temperature. At this time a large number of promoters recognized by a variety of potential host cells are well known. These promoters are operably linked to the encoding DNA by removing the promoter from the source DNA by restriction enzyme digestion and inserting the isolated promoter sequence into the vector.

Promoters suitable for use with prokaryotic and eukaryotic hosts are known in the art, and are described in further detail in WO97/25928.

Construction of suitable vectors containing one or more of the above-listed components employs standard ligation techniques. Isolated plasmids or DNA fragments are cleaved, tailored, and re-ligated in the form desired to generate the plasmids required. For analysis to confirm correct sequences in plasmids constructed, the ligation mixtures can be used to transform E. coli K12 strain 294 (ATCC 31,446) and successful transformants selected by ampicillin or tetracycline resistance where appropriate. Plasmids from the transformants are prepared, analyzed by restriction endonuclease digestion, and/or sequenced using standard techniques known in the art. [See, e.g., Messing et al., Nucleic Acids Res., 9:309 (1981); Maxam et al., Methods in Enzymology, 65:499 (1980)].

Expression vectors that provide for the transient expression in mammalian cells of the encoding DNA may be employed. In general, transient expression involves the use of an expression vector that is able to replicate efficiently in a host cell, such that the host cell accumulates many copies of the expression vector and, in turn, synthesizes high levels of a desired polypeptide encoded by the expression vector [Sambrook et al., supra]. Transient expression systems, comprising a suitable expression vector and a host cell, allow for the convenient positive identification of polypeptides encoded by cloned DNAs, as well as for the rapid screening of such polypeptides for desired biological or physiological properties.

Other methods, vectors, and host cells suitable for adaptation to the synthesis of the desired polypeptide in recombinant vertebrate cell culture are described in Gething et al., Nature, 293:620-625 (1981); Mantei et al., Nature, 281:40-46 (1979); EP 117,060; and EP 117,058.

Suitable host cells for cloning or expressing the DNA in the vectors herein include prokaryote, yeast, or higher eukaryote cells. Suitable prokaryotes for this purpose include but are not limited to eubacteria, such as Gram-negative or Gram-positive organisms, for example, *Enterobacteriaceae* such as Escherichia, e.g., *E. coli, Enterobacter, Erwinia, Klebsiella, Proteus, Salmonella,* e.g., *Salmonella typhimurium,* Serratia, e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as *P. aeruginosa,* and *Streptomyces.* Preferably, the host cell should secrete minimal amounts of proteolytic enzymes.

In addition to prokaryotes, eukaryotic microbes such as filamentous fungi or yeast are suitable cloning or expression hosts for vectors. Suitable host cells for the expression of glycosylated polypeptide are derived from multicellular organisms. Examples of all such host cells are described further in WO97/25428.

Host cells are transfected and preferably transformed with the above-described expression or cloning vectors and cultured in nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ and electroporation. Successful transfection is generally recognized when any indication of the operation of this vector occurs within the host cell.

Transformation means introducing DNA into an organism so that the DNA is replicable, either as an extrachromosomal element or by chromosomal integrant. Depending on the host cell used, transformation is done using standard techniques appropriate to such cells. The calcium treatment employing calcium chloride, as described in Sambrook et al., supra, or electroporation is generally used for prokaryotes or other cells that contain substantial cell-wall barriers. Infection with Agrobacterium tumefaciens is used for transformation of certain plant cells, as described by Shaw et al., Gene, 23:315 (1983) and WO 89/05859 published 29 June 1989. In addition, plants may be transfected using ultrasound treatment as described in WO 91/00358 published 10 January 1991.

For mammalian cells without such cell walls, the calcium phosphate precipitation method of Graham and van der Eb, Virology, 52:456-457 (1978) may be employed. General aspects of mammalian cell host system transformations have been described in U.S. Pat. No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Prokaryotic cells may be cultured in suitable culture media as described generally in Sambrook et al., supra. Examples of commercially available culture media include Ham's F10 (Sigma), Minimal Essential Medium ("MEM", Sigma), RPMI-1640 (Sigma), and Dulbecco's Modified Eagle's Medium ("DMEM", Sigma). Any such media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin^{™} drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

In general, principles, protocols, and practical techniques for maximizing the productivity of mammalian cell cultures can be found in Mammalian Cell Biotechnology: A Practical Approach, M. Butler, ed. (IRL Press, 1991).

The expressed polypeptides may be recovered from the culture medium as a secreted polypeptide, although may also may be recovered from host cell lysates when directly produced without a secretory signal. If the polypeptide is membrane-bound, it can be released from the membrane using a suitable detergent solution (e.g. Triton-X 100) or its extracellular region may be released by enzymatic cleavage.

When the polypeptide is produced in a recombinant cell other than one of human origin, it is free of proteins or polypeptides of human origin. However, it is usually necessary to recover or purify the polypeptide from recombinant cell proteins or polypeptides to obtain preparations that are substantially homogeneous. As a first step, the culture medium or lysate may be centrifuged to remove particulate cell debris. The following are procedures exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; and protein A Sepharose columns to remove contaminants such as IgG.

TACI receptor variants and BCMA receptor variants are contemplated for use. Such variants can be prepared using any suitable technique in the art. The receptor variants can be prepared by introducing appropriate nucleotide changes into the receptor DNA, and/or by synthesis of the desired receptor polypeptide. Those skilled in the art will appreciate that amino acid changes may alter post-translational processes of the receptor, such as changing the number or position of glycosylation sites or altering the membrane anchoring characteristics.

Variations in the native sequence receptor or in various domains of the receptors described herein, can be made, for example, using any of the techniques and guidelines for conservative and non-conservative mutations set forth, for instance, in U.S. Patent No. 5,364,934. Variations may be a substitution, deletion or insertion of one or more codons encoding the receptor that results in a change in the amino acid sequence of the receptor as compared with the native sequence receptor. Optionally the variation is by substitution of at least one amino acid with any other amino acid in one or more of the domains of the receptor. Guidance in determining which amino acid residue may be inserted, substituted or deleted without adversely affecting the desired activity may be found by comparing the sequence of the receptor with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

TACI or BCMA polypeptide fragments are provided herein. Such fragments may be truncated at the N-terminus or C-terminus, or may lack internal residues, for example, when compared with a full-length native protein. Certain fragments lack amino acid residues that are not essential for a desired biological activity of the receptor polypeptide. Optionally, the TACI or BCMA polypeptide fragments comprise ECD deletion variants in which one or more amino acid residues have been deleted from the N-terminus or C-terminus of the receptor ECD sequence. Preferably, such ECD deletion variants have at least one biological activity as compared to the native receptor sequence.

TACI or BCMA fragments may be prepared by any of a number of conventional techniques. Desired peptide fragments may be chemically synthesized. An alternative approach involves generating receptor fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR.

Conservative substitutions of interest are shown in Table 1 under the heading of preferred substitutions. If such substitutions result in a change in biological activity, then more substantial changes, denominated exemplary substitutions in Table 1, or as further described below in reference to amino acid classes, are introduced and the products screened.

**Table 1**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the receptor polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the receptor variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

Soluble forms of TACI receptors or BCMA receptors may also be employed as antagonists in the methods. Such soluble forms of TACI or BCMA may comprise or consist of extracellular domains of the respective receptor (and lacking transmembrane and intracellular domains of the respective receptor). The extracellular domain sequences themselves of TACI or BCMA may be used as antagonists, or may be further modified as described below (such as by fusing to an immunoglobulin, epitope tag or leucine zipper). Certain extracellular domain regions of TACI and BCMA have been described in the literature and may be further delineated using techniques known to the skilled artisan. Those skilled in the art will be able to select, without undue experimentation, a desired extracellular domain sequence of either TACI or BCMA to employ as an antagonist.

Immunoadhesin molecules are further contemplated for use in the methods herein. TACI receptor immunoadhesins may comprise various forms of TACI, such as the full length polypeptide as well as soluble forms of the receptor which comprise an extracellular domain (ECD) sequence or a fragment of the ECD sequence. The molecule may comprise a fusion of the TACI receptor with an immunoglobulin or a particular region of an immunoglobulin. For a bivalent form of the immunoadhesin, such a fusion could be to the Fc region of an IgG molecule. The Ig fusions preferably include the substitution of a soluble (transmembrane domain deleted or inactivated) form of the receptor polypeptide in place of at least one variable region within an Ig molecule. The immunoglobulin fusion may include the hinge, CH2 and CH3, or the hinge, CH1, CH2 and CH3 regions of an IgG1 molecule. For the production of immunoglobulin fusions, see also US Patent No. 5,428,130 issued June 27, 1995 and Chamow et al., TIBTECH, 14: 52-60 (1996).

The simplest and most straightforward immunoadhesin design combines the binding domain(s) of the adhesin (e.g. the extracellular domain (ECD) of a receptor) with the Fc region of an immunoglobulin heavy chain. Ordinarily, when preparing the immunoadhesins described herein, nucleic acid encoding the binding domain of the adhesin will be fused C-terminally to nucleic acid encoding the N-terminus of an immunoglobulin constant domain sequence, however N-terminal fusions are also possible.

Typically, in such fusions the encoded chimeric polypeptide will retain at least functionally active hinge, Cₐ2 and C_{H}3 domains of the constant region of an immunoglobulin heavy chain. Fusions are also made to the C-terminus of the Fc portion of a constant domain, or immediately N-terminal to the C_{K}1 of the heavy chain or the corresponding region of the light chain. The precise site at which the fusion is made is not critical; particular sites are well known and may be selected in order to optimize the biological activity, secretion, or binding characteristics of the immunoadhesin.

The adhesion sequence may be fused to the N-terminus of the Fc region of immunoglobulin G₁ (IgG₁). It is possible to fuse the entire heavy chain constant region to the adhesin sequence. However, more preferably, a sequence beginning in the hinge region just upstream of the papain cleavage site which defines IgG Fc chemically (*i.e*. residue 216, taking the first residue of heavy chain constant region to be 114), or analogous sites of other immunoglobulins is used in the fusion. The adhesin amino acid sequence may be used to (a) the hinge region and C_{H}2 and C_{H}3 or (b) the C_{H}1, hinge, C_{H}2 and C_{H}3 domains, of an IgG heavy chain.

For bispecific immunoadhesin, the immunoadhesins are assembled as multimers, and particularly as heterodimers or heterotetramers. Generally, these assembled immunoglobulins will have known unit structures. A basic four chain structural unit is the form in which IgG, IgD, and IgE exist. A four chain unit is repeated in the higher molecular weight immunoglobulins; IgM generally exists as a pentamer of four basic units held together by disulfide bonds. IgA globulin, and occasionally IgG globulin, may also exist in multimeric form in serum. In the case of multimer, each of the four units may be the same or different.

Various exemplary assembled immunoadhesins are schematically diagrammed below:
(a) AC_{L}-AC_{L};
(b) AC_{H}-(AC_{H}, AC_{L}'-AC_{H}, AC_{L}-V_{H}C_{H}, or V_{L}C_{L}-AC_{H});
(c) AC_{L}-AC_{H}-(AC_{L}-AC_{E}, AC_{L}-V_{H}C_{H}, V_{L}C_{L}-AC_{H}, or V_{L}C_{L}-V_{H}C_{H})
(d) AC_{L}-V_{H}C_{H}-(AC_{H}, or AC_{L}-V_{H}C_{H}, or V_{L}C_{L}-AC_{H});
(e) V_{L}C_{L}-AC_{H}-(AC_{L}-V_{H}C_{H}, or V_{L}C_{L}-AC_{H}); and
(f) (A-Y)ₙ-(V_{L}C_{L}-V_{H}C_{H})₂,
wherein each A represents identical or different adhesin amino acid sequences;
V_{L} is an immunoglobulin light chain variable domain;
V_{H} is an immunoglobulin heavy chain variable domain;
C_{L} is an immunoglobulin light chain constant domain;
C_{H} is an immunoglobulin heavy chain constant domain;
n is an integer greater than 1;
Y designates the residue of a covalent cross-linking agent.

In the interests of brevity, the foregoing structures only show key features; they do not indicate joining (J) or other domains of the immunoglobulins, nor are disulfide bonds shown. However, where such domains are required for binding activity, they shall be constructed to be present in the ordinary locations which they occupy in the immunoglobulin molecules.

Alternatively, the adhesin sequences can be inserted between immunoglobulin heavy chain and light chain sequences, such that an immunoglobulin comprising a chimeric heavy chain is obtained. In this embodiment, the adhesin sequences are fused to the 3' end of an immunoglobulin heavy chain in each arm of an immunoglobulin, either between the hinge and the C_{H}2 domain, or between the C_{H}2 and C_{H}3 domains. Similar constructs have been reported by Hoogenboom et al., Mol. Immunol., 28:1027-1037 (1991).

Although the presence of an immunoglobulin light chain is not required in the immunoadhesins of the present invention, an immunoglobulin light chain might be present either covalently associated to an adhesin-immunoglobulin heavy chain fusion polypeptide, or directly fused to the adhesin. In the former case, DNA encoding an immunoglobulin light chain is typically coexpressed with the DNA encoding the adhesin-immunoglobulin heavy chain fusion protein. Upon secretion, the hybrid heavy chain and the light chain will be covalently associated to provide an immunoglobulin-like structure comprising two disulfide-linked immunoglobulin heavy chain-light chain pairs. Methods suitable for the preparation of such structures are, for example, disclosed in U.S. Patent No. 4,816,567, issued 28 March 1989.

Immunoadhesins are most conveniently constructed by fusing the cDNA sequence encoding the adhesin portion in-frame to an immunoglobulin cDNA sequence. However, fusion to genomic immunoglobulin fragments can also be used (see, e.g. Aruffo et al., Cell, 61:1303-1313 (1990); and Stamenkovic et al., Cell, 66:1133-1144 (1991)). The latter type of fusion requires the presence of Ig regulatory sequences for expression. cDNAs encoding IgG heavy-chain constant regions can be isolated based on published sequences from cDNA libraries derived from spleen or peripheral blood lymphocytes, by hybridization or by polymerase chain reaction (PCR) techniques. The cDNAs encoding the "adhesin" and the immunoglobulin parts of the immunoadhesin are inserted in tandem into a plasmid vector that directs efficient expression in the chosen host cells.

Examples of such soluble ECD sequences include polypeptides comprising amino acids 2-166 of the TACI sequence shown in Figure 1, and described further in the Examples below. The TACI receptor immunoadhesin can be made according to any of the methods described in the art.

BCMA receptor immunoadhesins can be similarly constructed. Examples of soluble ECD sequences for use in constructing BCMA immunoadhesins may include polypeptides comprising amino acids 5-51 of the BCMA sequence shown in Figure 2, and described further in the Examples below.

The TACI or BCMA receptor may be covalently modified by linking the receptor polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689: 4,301,144; 4,670,417; 4,791,192 or 4,179,337. Such pegylated forms of the TACI or BCMA receptor may be prepared using techniques known in the art.

Leucine zipper forms of these molecules are also described . "Leucine zipper" is a term in the art used to refer to a leucine rich sequence that enhances, promotes, or drives dimerization or trimerization of its fusion partner (e.g., the sequence or molecule to which the leucine zipper is fused or linked to). Various leucine zipper polypeptides have been described in the art. See, e.g., Landschulz et al., Science, 240:1759 (1988); US Patent 5,716,805; WO 94/10308; Hoppe et al., FEBS Letters, 344;1991 (1994); Maniatis et al., Nature, 341:24 (1989). Those skilled in the art will appreciate that a leucine zipper sequence may be fused at either the 5' or 3' end of the TACI or BCMA receptor molecule.

The TACI or BCMA polypeptides may also be modified in a way to form chimeric molecules by fusing the receptor polypeptide to another, heterologous polypeptide or amino acid sequence. Preferably, such heterologous polypeptide or amino acid sequence is one which acts to oligimerize the chimeric molecule: Such a chimeric molecule may comprise a fusion of the TACI or BCMA receptor polypeptide with a tag polypeptide which provides an epitope to which an anti-tag antibody can selectively bind. The epitope tag is generally placed at the amino- or carboxyl-terminus of the receptor polypeptide. The presence of such epitope-tagged forms of the receptor can be detected using an antibody against the tag polypeptides. Also, provision of the epitope tag enables the receptor to be readily purified by affinity purification using an anti-tag antibody or another type of affinity matrix that binds to the epitope tag. Various tag polypeptides and their respective antibodies are well know in the art. Examples include poly-histidine (poly-his) or poly-histidine-glycine (poly-his-gly) tags; the flu HA tag polypeptide and its antibody 12CA5 [Field et al., Mol. Cell. Biol., 8;2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

Anti-TACI receptor antibodies, anti-BCMA receptor antibodies, anti-TALL-1 antibodies, or anti-APRIL antibodies may also be employed in the methods. Examples of such molecules include neutralizing or blocking antibodies which can preferably inhibit binding of TALL-1 or APRIL to the TACI or to the BCMA receptors. The anti-TACI antibodies, anti-BCMA, anti-TALL-1, or anti-APRIL antibodies may be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro*.

The immunizing agent will typically include the TACI or BCMA polypeptide, or TALL-1 or APRIL polypeptide, or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against TACI, BCMA, TALL-1 or APRIL. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980). Optionally, the anti-TACI, anti-BCMA, anti-TALL-1, or anti-APRIL antibodies will have a binding affinity of at least 10nM, preferably, of at least 5nM, and more preferably, of at least 1nM for the respective receptor or ligand, as determined in a binding assay.

After the desired hybridoma cells are identified, the clones may be subcloned by limiting dilution procedures and grown by standard methods [Goding, sura]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

Antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy- and light-chain constant domains in place of the homologous murine sequences (U.S. Patent No. 4,816,567; Morrison, et al., Proc. Natl Acad. Sci. USA, 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

A humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987)). Another method uses a particular framework derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immnol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the CDR residues are directly and most substantially involved in influencing antigen binding.

Alternatively, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci. USA, 90:2551 (1993); Jakobovits et al., Nature, 362:255-258 (1993); Bruggermann et al., Year in Immuno., 7;33 (1993); and Duchosal et al., Nature, 355:258 (1992). Human antibodies can also be derived from phage-display libraries (Hoogenboom et al., J. Mol. Biol., 227:381 (1991); Marks et al., J. Mol. Biol., 222:581-597 (1991); Vaughan et al., Nature Biotech, 14:309 (1996)).

As described in the Examples below, particular anti-APRIL antibodies have been prepared. Four of these antibodies, 3C6.4.2, 5G8.2.2, 5E8.7.4, and 5E11.1.2, have been deposited with ATCC, and have been assigned accession numbers PTA-1347, PTA-1345, PTA-1344 and PTA-1346. The anti-APRIL antibodies disclosed herein may have the same biological characteristics as the monoclonal antibodies secreted by the hybridoma call lines deposited under accession numbers PTA-1347, PTA-1345, PTA-1344 or PTA-1346. The term "biological characteristics" is used to refer to the *in vitro* and/or *in vivo* activities or properties of the monoclonal antibody, such as the ability to bind to APRIL or to substantially block or reduce TACI or BCMA binding or activation by APRIL. Optionally, the anti-APRIL monoclonal antibody will have the same blocking activity as the 3C6.4.2 antibody, as determined by its ability to block binding of APRIL to TACI or BCMA. Optionally, the anti-APRIL monoclonal antibody will bind to the same epitope as the 5G8.2.2 antibody, the 5E8.7.4 antibody, the 3C6.4.2 antibody or the 5E11.1.2 antibody disclosed in the Examples below. Such epitope binding property can be determined for instance in a competitive 64 inhibition binding assay, which techniques are known in the art. Such an anti-APRIL antibody will preferably competitively inhibit binding of either the 5G8.2.2 antibody, the 5E8.7.4 antibody, the 3C6.4.2 antibody or the 5E11.1.2 antibody to APRIL. It is contemplated that chimeric or humanized anti-APRIL antibodies can be constructed (such as by using the techniques described above) using or incorporating selected fragments or domain sequences from any of the afore-mentioned deposited anti-APRIL antibodies.

### B. ASSAY METHODS

Ligand/receptor binding studies may be carried out in any known assay method, such as competitive binding assays, direct and indirect sandwich assays, and immunoprecipitation assays. Cell-based assays and animal models can be used to further understand the interaction between the ligands and receptors identified herein and the development and pathogenesis of the conditions and diseases referred to herein.

In one approach, mammalian cells may be transfected with the ligands or receptors described herein, and the ability of the agonists or antagonists to stimulate or inhibit binding or activity is analyzed. Suitable cells can be transfected with the desired gene, and monitored for activity. Such transfected cell lines can then be used to test the ability of antagonist(s) or agonist(s) to inhibit or stimulate, for example, to modulate B-cell proliferation or Ig secretion. Cells transfected with the coding sequence of the genes identified herein can further be used to identify drug candidates for the treatment of immune related diseases or cancer.

In addition, primary cultures derived from transgenic animals can be used in the cell-based assays. Techniques to derive continuous cell lines from transgenic animals are well known in the art. [see, e.g., Small et al., Mol. Cell. Biol., 5:642-648 (1985)].

One suitable cell based assay is the addition of epitope-tagged ligand (e.g., AP or Flag) to cells that have or express the respective receptor, and analysis of binding (in presence or absence or prospective antagonists) by FACS staining with anti-tag antibody. In another assay, the ability of an antagonist to inhibit the TALL-1 or APRIL induced proliferation of B cells is assayed. B cells or cell lines are cultured with TALL-1 or APRIL in the presence or absence or prospective antagonists and the proliferation of B cells can be measured by ³H-thymidine incorporation or cell number.

The results of the cell based *in vitro* assays can be further verified using *in vivo* animal models. A variety of well known animal models can be used to further understand the role of the agonists and antagonists identified herein in the development and pathogenesis of for instance, immune related disease or cancer, and to test the efficacy of the candidate therapeutic agents. The *in vivo* nature of such models makes them particularly predictive of responses in human patients. Animal models of immune related diseases include both non-recombinant and recombinant (transgenic) animals. Non-recombinant animal models include, for example, rodent, e.g., murine models. Such models can be generated by introducing cells into syngeneic mice using standard techniques, e.g. subcutaneous injection, tail vein injection, spleen implantation, intraperitoneal implantation, and implantation under the renal capsule.

Animal models, for example, for graft-versus-host disease are known. Graft-versus-host disease occurs when immunocompetent cells are transplanted into immunosuppressed or tolerant patients. The donor cells recognize and respond to host antigens. The response can vary from life threatening severe inflammation to mild cases of diarrhea and weight loss. Graft-versus-host disease models provide a means of assessing T cell reactivity against MHC antigens and minor transplant antigens. A suitable procedure is described in detail in Current Protocols in Immunology, unit 4.3.

An animal model for skin allograft rejection is a means of testing the ability of T cells to mediate *in vivo* tissue destruction which is indicative of and a measure of their role in anti-viral and tumor immunity. The most common and accepted models use murine tail-skin grafts. Repeated experiments have shown that skin allograft rejection is mediated by T cells, helper T cells and killer-effector T cells, and not antibodies. [Auchincloss, H. Jr. and Sachs, D. H., Fundamental Immunology, 2nd ed., W. E. Paul ed., Raven Press, NY, 1989, 889-992]. A suitable procedure is described in detail in Current Protocols in Immunology, unit 4.4. Other transplant rejection models which can be used to test the compositions of the invention are the allogeneic heart transplant models described by Tanabe, M. et al., Transplantation, (1994) 58:23 and Tinubu, S. A. et al., J. Immunol., (1994) 4330-4338.

Animal models for delayed type hypersensitivity provides an assay of cell mediated immune function as well. Delayed type hypersensitivity reactions are a T cell mediated in vivo immune response characterized by inflammation which does not reach a peak until after a period of time has elapsed after challenge with an antigen. These reactions also occur in tissue specific autoimmune diseases such as multiple sclerosis (MS) and experimental autoimmune encephalomyelitis (EAE, a model for MS). A suitable procedure is described in detail in Current Protocols in Immunology, unit 4.5.

An animal model for arthritis is collagen-induced arthritis. This model shares clinical, histological and immunological characteristics of human autoimmune rheumatoid arthritis and is an acceptable model for human autoimmune arthritis. Mouse and rat models are characterized by synovitis, erosion of cartilage and subchondral bone. The compounds of the invention can be tested for activity against autoimmune arthritis using the protocols described in Current Protocols in Immunology, above, units 15.5. See also the model using a monoclonal antibody to CD18 and VLA-4 integrins described in Issekutz, A. C. et al., Immunology, (1996) 88:569. Example 10 below further describes a CIA murine model.

A model of asthma has been described in which antigen-induced airway hyper-reactivity, pulmonary eosinophilia and inflammation are induced by sensitizing an animal with ovalbumin and then challenging the animal with the same protein delivered by aerosol. Several animal models (guinea pig, rat, non-human primate) show symptoms similar to atopic asthma in humans upon challenge with aerosol antigens. Murine models have many of the features of human asthma. Suitable procedures to test the compositions of the invention for activity and effectiveness in the treatment of asthma are described by Wolyniec, W. W. et al., Am. J. Respir. Cell Mol. Biol., (1998) 18:777 and the references cited therein.

Additionally, the compositions of the invention can be tested on animal models for psoriasis like diseases. The compounds of the invention can be tested in the scid/scid mouse model described by Schon, M. P. et al., Nat. Med., (1997) 3:183, in which the mice demonstrate histopathologic skin lesions resembling psoriasis. Another suitable model is the human skin/scid mouse chimera prepared as described by Nickoloff, B. J. et al., Am. J. Path., (1995) 146:580.

Various animal models are well known for testing anti-cancer activity of a candidate therapeutic composition. These include human tumor xenografting into athymic nude mice or scid/scid mice, or genetic murine tumor models such as p53 knockout mice.

Recombinant (transgenic) animal models can be engineered by introducing the coding portion of the molecules identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, e.g. baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (e.g., Van der Putten et al., Proc. Natl. Acad, Sci. USA, 82, 6148-615 [1985]); gene targeting in embryonic stem cells (Thompson et al., Cell, 56, 313-321 [1989]); electroporation of embryos (Lo, Mol. Cel. Biol., 3, 1803-1814 [1983]); sperm-mediated gene transfer (Lavitrano et al., Cell, 57, 717-73 [1989]). For review, see, for example, U.S. Patent No. 4,736,866.

Transgenic animals include those that carry the transgene only in part of their cells ("mosaic animals"). The transgene can be integrated either as a single transgene, or in concatamers, e.g., head-to-head or head-to-tail tandems. Selective introduction of a transgene into a particular cell type is also possible by following, for examples, the technique of Lasko et al., Proc. Natl. Acad. Sci. USA, 89, 6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry. The animals may be further examined for signs of immune disease pathology, for example by histological examination to determine infiltration of immune cells into specific tissues or for the presence of cancerous or malignant tissue.

Alternatively, "knock out" animals can be constructed which have a defective or altered gene encoding a polypeptide identified herein, as a result of homologous recombination between the endogenous gene encoding the polypeptide and altered genomic DNA encoding the same polypeptide introduced into an embryonic cell of the animal. For example, cDNA encoding a particular polypeptide can be used to clone genomic DNA encoding that polypeptide in accordance with established techniques. A portion of the genomic DNA encoding a particular polypeptide can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)] . The selected cells are then injected into a blastocyst of an animal (e.g., a mouse or rat) to form aggregation chimeras [see e.g., Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

### C. FORMULATIONS

The antagonists or agonists described herein are preferably employed in a carrier. Suitable carriers and their formulations are described in Remington's Pharmaceutical Sciences, 16th ed., 1980, Mack Publishing Co., edited by Oslo et al. Typically, an appropriate amount of a pharmaceutically-acceptable salt is used in the carrier to render the formulation isotonic. Examples of the carrier include saline, Ringer's solution and dextrose solution. The pH of the solution is preferably from about 5 to about 8, and more preferably from about 7.4 to about 7.8. It will be apparent to those persons skilled in the art that certain carriers may be more preferable depending upon, for instance, the route of.administration and concentration of agent being administered. The carrier may be in the form of a lyophilized formulation or aqueous solution.

Acceptable carriers, excipients, or stabilizers are preferably nontoxic to cells and/or recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; and/or non-ionic surfactants such as TWEEN^{™}, PLURONICS^{™} or polyethylene glycol (PEG).

The formulation may also contain more than one active compound as necessary for the particular indication being treated, preferably those with complementary activities that do not adversely affect each other.

The antagonist or agonist may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Oslo, A. Ed. (1980).

The formulations to be used for *in vivo* administration should be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethylmethacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

### D. MODES OF THERAPY

The antagonist or agonist molecules described herein are useful in treating various pathological conditions, such as immune related diseases or cancer. These conditions can be treated by stimulating or inhibiting a selected activity associated with TALL-1, APRIL, TACI, or BCMA in a mammal through administration of one or more antagonists or agonists.

Diagnosis in mammals of the various pathological conditions described herein can be made by the skilled practitioner. Diagnostic techniques are available in the art which allow, e.g., for the diagnosis or detection of cancer or immune related disease in a mammal. For instance, cancers may be identified through techniques, including but not limited to, palpation, blood analysis, x-ray, NMR and the like. Immune related diseases can also be readily identified. In systemic lupus erythematosus, the central mediator of disease is the production of auto-reactive antibodies to self proteins/tissues and the subsequent generation of immune-mediated inflammation. Multiple organs and systems are affected clinically including kidney, lung, musculoskeletal system, mucooutaneous, eye, central nervous system, cardiovascular system, gastrointestinal tract, bone marrow and blood.

Rheumatoid arthritis (RA) is a chronic systemic autoimmune inflammatory disease that mainly involves the synovial membrane of multiple joints with resultant injury to the articular cartilage. The pathogenesis is T lymphocyte dependent and is associated with the production of rheumatoid factors, auto-antibodies directed against self IgG, with the resultant formation of immune complexes that attain high levels in joint fluid and blood. These complexes in the joint may induce the marked infiltrate of lymphocytes and monocytes into the synovium and subsequent marked synovial changes; the joint space/fluid if infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints; the nodules late stage have necrotic centers surrounded by a mixed inflammatory cell infiltrate. Other manifestations which can occur in RA include: pericarditis, pleuritis, coronary arteritis, intestitial pneumonitis with pulmonary fibrosis, keratoconjunctivitis sicca, and rhematoid nodules.

Juvenile chronic arthritis is a chronic idiopathic inflammatory disease which begins often at less than 16 years of age. Its phenotype has some similarities to RA; some patients which are rhematoid factor positive are classified as juvenile rheumatoid arthritis. The disease is sub-classified into three major categories: pauciarticular, polyarticular, and systemic. The arthritis can be severe and is typically destructive and leads to joint ankylosis and retarded growth. Other manifestations can include chronic anterior uveitis and systemic amyloidosis.

Spondyloarthropathies are a group of disorders with some common clinical features and the common association with the expression of HLA-B27 gene product. The disorders include: ankylosing sponylitis, Reiter's syndrome (reactive arthritis), arthritis associated with inflammatory bowel disease, spondylitis associated with psoriasis, juvenile onset spondyloarthropathy and undifferentiated spondyloarthropathy. Distinguishing features include sacroileitis with or without spondylitis; inflammatory asymmetric arthritis; association with HLA-B27 (a serologically defined allele of the HLA-B locus of class I MHC); ocular inflammation, and absence of autoantibodies associated with other rheumatoid disease. The cell most implicated as key to induction of the disease is the CD8+ T lymphocyte, a cell which targets antigen presented by class I MHC molecules. CD8+ T cells may react against the class I MHC allele HLA-B27 as if it were a foreign peptide expressed by MHC class I molecules. It has been hypothesized that an epitope of HLA-B27 may mimic a bacterial or other microbial antigenic epitope and thus induce a CD8+ T cells response.

Systemic sclerosis (scleroderma) has an unknown etiology. A hallmark of the disease is induration of the skin; likely this is induced by an active inflammatory process. Scleroderma can be localized or systemic; vascular lesions are common and endothelial cell injury in the microvasculature is an early and important event in the development of systemic sclerosis; the vascular injury may be immune mediated. An immunologic basis is implied by the presence of mononuclear cell infiltrates in the cutaneous lesions and the presence of anti-nuclear antibodies in many patients. ICAM-1 is often upregulated on the cell surface of fibroblasts in skin lesions suggesting that T cell interaction with these cells may have a role in the pathogenesis of the disease. Other organs involved include: the gastrointestinal tract: smooth muscle atrophy and fibrosis resulting in abnormal peristalsis/motility; kidney: concentric subendothelial intimal proliferation affecting small arcuate and interlobular arteries with resultant reduced renal cortical blood flow, results in proteinuria, azotemia and hypertension; skeletal muscle: atrophy, interstitial fibrosis; inflammation; lung: interstitial pneumonitis and interstitial fibrosis; and heart: contraction band necrosis, scarring/fibrosis.

Idiopathic inflammatory myopathies including dermatomyositis, polymyositis and others are disorders of chronic muscle inflammation of unknown etiology resulting in muscle weakness. Muscle injury/inflammation is often symmetric and progressive. Autoantibodies are associated with most forms. These myositis-specific autoantibodies are directed against and inhibit the function of components, proteins and RNA's, involved in protein synthesis.

Sjogren's syndrome is due to immune-mediated inflammation and subsequent functional destruction of the tear glands and salivary glands. The disease can be associated with or accompanied by inflammatory connective tissue diseases. The disease is associated with autoantibody production against Ro and La antigens, both of which are small RNA-protein complexes. Lesions result in keratoconjunctivitis sicca, xerostomia, with other manifestations or associations including bilary cirrhosis, peripheral or sensory neuropathy, and palpable purpura.

Systemic vasculitis are diseases in which the primary lesion is inflammation and subsequent damage to blood vessels which results in ischemia/necrosis/degeneration to tissues supplied by the affected vessels and eventual end-organ dysfunction in some cases. Vasculitides can also occur as a secondary lesion or sequelae to other immune-inflammatory mediated diseases such as rheumatoid arthritis, systemic sclerosis, etc., particularly in diseases also associated with the formation of immune complexes. Diseases in the primary systemic vasculitis group include: systemic necrotizing vasculitis: polyarteritis nodosa, allergic angiitis and granulomatosis, polyangiitis; Wegener's granulomatosis; lymphomatoid granulomatosis; and giant cell arteritis. Miscellaneous vasculitides include: mucocutaneous lymph node syndrome (MLNS or Kawasaki's disease), isolated CNS vasculitis, Behet's disease, thromboangiitis obliterans (Buerger's disease) and cutaneous necrotizing venulitis. The pathogenic mechanism of most of the types of vasculitis listed is believed to be primarily due to the deposition of immunoglobulin complexes in the vessel wall and subsequent induction of an inflammatory response either via ADCC, complement activation, or both.

Sarcoidosis is a condition of unknown etiology which is characterized by the presence of epithelioid granulomas in nearly any tissue in the body; involvement of the lung is most common. The pathogenesis involves the persistence of activated macrophages and lymphoid cells at sites of the disease with subsequent chronic sequelae resultant from the release of locally and systemically active products released by these cell types.

Autoimmune hemolytic anemia including autoimmune hemolytic anemia, immune pancytopenia, and paroxysmal noctural hemoglobinuria is a result of production of antibodies that react with antigens expressed on the surface of red blood cells (and in some cases other blood cells including platelets as well) and is a reflection of the removal of those antibody coated cells via complement mediated lysis and/or ADCC/Fc-receptor-mediated mechanisms.

In autoimmune thrombocytopenia including thrombocytopenic purpura, and immune-mediated thrombocytopenia in other clinical settings, platelet destruction/removal occurs as a result of either antibody or complement attaching to platelets and subsequent removal by complement lysis, ADCC or FC-receptor mediated mechanisms.

Thyroiditis including Grave's disease, Hashimoto's thyroiditis, juvenile lymphocytic thyroiditis, and atrophic thyroiditis, are the result of an autoimmune response against thyroid antigens with production of antibodies that react with proteins present in and often specific for the thyroid gland. Experimental models exist including spontaneous models: rats (BUF and BB rats) and chickens (obese chicken strain); inducible models: immunization of animals with either thyroglobulin, thyroid microsomal antigen (thyroid peroxidase).

Type I diabetes mellitus or insulin-dependent diabetes is the autoimmune destruction of pancreatic islet β cells; this destruction is mediated by auto-antibodies and auto-reactive T cells. Antibodies to insulin or the insulin receptor can also produce the phenotype of insulin-non-responsiveness.

Immune mediated renal diseases, including glomerulonephritis and tubulointerstitial nephritis, are the result of antibody or T lymphocyte mediated injury to renal tissue either directly as a result of the production of autoreactive antibodies or T cells against renal antigens or indirectly as a result of the deposition of antibodies and/or immune complexes in the kidney that are reactive against other, non-renal antigens. Thus other immune-mediated diseases that result in the formation of immune-complexes can also induce immune mediated renal disease as an indirect sequelae. Both direct and indirect immune mechanisms result in inflammatory response that produces/induces lesion development in renal tissues with resultant organ function impairment and in some cases progression to renal failure. Both humoral and cellular immune mechanisms can be involved in the pathogenesis of lesions.

Demyelinating diseases of the central and peripheral nervous systems, including Multiple Sclerosis; idiopathic demyelinating polyneuropathy or Guillain-Barr syndrome; and Chronic Inflammatory Demyelinating Polyneuropathy, are believed to have an autoimmune basis and result in nerve demyelination as a result of damage caused to oligodendrocytes or to myelin directly. In MS there is evidence to suggest that disease induction and progression is dependent on T lymphocytes. Multiple Sclerosis is a demyelinating disease that is T lymphocyte-dependent and has either a relapsing-remitting course or a chronic progressive course. The etiology is unknown; however, viral infections, genetic predisposition, environment, and autoimmunity all contribute. Lesions contain infiltrates of predominantly T lymphocyte mediated, microglial cells and infiltrating macrophages; CD4+T lymphocytes are the predominant cell type at lesions. The mechanism of oligodendrocyte cell death and subsequent demyelination is not known but is likely T lymphocyte driven.

Inflammatory and Fibrotic Lung Disease, including Eosinophilic Pneumonias; Idiopathic Pulmonary Fibrosis, and Hypersensitivity Pneumonitis may involve a disregulated immune-inflammatory response. Inhibition of that response would be of therapeutic benefit.

Autoimmune or Immune-mediated Skin Disease including Bullous Skin Diseases, Erythema Multiforme, and Contact Dermatitis are mediated by auto-antibodies, the genesis of which is T lymphocyte-dependent.

Psoriasis is a T lymphocyte-mediated inflammatory disease. Lesions contain infiltrates of T lymphocytes, macrophages and antigen processing cells, and some neutrophils.

Allergic diseases, including asthma; allergic rhinitis; atopic dermatitis; food hypersensitivity; and urticaria are T lymphocyte dependent. These diseases are predominantly mediated by T lymphocyte induced inflammation, IgE mediated-inflammation or a combination of both.

Transplantation associated diseases, including Graft rejection and Graft-Versus-Host-Disease (GVHD) are T lymphocyte-dependent; inhibition of T lymphocyte function is ameliorative.

Other diseases in which intervention of the immune and/or inflammatory response have benefit are Infectious disease including but not limited to viral infection (including but not limited to AIDS, hepatitis A, B, C, D, E) bacterial infection, fungal infections, and protozoal and parasitic infections (molecules (or derivatives/agonists) which stimulate the MLR can be utilized therapeutically to enhance the immune response to infectious agents), diseases of immunodeficiency (molecules/derivatives/agonists) which stimulate the MLR can be utilized therapeutically to enhance the immune response for conditions of inherited, acquired, infectious induced (as in HIV infection), or iatrogenic (i.e. as from chemotherapy) immunodeficiency), and neoplasia.

The antagonist(s) or agonist(s) can be administered in accord with known methods, such as intravenous administration as a bolus or by continuous infusion over a period of time, by intramuscular, intraperitoneal, intracerebrospinal, subcutaneous, intra-articular, intrasynovial, intrathecal, oral, topical, or inhalation routes. Optionally, administration may be performed through mini-pump infusion using various commercially available devices. The antagonists or agonists may also be employed using gene therapy techniques which have been described in the art.

Effective dosages and schedules for administering antagonists or agonists may be determined empirically, and making such determinations is within the skill in the art. Single or multiple dosages may be employed. It is presently believed that an effective dosage or amount of antagonist or agonist used alone may range from about 1 µg/kg to about 100 mg/kg of body weight or more per day. Interspecies scaling of dosages can be performed in a manner known in the art, e.g., as disclosed in Mordenti et al., Pharmaceut. Res., 8:1351 (1991).

When *in vivo* administration of an agonist or antagonist thereof is employed, normal dosage amounts may vary from about 10 ng/kg to up to 100 mg/kg of mammal body weight or more per day, preferably about 1 µg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue. Those skilled in the art will understand that the dosage of antagonist or agonist that must be administered will vary depending on, for example, the mammal which will receive the agonist or antagonist, the route of administration, and other drugs or therapies being administered to the mammal.

Depending on the type of cells and/or severity of the disease, about 1 µg/kg to 15 mg/kg (e.g. 0.1-20 mg/kg) of antagonist antibody or agonist antibody is an initial candidate dosage for administration, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful.

Optionally, prior to administration of any antagonist or agonist, the mammal or patient can be tested to determine levels or activity of TALL-1 or APRIL, or TACI or BCMA. Such testing may be conducted by ELISA or FACS of serum samples or peripheral blood leukocytes.

A single type of antagonist or agonist may be used in the methods. For example, a TALL-1 antagonist, such as a TACI receptor immunoadhesin molecule, may be administered. Alternatively, the skilled practitioner may opt to employ a combination of antagonists or agonists in the methods, e.g., a combination of a TACI receptor immunoadhesin and an anti-APRIL antibody. It may further be desirable to employ a dual antagonist, i.e., an antagonist which acts to block or inhibit both TALL-1 and APRIL. Such an antagonist molecule may, for instance, bind to epitopes conserved between TALL-1 and APRIL, or TACI and BCMA.

It is contemplated that yet additional therapies may be employed in the methods. The one or more other therapies may include but are not limited to, administration of radiation therapy, cytokine(s), growth inhibitory agent(s), chemotherapeutic agent(s), cytotoxic agent(s), tyrosine kinase inhibitors, ras farnesyl transferase inhibitors, angiogenesis inhibitors, and cyclin-dependent kinase inhibitors which are known in the art and defined further with particularity in Section I above. In addition, therapies based on therapeutic antibodies that target tumor antigens such as Rituxan^{™} or Herceptin^{™} as well as anti-angiogenic antibodies such as anti-VEGF.

Preparation and dosing schedules for chemotherapeutic agents may be used according to manufacturers' instructions or as determined empirically by the skilled practitioner. Preparation and dosing schedules for such chemotherapy are also described in Chemotherapy Service Ed., M.C. Perry, williams & Wilkins, Baltimore, MD (1992). The chemotherapeutic agent may precede, or follow administration of, e.g. an antagonist, or may be given simultaneously therewith. The antagonist may also be combined with an anti-oestrogen compound such as tamoxifen or an anti-progesterone such as onapristone (see, EP 616812) in dosages known for such molecules.

It may be desirable to also administer antibodies against other antigens, such as antibodies which bind to CD20, CD11a, CD18, CD40, ErbB2, EGFR, ErbB3, ErbB4, or vascular endothelial factor (VEGF). Alternatively, or in addition, two or more antibodies binding the same or two or more different antigens disclosed herein may be co-administered to the patient. Sometimes, it may be beneficial to also administer one or more cytokines to the patient. In one embodiment, the antagonists herein are co-administered with a growth inhibitory agent. For example, the growth inhibitory agent may be administered first, followed by an antagonist of the present invention.

The antagonist or agonist (and one or more other therapies) may be administered concurrently or sequentially. Following administration of antagonist or agonist, treated cells *in vitro* can be analyzed. Where there has been *in vivo* treatment, a treated mammal can be monitored in various ways well known to the skilled practitioner. For instance, markers of B cell activity such as Ig production (non-specific or antigen specific) can be assayed.

### III. METHOD OF SCREENING

The invention also encompasses methods of screening molecules to identify those which can act as agonists or antagonists of the APRIL/TACI/BCMA interaction or the TALL-1/TACI/BCMA interaction as defined in the claims. Such molecules may comprise small molecules or polypeptides, including antibodies. Examples of small molecules include, but are not limited to, small peptides or peptide-like molecules, preferably soluble peptides, and synthetic non-peptidyl organic or inorganic compounds. The screening assays for drug candidates are designed to identify compounds or molecules that bind or complex with the ligand or receptor polypeptides identified herein, or otherwise interfere with the interaction of these polypeptides with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

Assays for, for instance, antagonists are common in that they call for contacting the drug candidate with a ligand or receptor polypeptide identified herein under conditions and for a time sufficient to allow these two components to interact.

In binding assays, the interaction is binding and the complex formed can be isolated or detected in the reaction mixture. In a particular embodiment, the ligand or receptor polypeptide identified herein or the drug candidate is immobilized on a solid phase, e.g., on a microtiter plate, by covalent or non-covalent attachments. Non-covalent attachment generally is accomplished by coating the solid surface with a solution of the ligand or receptor polypeptide and drying. Alternatively, an immobilized antibody, e.g., a monoclonal antibody, specific for the ligand or receptor polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular ligand or receptor polypeptide identified herein, its interaction with that polypeptide can be assayed by methods well known for detecting protein-protein interactions. Such assays include traditional approaches, such as, e.g., cross-linking, co-immunoprecipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER^{™}) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds or molecules that interfere with the interaction of a ligand or receptor polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the ligand or receptor polypeptide may be added to a cell along with the compound to be screened for a particular activity and the ability of the compound to inhibit the activity of interest in the presence of the ligand or receptor polypeptide indicates that the compound is an antagonist to the ligand or receptor polypeptide. Alternatively, antagonists may be detected by combining the ligand or receptor polypeptide and a potential antagonist with membrane-bound polypeptide receptors or recombinant receptors under appropriate conditions for a competitive inhibition assay. The ligand or receptor polypeptide can be labeled, such as by radioactivity, such that the number of polypeptide molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the ligand or receptor polypeptide and a cDNA library created from this RNA is divided into pools and used to transfect COS cells or other cells that are not responsive to the ligand or receptor polypeptide. Transfected cells that are grown on glass slides are exposed to labeled ligand or receptor polypeptide. The ligand or receptor polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach, labeled ligand polypeptide can be photoaffinity-linked with cell membrane or extract preparations that express receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro- sequencing would be used to design a set of degenerate oligonucleotide probes to screen a cDNA library to identify the gene encoding the putative receptor.

### IV. ARTICLES OF MANUFACTURE

An article of manufacture containing materials useful for the treatment of the disorders described above is described. The article of manufacture comprises a container and a label. Suitable containers include, for example, bottles, vials, syringes, and test tubes. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). The active agents in the composition may comprise TALL-1 antagonist(s) or APRIL antagonist(s), or TACI agonist(s) or BCMA agonist(s). The label on, or associated with, the container indicates that the composition is used for treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

The following examples are offered by way of illustration and not by way of limitation.

### EXAMPLE 1

### Identification and Expression cloning of TACI, A Receptor for TALL-1 Ligand

A chimeric protein, referred to as "AP-TALL-1", was prepared using human placenta alkaline phosphatase (AP) fused to the N-terminus of a TALL-1 polypeptide consisting of amino acids 136-285 shown in Figure 3. The AP was obtained by PCR amplification using pAPtag-5 (Genehunter Corporation) as a template, and fused and cloned into the expression vector, pCMV-1 Flag (Sigma), with AP at the N-terminus of TALL-1. The AP-TALL-1 was transiently transfected (using Lipofectamine reagent; Gibco-BRL) and expressed in human embryonic kidney 293 cells (ATCC). AP-TNF-alpha (Pennica et al., infra) was similarly prepared. AP-EDA (comprising amino acids 241-391 of EDA; Srivastava et al., Proc. Natl. Acad. Sci., 94:13069-13074 (1997)) was also similarly prepared. The conditioned medium from the transfected 293 cells was filtered (0.45 micron), stored at 4°C in a buffer containing 20mM Hepes (pH 7.0) and 1 mM sodium azide, and used for subsequent cell staining procedures.

In addition, an N-terminal Flag-tagged form of TALL-1 was constructed in a pCKV-1 Flag vector. To promote the trimerization of this Flag-tagged TALL-1 construct, a trimeric form of leucine-zipper sequence [Science, 262:1401-1407 (1993)] was inserted between the Flag-tag and the TALL-1 (consisting of amino acids 136-285 of Figure 3), and this construct was referred to as "Flag-LZP-TALL-1". The Flag-LZP-TALL-1 was purified using M2-agarose gel (Sigma) from serum-free medium of 293 cells transfected with the Flag-LZP-TALL-1 expressing plasmid.

AP reactivity could be readily detected when AP-TALL-1 conditioned medium, but not control AP conditioned medium, was applied to IM-9 cells (ATCC) which have been shown to exhibit high levels of TALL-1 binding activity (data not shown). The purified Flag-LZF-TALL-1 also bound to the IM-9 cells, as determined by FACS analysis (Data not shown). Importantly, the binding of AP-TALL-1 to IM-9 cells was effectively blocked by preincubation with purified Flag-LZP-TALL-1 but not with purified TNF-alpha [prepared essentially as described in Pennica et al., Nature, 312:724-729 (1984)], suggesting that both forms of TALL-1 were functional and the respective binding of AP-TALL-1 and Flag-LZP-TALL-1 to IM-9 cells was specific.

To identify a receptor for TALL-1, a cDNA expression library was constructed in pRK5 vector (EP 307,247, published March 15, 1989) using PolyA+.mRNA derived from the IM-9 cells [Flanagan et al., Cell, 63:185 (1990)]. Pools of -1000 cDNA clones (Miniprep DNA (Qiagen)) from the library were transfected (using Lipofectamine) into COS 7 cells (ATCC) in 6 well plates, which after 36-48 hours, were then incubated with AP-TALL-1 conditioned medium, washed, and stained for AP activity in situ. A positive pool was broken down to successively smaller size pools. After three rounds of screening, a single cDNA encoding an AP-TALL-1 binding activity was identified. Sequencing of the cDNA insert revealed a single open reading frame predicted to encode a protein of 265 amino acids. This 265 amino acid polypeptide (referred to as "hTACI (265)" in Figure 6), when aligned with the TACI sequence shown in Fig. 1 (referred to as "hTACI" in Figure 6), revealed a high percentage of sequence identity, particularly in the ECD. The alignment of these two TACI sequences is shown in Figure 6. It is believed that the 265 amino acid form of TACI may be a spliced variant of the TACI sequence shown in Figure 1.

In an *in vitro* assay, COS 7 cells (ATCC) were seeded into 12 well plates 24 hours before transfection. The cells were then transfected with 1 microgram TACI (the 265 amino acid form of human TACI described above, cloned in pRK5B vector, infra) or vector plasmid (pRK5B) alone. 18-24 hours after transfection, the cells were incubated with conditioned medium containing AP-TALL-1; AP-TNF-alpha; or AP-EDA for 1 hour at room temperature and stained for AP activity in situ as described in Tartaglia et al., Cell, 83:1263-1271 (1995).

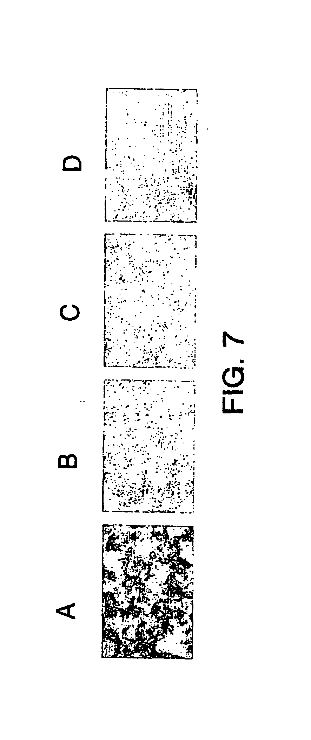

As shown in Fig. 7, AP-TALL-1 (Fig. 7A) but not AP-TNF-alpha (Fig. 7B) or AP-EDA (Fig. 7C) was found to bind COS 7 cells transfected with TACI. The AP-TALL-1 did not stain cells transfected with vector plasmid alone (Fig. 7D). AP-TALL-1 binding to the TACI transfected COS 7 cells was effectively blocked by a Flag-tagged form of TALL-1, but not by a Flag-tagged form of LIGHT (Mauri et al., supra], another TNF homolog (data not shown).

### EXAMPLE 2

### Binding of TALL-1 or APRIL to TACI-IgG and BCMA-IgG

Flag-tagged ligands were prepared as follows. Amino acids 82-240 of LIGHT (Mauri et al., Immunity, 8:21-30 (1998)) were subcloned into pCMV-1 Flag (Sigma) using a NotI site to fuse amino acids 1-27 of the Flag signal and tag sequence upstream of the LIGHT sequence. Amino acids 105-250 of APRIL (see Fig. 4) were similarly cloned into pCMV-1 Flag (Sigma), except that a HindIII site was used, resulting in fusion to amino acids 1-24 of the Flag signal and tag sequence. Amino acids 124-285 of TALL-1 (see Fig. 3) were fused to amino acids 1-27 of the Flag signal and tag sequence, as described above for Flag-LIGHT. AP-APRIL was prepared by cloning amino acids 105-250 of APRIL (see Fig. 4) into a pCMV-1 Flag vector encoding human placental alkaline phosphatase such that the APRIL encoding sequence was fused C-terminally to AP, while the AP was fused C-terminally to Flag. AP-TALL-1 was prepared by cloning amino acids 135-285 of TALL-1 (see Fig. 3) into the pCMV-1 Flag, AP vector, as described above for AP-APRIL. The respective tagged proteins were then expressed in 293 cells or CHO cells and purified using M2 anti-Flag resin (Sigma).

One µg of the purified human or Flag-LIGHT (control), Flag-APRIL, or Flag-TALL-1, or Flag-AP-APRIL, or Flag-AP-TALL-1 was incubated with 1 µg of purified human immunoadhesin containing the IgG1-Fc fusion of the ECD of DcR3 (control; Pitti et al., Nature, 396:699-703 (1998)) or TACI or BCMA overnight at 4° C in duplicate. The TACI-ECD.hFc immunoadhesins were prepared by methods described in Ashkenazi et al., Proc. Natl. Acad. Sci., 88:10535-10539 (1991). The immunoadhesin constructs consisted of amino acids 2-166 of the human TACI polypeptide (see Figure 1). The TACI-ECD constructs were expressed in CHO cells using a heterologous signal sequence (pre-pro trypsin amino acids 1-17 of pCMV-1 Flag (Sigma)) and encoding the human IgG1 Fc region downstream of the TACI sequence, and then purified by protein A affinity chromatography. The BCMA-ECD immunoadhesins were prepared by methods described in Ashkenazi et al., as cited above. The immunoadhesin constructs consisted of amino acids 5-51 of the human BCMA polypeptide (see Figure 2). The BCMA-ECD constructs were expressed in CHO cells using a heterologous signal sequence (pre-pro trypsin amino acids 1-17 of pCMV-1 Flag (Sigma)) and encoding the human IgG1 Fc region downstream of the BCMA sequence, and then purified by protein A affinity chromatography.

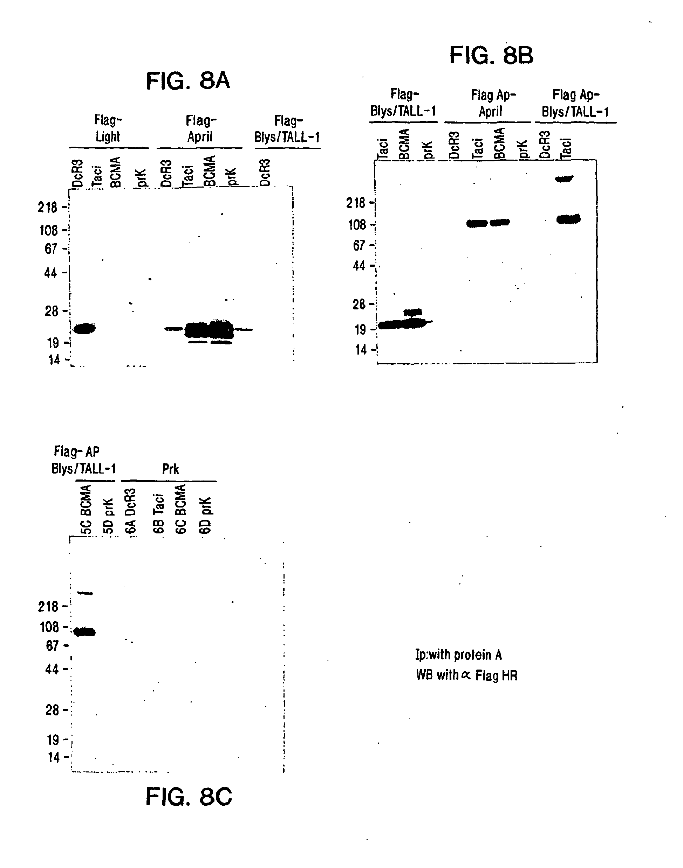

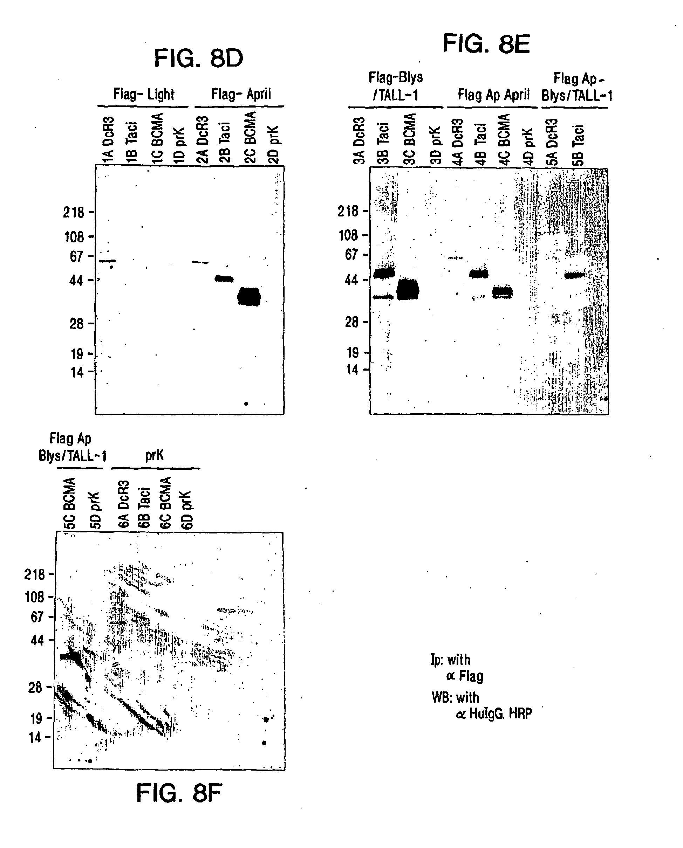

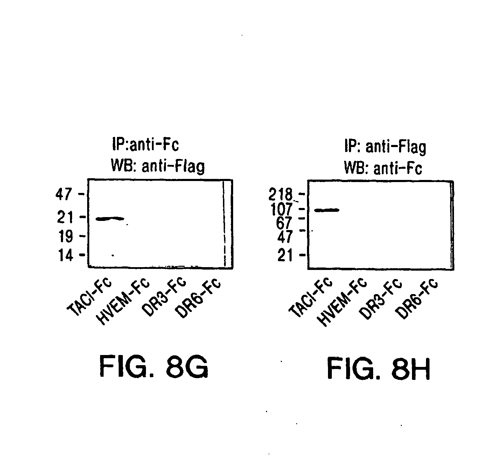

One set of reactions (Figure 8; Panels A-C) was subjected to immunoprecipitation through the receptor-immunoadhesin with protein A-agarose (Repligen). The second set of reactions (Figure 8; panels D-F) was subjected to immunoprecipitation through the Flag-tagged ligand with Anti-Flag mAb-M2-agarose (Sigma). The immunoprecipitates were then analyzed by Western blot with horseradish peroxidase-conjugated anti-Flag M2 mAb (Sigma) to detect the Flag-tagged ligands (Figures 8A-C) or horseradish peroxidase-conjugated goat anti-human IgG pAb (Amersham) to detect the receptor-immunoadhesins (Figures 8D-F).

The data shows that Flag-LIGHT bound to DcR3-IgG, but not to TACI-IgG or BCMA-IgG. Flag-APRIL and Flag-AP-APRIL bound to TACI-IgG and BCMA-IgG, but not to DcR3-IgG. Similarly, Flag-TALL-1 and Flag-AP-TALL-1 bound to TACI-IgG and BCMA-IgG, but not to DcR3-IgG. These assay results indicated that APRIL and TALL-1 can each bind in a specific and stable manner to TACI and to BCMA.

In a similarly conducted co-immunoprecipitation assay, TACI-Fc (described in Example 2), HVEM-Fc (Montgomery et al., supra); DR3-Fc (Chinnaiyan et al., Science, 274:990 (1996); Marsters et al., Curr. Biol., 6:1669 (1996)); or DR6-Fc (Pan et al., FEBS Letters, 431:351-356 (1998))(1 µg/ml) was incubated with Flag-TALL-1 (1 µg/ml; prepared as described in Example 2). One set of reactions was subjected to immunoprecipitation through the receptor-Fc fusion with protein A Agarose; the second set of reactions was subjected to immunoprecipitation through the ligand with anti-Flag antibody. The samples were analyzed by Western blot, as above. Flag-TALL-1 was not detected in anti-Fc co-immunoprecipitation with the Fc fusion constructs of HVEM, DR3, or DR6 (Figure 8G). Conversely, TACI-Fc was not detected in anti-Flag co-immunoprecipitations with HVEM-Fc, DR3-Fc, or DR6-Fc (Figure 8H).

Additional assays were conducted to determine whether TACI could serve as a receptor for other members of the TNF family of ligands. COS 7 cells (ATCC) were transiently transfected (using Lipofectamine reagent) with membrane forms of various ligands of TNF family. Among the ligands tested were APRIL, TALL-1, 4-1 BBL, CD27L, CD30L, CD40L, EDA, FasL, GITRL, LT-alpha, OX-40L, RANKL, TNF-alpha, TNF-beta and Apo2L/TRAIL.

Human TNF-alpha was cloned into pRK5B vector (pRK5B is a precursor of pRK5D that does not contain the SfiI site; see Holmes et al., Science, 253:1278-1280 (1991)). For the detection of TNF-alpha expression on the cell surface, a Flag tag was inserted between amino acid 70 and amino acid 71 (using the numbering according to the sequence in Pennica et al., supra). An extracellular region of TALL-1 (aa 75-285; see Figure 3), 4-1BBL (aa 59-254; Goodwin et al., Eur. J. Immunol., 23:2631-2641 (1993)), CD27 ligand (aa 40-193; Goodwin et al., Cell, 73:447-456 (1993)), CD30 ligand (aa 61-234; Smith et al., Cell, 73:1349-1360 (1993)), RANKL (aa 71-317; see WO98/28426), Apo-2 ligand (aa 40-281; see WO97/25428) or Apo-3L (aa 46-249; see WO99/19490) was individually cloned at the BamHI site. This resulted in a chimeric ligand with the intracellular and transmembrane regions from TNF-alpha and the extracellular region from the various ligands. For APRIL (see Figure 4) and EDA (Srivastava et al., supra), full length cDNA clones without Flag tag were used.

Transfected COS 7 cells were subsequently incubated with TACI.ECD.hFC immunoadhesin (prepared as described above). Cells were incubated with the TACI ECD-IgG (or a TNFR1-IgG construct prepared as described in Ashkenazi et al., Proc. Natl. Acad. Sci., 88:10535-10539 (1991)) at 1 µg/ml for 1 hour in PBS. Cells were subsequently washed three times with PBS and fixed with 4% paraformaldehyde in PBS. Cell staining was visualized by incubation with biotinylated goat anti-human antibody (Jackson Labs, at 1:200 dilution) followed by Cy3-streptavidin (Jackson Labs, at 1:200 dilution).

To identify potential ligand(s) of BCMA, similar binding experiments, as described above for TACI, were performed. A BCMA.ECD.hFc immunoadhesin was prepared, as described above.

Similar to TACI, BCMA only interacted with APRIL and TALL-1. As shown in Fig. 9A, TACI-hFc and BCMA-hFc bound to cells transfected with TALL-1 or April but not TNF-alpha. Conversely, AP-TALL-1 or AP-APRIL specifically bound to cells transfected with TACI or BCMA (Fig. 9B).

### EXAMPLE 3

### Induction of IgM production by TALL-1 and APRIL and Inhibition of the Induction by TACI-IgG and BCMA-IgG

Human peripheral blood mononuclear cells (PBMC) were isolated on a Ficol gradient according to manufacturer's instructions (LSM media, ICN/Cappel, OH). Peripheral blood leukocytes (PBL) were then obtained from the PBMC using standard removal of plastic-adherent cells. PBLs were plated in 48-well dishes (3 x 10⁴ cells/well on 0.3 ml of RPMI1640 medium containing 10% FBS) and incubated for 72 hours at 37° C, 5% CO₂, with PBS (control), or IL-4 (100ng/ml, control, R & D Systems, Minneapolis, MN), or Flag-TALL-1 (as described in Example 2 above) (1 µg/ml). For inhibition analysis, the cells were incubated with each of the above in combination with 20 µg/ml of TACI-IgG or BCMA-IgG (prepared as described in Example 2 above), or an isotype-matched immunoadhesin control. Cell supernatants were collected and analyzed for IgM levels using an IgM ELISA kit according to manufacturer's instructions (Bethyl Laboratories, TX).

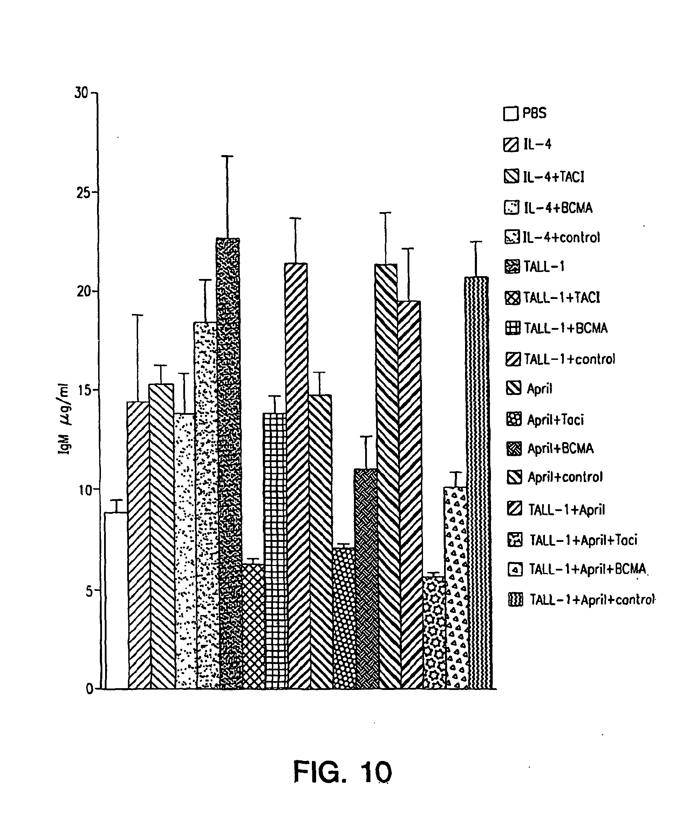

The results are shown in Figure 10. IL-4, used as a positive control, induced IgM production compared to the control PBS. TALL-1 or APRIL induced at least as much IgM production as IL-4. The combination of TALL-1 and APRIL showed no further induction of IgM compared to each ligand alone. TACI-IgG did not block the effect of IL-4, but it blocked the effect of TALL-1 and/or APRIL completely.

BCMA-IgG did not block the effect of IL-4, but blocked the effect of TALL-1 and/or APRIL substantially, though not completely. The control immunoadhesin did not block any of the ligands. These results show that TALL-1 and APRIL can induce IgM production in PBL. Moreover, the data show that TACI-IgG and BCMA-IgG can block the effects of TALL-1 or APRIL on IgM production, confirming their respective ability to bind to each ligand and demonstrating their respective ability to block the ligand's activity on target cells.

### EXAMPLE 4

### Interaction between TALL-1 or APRIL with TACI and/or BCMA Results in Activation of NF-κB

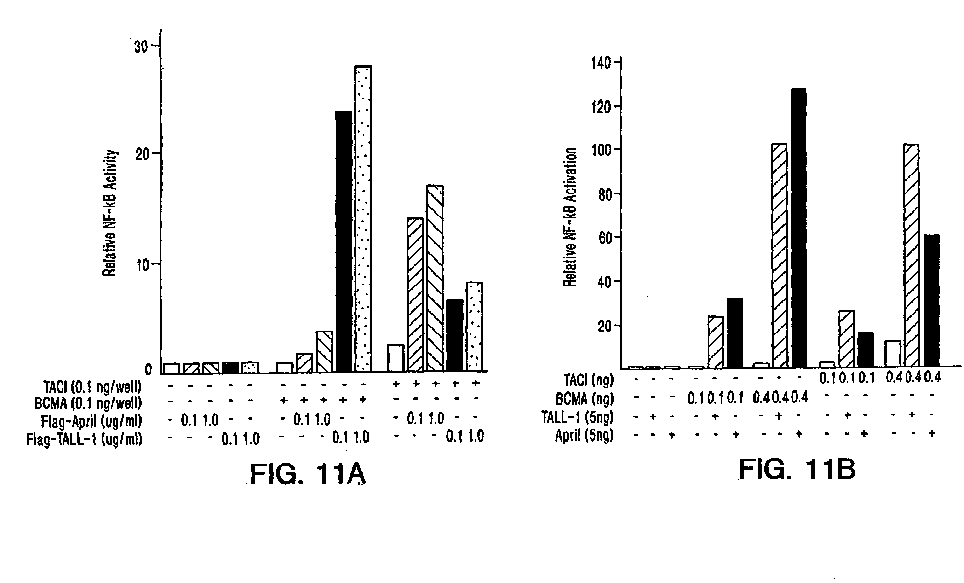

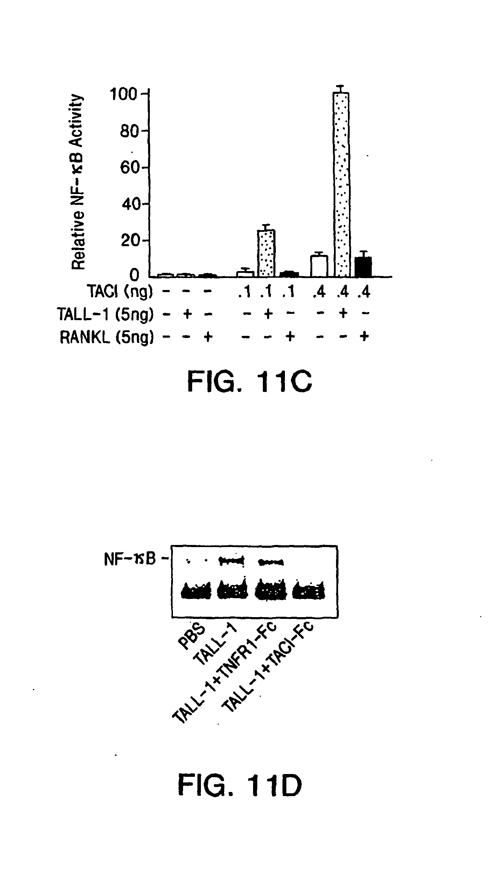

293 cells (ATCC) were seeded 24 hours before transfection at 1 x 10⁵ cells/well into 12-well plates and transfected with 0.25 µg of ELAM-luciferase reporter gene plasmid, 25 ng pRL-TK (Promega) and the indicated amounts of each expression construct (see Figure 11). Total amount of transfected DNA was kept constant at 1 mg by supplementation with empty pRK5B vector (see Example 2). In some assay wells, Flag-tagged ligands (prepared as described in Example 2) were added at concentrations indicated 4 hours after transfection. In other assay wells, the cells were co-transfected with full length TALL-1 (Fig. 3) or RANKL (WO98/28426). Cells were harvested 20-24 hours after transfection and reporter gene activity determined with the Dual-Luciferase Reporter Assay System (Promega).

Only minimal activation of NF-kB was observed when TACI or BCMA was expressed alone at low levels (such as at 0.1 ng). The activation of NF-κB, however, was greatly augmented by either addition of Flag-TALL-1 or Flag-APRIL (Fig. 11A), or by co-transfection with full length TALL-1 or APRIL (Fig. 11B; 11C).

Treatment of untransfected IM-9 cells with Flag-TALL-1 also resulted in activation of NF-kB (see Fig. 11D). The IM-9 cells (ATCC) were incubated with Flag-TALL-1 (0.3 µg/ml) or PBS alone or in combination with 20 µg/ml TACI-IgG or TNFR1-IgG (prepared as described in Example 2). The NF-kB activity was measured by an electrophoretic mobility shift assay as described in Montgomery et al., Cell, 87:427-436 (1996); Marsters et al., J. Biol. Chem., 272:14029 (1997); Chinnaiyan et al., Science, 274:990 (1996); Marsters et al., Curr. Biol., 6:1669 (1996); Pan et al., FEBS Letters, 431:351 (1998).

The data suggests that one physiological consequence of TALL-1/APRIL-TACI/BCMA interaction is the activation of the NF-kB pathway.

### EXAMPLE 5

### Inhibition of Germinal Center Formation and Antibody Production in TACI-IgG or BCMA-IgG Treated, Immunized Mice

*In vivo* assays were conducted to determine whether the blocking of the TALL-1/TACI or TALL-1/BCMA interaction impairs humoral immune responses. Three groups of female C57BL/6 mice of 6 - 8 week of age were immunized intraperitoneally (i.p.) with 100 µg of acetyl-conjugated chicken gamma globulin (NP₂₃-CgG) (Biosource Technologies) precipitated in alum (4-hydroxy-3-nitrophenyl). The groups of animals were treated daily for 14 days with 50 µg of TACI-Fc or BCMA-Fc (prepared as described in Example 2) in 100 µl saline (and control animals were treated with 100 µl saline) by i.p. injection.

After 14 days, mouse sera were analyzed for NP-specific IgM, low-affinity IgG1, and high-affinity IgG1 using a standard ELISA method. NP-specific IgG1, both high-affinity antibodies and total (high- plus low-affinity) antibodies were quantified by ELISA in wells coated with NP_{2.5} and NP₂₃-conjugated bovine serum albumin, respectively. Bound antibodies were detected with AP-conjugated goat anti-mouse IgM or IgG1 (Pharmingen).

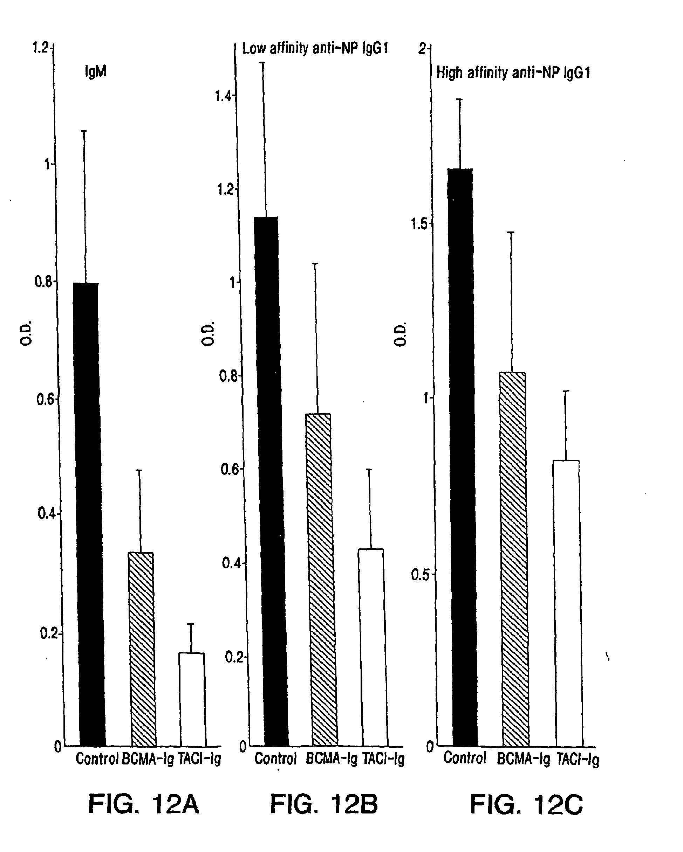

The results are shown in Figure 12. TACI-Fc and BCMA-Fc substantially inhibited the production of NP-specific IgM antibodies as compared to control (Fig. 12A), indicating that TALL-1/TACI and TALL-1/BCMA interactions (and APRIL/TACI and APRIL/BCMA interactions) are important during the early phase of B cell activation that leads to IgM secretion. TACI-Fc and BCMA-Fc inhibited low- and high-affinity NP-specific IgG1 responses as well (Fig. 12 B, C), suggesting that both TALL-1/TACI and TALL-1/BCMA interactions (and both APRIL/TACI and APRIL/BCMA interactions) are important also for Ig class switching and affinity maturation.

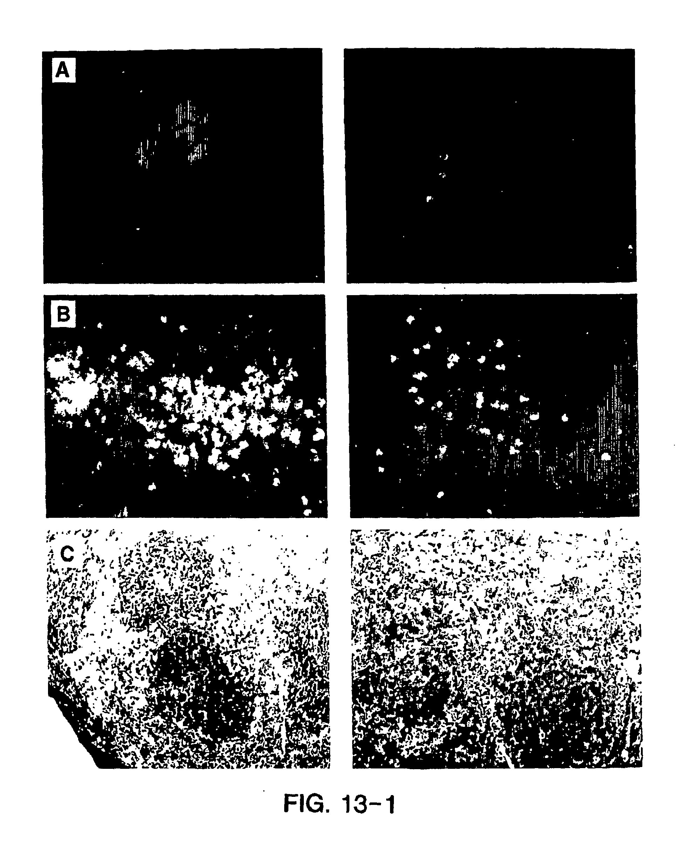

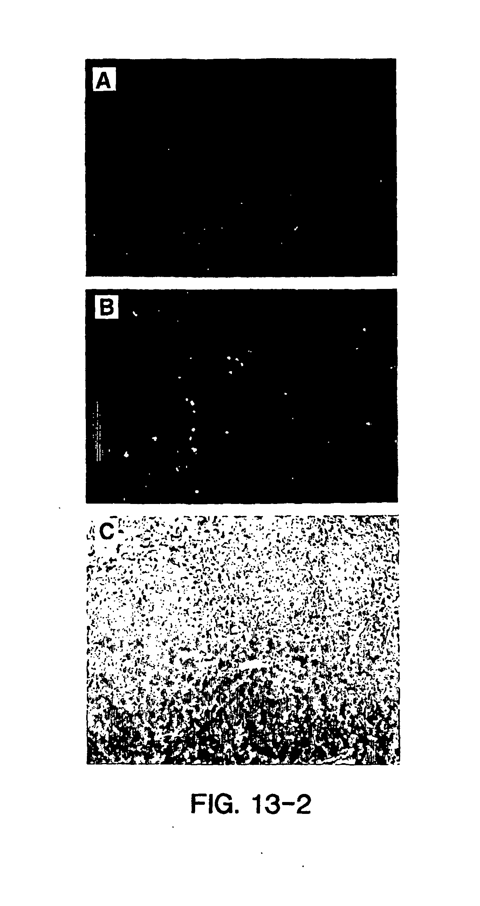

During the early part of an antigen-specific antibody response, B cells differentiate into antibody-forming cells (AFC). This takes place in extrafollicular areas of the spleen composed of periarteriolar lymphoid sheaths (PALS) [Gray et al., Immunology, 65:73 (1988); NacLennan, Ann. Rev. Immunol., 12:117 (1988)], where Ig class switching subsequently occurs. The PALS-associated regions were compared from spleens of NP₂₃-CgG-immunized mice treated for 10 days with control Ig, TACI-Fc, or BCMA-Fc, similar to as described above. Immunohistochemical analysis of the various spleen sections was then conducted. Spleen sections prepared 10 days after immunization and stained with FITC-conjugated anti-IgG1 are shown in Figure 13-1 (Panel A) and Figure 13-2 (Panel A). As expected, control mice displayed a large number of clustered AFC foci that stained intensely with anti-IgG1 and contained many immunoblast-like cells (Fig. 13-1, Panel A, left). In contrast, TACI-Fc treated mice showed only few, isolated, IgG1-positive cells, with no formation of AFC foci (Fig. 13-1, Panel A, right). BCMA-Fc treated mice likewise showed only few, isolated, IgG1-positive cells, with no formation of AFC foci (Fig. 13-2, Panel A). Thus, TALL-1/TACI and TALL-1/BCMA (and APRIL/TACI and APRIL/BCMA) interactions are important for the extrafollicular differentiation of B cells that precedes Ig class switching in splenic PALS-associated areas.

To study the potential role of TALL-1/TACI and TALL-1/BCMA interactions in antibody affinity maturation, the formation of germinal centers (GC) was examined in the spleens of NP₂₃-CgG-immunized mice at day 14. Spleen sections were prepared 14 days after immunization and stained with FITC-conjugated anti-PNA (green fluorescence) and Texas Red-conjugaed anti-IgM (red fluorescence). As expected, splenic follicles from controls displayed intense staining with peanut agglutinin (PNA), a lectin that binds specifically to GC B cells (Fig. 13-1, Panel B, left). In sharp contrast, splenic follicles from TACI-Fc treated mice were devoid of GCs, and displayed only few, isolated, PNA-staining cells (Fig. 13-1, Panel B, right). Splenic follicles from the BCMA-Fc treated mice were also devoid of GCs, and displayed only few, isolated, PNA-staining cells (Fig. 13-2, Panel B).

Despite the lack of GCs, there were no abnormalities in splenic follicular architectures of TACI-Fc or BCMA-Fc treated mice, as judged by hematoxilyn and eosin staining of spleen sections at day 14 (Figure 13-1 - Panel C, left (Controls) and Panel C, right (TACI-Fc treated); and Figure 13-1 Panel C (BCMA-Fc treated). This suggests that in TACI-Fc or BCMA-Fc treated mice, some follicular B cells could differentiate into AFCs, but could not proceed to form GCs. Thus, TALL-1/TACI and TALL-1/BCMA interactions (as well as APRIL/TACI and APRIL/BCMA interactions) appear to be critical for proper GC formation.

The blocking of the TALL-1/TACI and TALL-1/BCMA interactions (or APRIL/TACI or APRIL/BCMA interactions) in mice during primary immunization inhibited several aspects of the B cell response: (a) the early phase of extrafollicular B cell activation that leads to antigen-specific IgM production; (b) the differentiation of B cells that leads to Ig class switching; (c) the formation of splenic GCs, where affinity maturation occurs and memory B cells are generated. While GC formation was blocked completely by TACI-Fc or BCMA-Fc, some residual IgM and IgG1 production and affinity maturation occurred. That attenuated antibody responses can proceed despite the absence of GCs has been observed in other systems [see, e.g., Matsumoto et al., Nature, 382:462 (1996); Kato et al., J. Immunol., 160:4788 (1998); Futtere et al., Immunity, 9:59 (1998). It is possible that other factors besides TALL-1 or APRIL and TACI or BCMA mediate the remaining antibody production. Alternatively, the selected TACI-Fc or BCMA-Fc treatment *in vivo* may not have sufficed to prevent all TALL-1/TACI, TALL-1/BCMA, APRIL/TACI or APRIL/BCMA binding events.

Previous studies indicate that CD40L-CD40 [Foy et al., Ann. Rev. Immunol., 14:591 (1996)] and CD86-CD28/CTLA-4 [Han et al., J. Immunol., 155:556 (1995); Lenschow et al., Ann. Rev. Immunol., 14:233 (1996)] interactions are important for entry of extrafollicular B cells into GC areas and for GC establishment. Inhibition of these interactions through gene knockouts or by treatment with blocking antibodies or receptor-Fc fusions diminishes antibody production and blocks GC formation [Lane et al., J. Exp. Med., 179:819 (1994); Durie et al., Immunol. Today, 15:406 (1994); Hathcock et al., Science, 262:905 (1993); Linsey et al., Science, 257:7992 (1992); Renshaw et al., J. Exp. Med., 180:1889 (1994); Xu et al., Immunity, 1:423 (1994); Kawabe et al., Immunity, 1:167 (1994); Foy et al., J. Exp. Med., 180:157 (1994)). There are some striking similarities between the TALL-1/TACI, TALL-1/BCMA and CD40L-CD40 systems: both ligands are related to TNF and are expressed on activated T cells and both receptors are TNFR homologs that stimulate NF-KB and are expressed on B cells. Hence, the interaction of TALL-1 or APRIL with TACI or BCMA might mediate T-cell help to B cells similar to CD40L and CD40. TALL-1 also may contribute to the activation of B cells by dendritic cells, which do express the TALL-1 ligand. Unlike CD40L and CD40 knockout mice, which exhibit impaired IgG but not IgM responses, and unlike CD40L-deficient patients with hyper-IgM syndrome [Callard et al., Immunol. Today, 14:559 (1993); Allen at al., Science, 259:990 (1993); Aruffo et al., Cell, 72:291 (1993)], TACI-Fc-treated or BCMA-Fc-treated mice showed a marked deficit in both IgM and IgG production. Thus, it is possible that TALL-1 or APRIL and TACI or BCMA operate early in B cell activation, such that their blockade impairs all phases of the humoral response. In contrast, CD40L and CD40 may operate later in B cell activation, such that their blockade impairs only late phases of the antibody response.

### EXAMPLE 6

### Preparation of Anti-APRIL Monoclonal Antibodies

Balb/c mice (obtained from Charles River Laboratories) were immunized by injecting 1.0µg of Flag-APRIL (diluted in MPL-TDM adjuvant purchased from Ribi Immunochemical Research Inc., Hamilton, MT) 10 times into each hind foot pad. The immunization consisted of a series of 6 injections (one injection/week for 6 weeks). The animals then rested for two months, and subsequent immunization injections were given once per week for 4 weeks. The Flag-tagged APRIL fusion protein was prepared as described in Example 2 above and purified by anti-Flag M2 agarose affinity chromatography (Sigma).

Three days after the final boost, popliteal lymph nodes were removed from the mice and a single cell suspension was prepared in DMEM media (obtained from Biowhitakker Corp.) supplemented with 1% penicillin-streptomycin. The lymph node cells were then fused with murine myeloma cells P3X63AgU.1 (ATCC CRL 1597) using 35% polyethylene glycol and cultured in 96-well culture plates. Hybridomas resulting from the fusion were selected in HAT medium. Ten days after the fusion, hybridoma culture supernatants were screened in an ELISA to test for the presence of monoclonal antibodies binding to the Flag-APRIL protein. As a negative to discard monoclonal antibodies binding to the Flag portion or the molecule, the monoclonal antibodies were also screened for any binding to Flag-tagged Apo-3.

In the ELISA, 96-well microtiter plates (Maxisorb; Nunc, Kamstrup, Denmark) were coated by adding 50 µl of .25 µg/ml Flag-APRIL or Flag-Apo-3 in 50mM carbonate buffer, pH 9.6, to each well and incubating at 4°C overnight. The plates were then washed three times with wash buffer (PBS containing 0.05% Tween 20). The wells in the microtiter plates were then blocked with 200 µl of 2.0% bovine serum albumin in PBS and incubated at room temperature for 1 hour. The plates were then washed again three times with wash buffer.

Following the wash steps, 100 µl of the hybridoma supernatants or various concentrations of polyclonal sera was added to designated wells. 100 µl of P3X63AgU.1 myeloma cell conditioned medium was added to other designated wells as controls. The plates were incubated at room temperature for 1 hour on a shaker apparatus and then washed three times with wash buffer.

Next, 50 µl HRP-conjugated goat anti-mouse IgG Fc (purchased from Cappel Laboratories), diluted 1:1000 in assay buffer (0.5% bovine serum albumin, 0.05% Tween-20, 0.01% Thimersol in PBS), was added to each well and the plates incubated for 1 hour at room temperature on a shaker apparatus. The plates were washed three times with wash buffer, followed by addition of 50 µl of substrate (TMB microwell peroxidase substrate, Kirkegaard & Perry, Gaithersburg, MD) to each well and incubation at room temperature for 10 minutes. The reaction was stopped by adding 50 µl of TMB 1-component stop solution (diethyl glycol, Kirkegaard & Perry) to each well, and absorbance at 490 nm was read in an automated microtiter plate reader.

The supernatants testing positive in the ELISA were then cloned twice by limiting dilution.

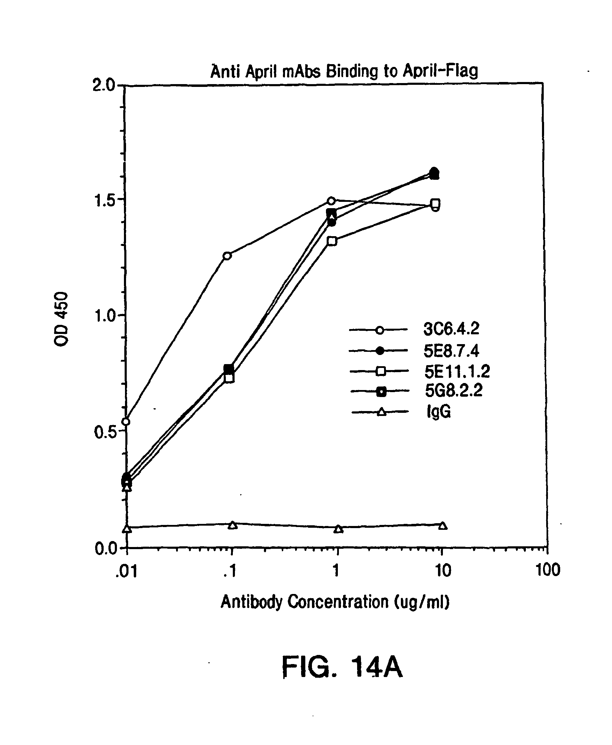

As shown in Figure 14A, the 3C6.4.2, 5E8.7.4, 5E11.1.2 and 5G8.2.2 antibodies were found to bind Flag-APRIL.

### EXAMPLE 7

### Isotyping of anti-APRIL Antibodies

The isotypes of the anti-APRIL monoclonal antibodies (see Example 6) were determined by coating plates with isotype specific goat anti-mouse Ig (Fisher Biotech, Pittsburgh, PA) at 4° C overnight. After non-specific binding sites were blocked with 2% BSA, 100 µl of hybridoma culture supernatants or .5 µg/ml of purified mAbs were added. After incubation for 30 minutes at room temperature, plates were incubated with HRP-conjugated goat anti-mouse Ig for 30 minutes at room temperature. The level of HRP bound to the plate was detected using HRP substrate as described above.

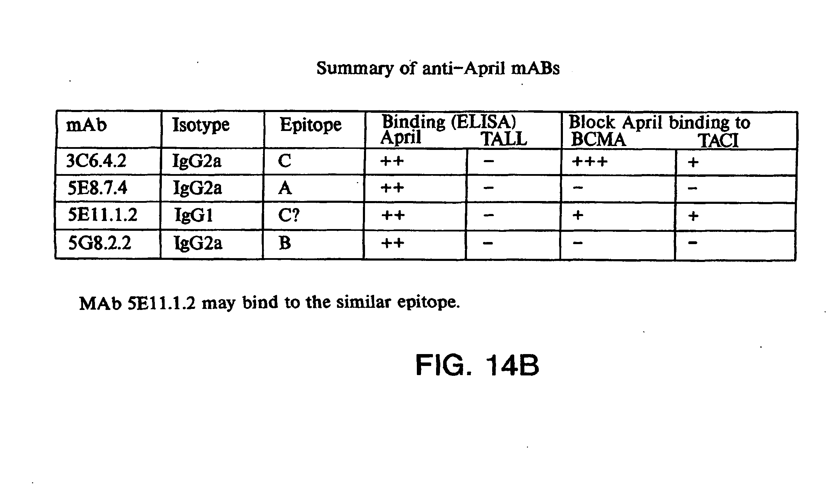

As shown in the Table in Figure 14B, the anti-APRIL antibodies, 5E8.7.4, 5G8.2.2, and 3C6.4.2, were found to be isotype IgG2a antibodies. Anti-APRIL antibody 5E11.1.2 was found to be an isotype IgG1 antibody.

### EXAMPLE 8

### Binding Assay Showing Blocking Activity of anti-APRIL mAbs

Microtiter plates (Nunc, Denmark) were coated with 50 µl/well goat anti-human Fc antibody (Boehringer Manheim) at 5 µg/ml in carbonate buffer overnight at 4° C. The plates were then blocked with 150 µl/well of 2% BSA in PBS buffer for 1 hour at room temperature. The respective immunoadhesins, BCMA-IgG or TACI-IgG (prepared as described in Example 2 above) were added in a 50 µl/well volume at 5 µg/ml in block buffer and incubated at room temperature for 1 hour. All antibodies (which were identified in the fusion described in Example 6) were diluted at 1:100 and 25 µl/well was added to the plate along with 25 µl/well of 2 µg/ml Flag-APRIL (see Example 2) and incubated for 1 hour at room temperature. The signal was developed with successive incubations with biotinylated anti-Flag antibody (Sigma Aldrich, Missouri) and streptavidin-horseradish peroxidase (Amersham Life Science, New Jersey). All steps except the first were preceeded with a wash step with PBS/0.01% Tween 20.

| Mab Identification | % Max Binding to BCMA | SD | % Max Binding to TACI | SD |
|---|---|---|---|---|
| 3C6.4.2 | 10 | 0 | 18 | 0 |
| 5E8.7.4 | 96 | 2 | 88 | 6 |
| 5E11.1.2 | 65 | 0 | 52 | 4 |
| 5G8.2.2 | 101 | 5 | 94 | 9 |
| IgG Ctrl | 100 | 0 | 100 | 0 |

As shown in the Table above, anti-APRIL antibody 3C6.4.2 effectively blocked the APRIL binding to BCMA and to TACI. In the assay, antibody 5E11.1.2 also showed partial blocking of APRIL to BCMA and to TACI.

### Example 9

### Competitive Binding ELISA

To determine whether the anti-APRIL antibodies, 3C6.4.2, 5E8.7.4, 5E11.1.2 and 5G8.2.2 (described in the Examples above) recognized the same or different epitopes, a competitive binding ELISA was performed as described in J. Immunol. Methods, 156:9-17 (1992) using biotinylated anti-APRIL antibodies. The anti-APRIL monoclonal antibodies were biotinylated using N-hydroxyl succinimide as described in J. Immunol. Methods, 156:9-17 (1992). Microtiter wells were coated with 50µl of 0.5µg/ml of Flag-APRIL (Example 2) in 50mM carbonate buffer, pH 9.6, overnight at 4° C. After washing, the nonspecific binding sites were blocked with 200µl of 2% BSA for 1 hour. After washing, a mixture of a predetermined optimal concentration of biotinylated anti-APRIL antibodies and a 100-fold excess of unlabeled monoclonal antibodies were added to each well. Following a 1 hour incubation at room temperature, plates were washed and the amount of biotinylated anti-APRIL antibody was detected by the addition of HRP-streptavidin. After washing the microtiter wells, the bound enzyme was detected by the addition of substrate, and the plates were read at 450nM with an ELISA plate reader.

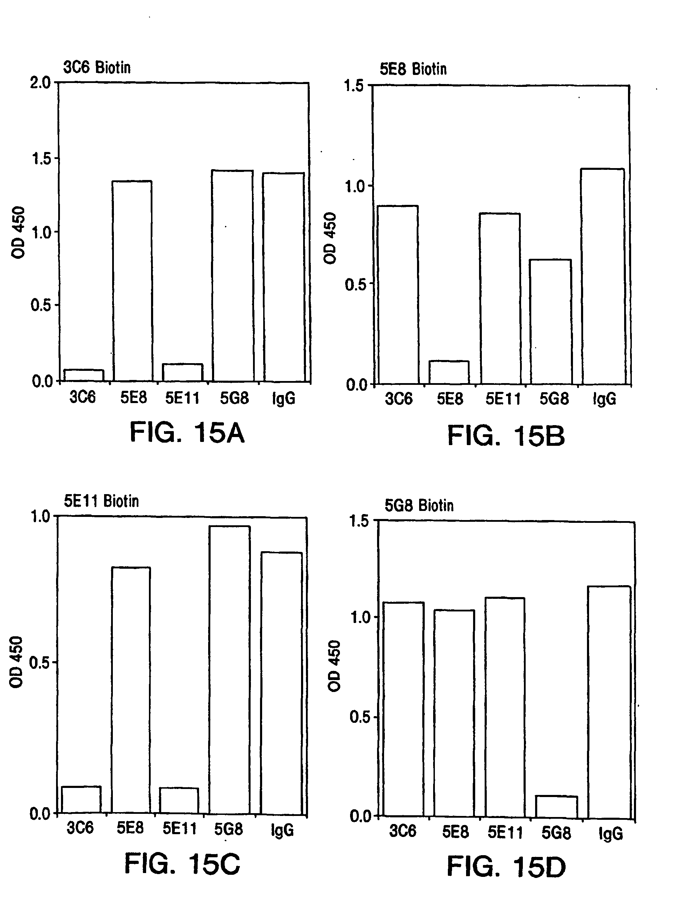

The results are shown in Figure 15. The data shows that antibody 3C6.4.2 and antibody 5E11.1.2 may recognize shared epitopes since unlabeled antibodies 3C6.4.2 or 5E11.1.2 were able to inhibit the binding of both biotinylated forms of the antibodies (Bio-3C6.4.2, Bio-5E11.1.2). Both antibodies 3C6.4.2 and 5E11.1.2 recognize different epitopes from those recognized by 5E8.7.4 and 5G8.2.2 since neither antibodies blocked the binding of Bio-5E8.7.4 and Bio-5G8.2.2. Further, antibodies 5E8.7.9 and 5G8.2.2 were able to block only its own biotinylated monoclonal antibody but not others. Accordingly, among the four monoclonal antibodies tested, it appears that three major epitopes on APRIL were detected.

### EXAMPLE 10

### Effects of TACI Immunoadhesin in Murine Arthritis Model

An *in vivo* assay in a collagen-induced arthritis (CIA) murine model was conducted to determine if inhibition of TACI interaction with its ligand(s) could prevent progression of CIA.

Rheumatoid arthritis (RA) is an autoimmune disease in which the synovial membrane of multiple joints can become inflamed, leading to destruction of joint tissues including bone and cartilage. The synovium of RA can be highly inflammatory in nature and is typically characterized by lymphocyte and mononuclear cell infiltration, T cell and antigen pressing cell (APC) activation, B cell immunoglobulin (Ig) secretion and pro-inflammatory cytokine production [Potocnik et al., Scand. J. Immunol., 31:213 (1990); Wernick et al., Arthritis Rheum., 28:742 (1985); Ridley et al., Br. J. Rheumatology, 29:84 (1990); Thomas et al., J. Immunol., 152:2613 (1994) ; Thomas et al., J. Immunol., 156:3074 (1996)]. Chronically inflamed synovium is usually accompanied by a massive CD4 T cell infiltration [Pitzalis et al., Eur. J. Immunol., 18:1397 (1988); Morimoto et al., Am. J. Med., 8,4:817 (1988)].

Collagen-induced arthritis (CIA) is an animal model for human RA, which resembles human disease, and can be induced in susceptible strains of mice by immunization with heterologous type-II collagen (CII) [Courtenay et al., Nature, 283:665 (1980); Cathcart et al., Lab. Invest., 54:26 (1986)]. Both CD4 T cells and antibodies to CII are required to develop CIA. Transfer of anti-CII to naive animals only leads to partial histo-pathology that is quite different from CIA, and complete symptoms of CIA do not develop [Holmdahl et al., Agents Action, 19:295 (1986)]. In contrast, adoptive transfer of both CD4 T cells and anti-CII antibodies from CII immunized mice to naive recipients completely reconstitutes the symptoms of classical CIA [Seki et al., J. Immunol., 148:3093 (1992)]. Involvement of both T cells and antibodies in CIA is also consistent with histo-pathological findings of inflamed joints in CIA. Thus, agents that block B cell or T cell functions, or inhibit pro-inflammatory cytokines induced by T cells, may be efficacious to prevent or treat arthritis. Indeed, depletion of CD4 T cells, blockade of CD40-CD40L interactions, neutralization of TNF-alpha or blocking of IL-1 receptors can all lead to prevention of CIA in mice [Maini et al., Immunol. Rev., 144:195 (1995); Joosten et al., Arthritis Rheum., 39:797 (1996); Durie et al., Science, 261:1328 (1993)].

In Applicants' study, two groups of mice (7 to 8 week old male DBA/1 mice (Jackson Laboratory)) were immunized intradermally with 100 µg bovine collagen type-II (BCII) (Sigma Chemical Co.) emulsified in complete Freund's adjuvant (CFA) (Difco). The mice were then rechallenged with BCII in incomplete Freund's adjuvant 21 days later. A dramatic disease with clinical signs of arthritis developed in the animals that progressed to a more severe form with time. Starting on day 24, one group of mice were injected with 100 µg of TACI-Fc three times per week intraperitoneally for six weeks (N=9), and a second group received 100 µg of murine IgG as a control (N=10). The TACI-Fc construct was prepared by using primers based on the human TACI sequence (described herein) to amplify the mouse TACI cDNA from a mouse spleen library. A PCR product of about 0.45 kb was cloned. A cDNA clone containing the complete open reading frame of mouse TACI was subsequently isolated from the same library (GenBank accession number AF257673). The murine TACI-Fc was constructed by cloning the extracellular domain of mouse TACI (amino acids 2-129) between a pro-trypsin signal sequence and mouse IgG1-Fc sequence and the immunoadhesion prepared as described in the Examples above. Animals were then monitored for the clinical signs of arthritis, and at the end of the study, as described below, a radiological and histo-pathological examination was performed.

Mice were examined daily for signs of joint inflammation and scored as follows: 0, normal; 1, erythema and mild swelling confined to the ankle joint; 2, erythema and mild swelling extending from the ankle to metatarsal/metacarpal joints; 3, erythema and moderate swelling extending from the ankle to the metatarsophalangeal/metacarpophalengeal joints; 4, erythema and severe swelling extending from the ankle to the digits. The maximal arthritic score per foot is 4 and the maximal disease score per mouse is 16; the mean arthritic score was calculated from all animals in the group.

For radiological analysis at the end of the study, both fore-and hind-paws were radio-graphed using X-ray Faxitron Imaging System (Faxitron X-ray Corp., Wheeling, IL). Data was digitized and pictures of radiographs were prepared. The radiographs were then examined for bone erosion and soft tissue swelling. For histo-pathological analysis, paws from the mice were excised, fixed in 10% formalin, decalcified, and embedded in paraffin. Joint sections (6 - 8 µm) were prepared and stained with hematoxylin and eosin using standard histochemical methods. Microscopic evaluation of arthritic paws was performed in a blinded fashion. Arthritic changes in the ankle, metacarpophalangeal/metatarsophalangeal, proximal interphalangeal, and joints were examined for articular cartilage and subchondral bone erosion.

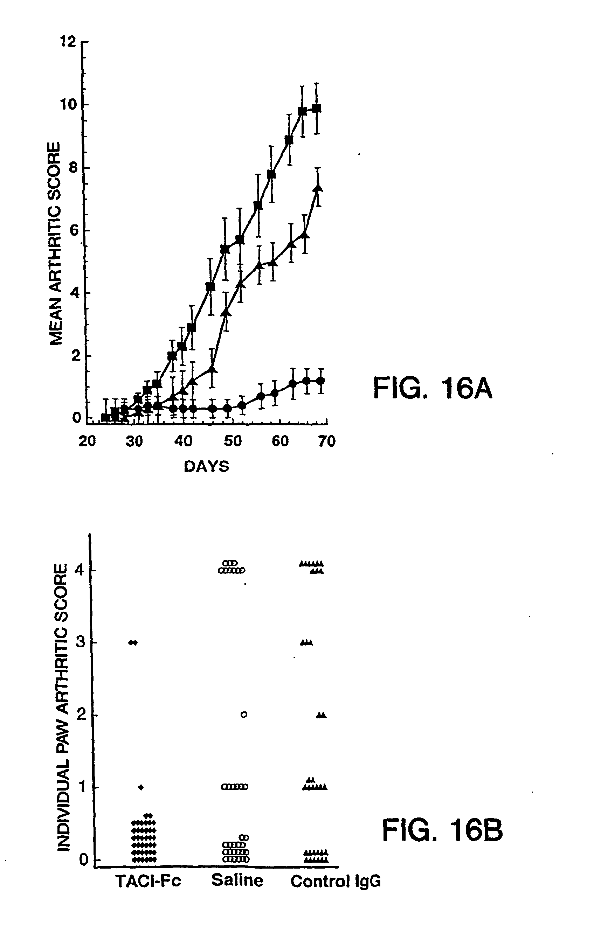

Fig. 16A illustrates the disease courses in TACI-Fc treated mice (circles), or control IgG treated mice mice (boxes) and saline treated mice (triangles). Each data point represents a mean ± SD from a total of 9 (for TACI-Fc treated group) or 10 (for control groups) mice. The differences between the TACI-Fc treated group and each of the two other groups are statistically significant. Mice in the control group developed typical clinical symptoms of arthritis, which started at about day 30 and progressed to very high arthritic scores rapidly (Fig. 16A). In contrast, in the mice treated with TACI-Fc, progression of arthritis was markedly inhibited. Fig. 16B shows the disease scores of individual feet 3 weeks after the second immunization. Each data point represents an individual foot. The differences between the control and TACI-Fc-treated groups are statistically significant. The arthritic scores in the TACI-Fc treated mice reached only to 1.0, and that happened only towards the end of study, whereas in the control group, the arthritic scores reached to >7.0. These data clearly demonstrate that TACI interaction with its ligand(s) is important for the development of CIA.

To determine if the TACI-Fc treatment of the mice had any effects on histo-pathology of the joints, at the end of the study his to-pathological examination of the paws of mice was performed. At the termination of the study (48 days after the second immunization), the mice were sacrificed and their ankle joints were analyzed (HE staining) for histology.

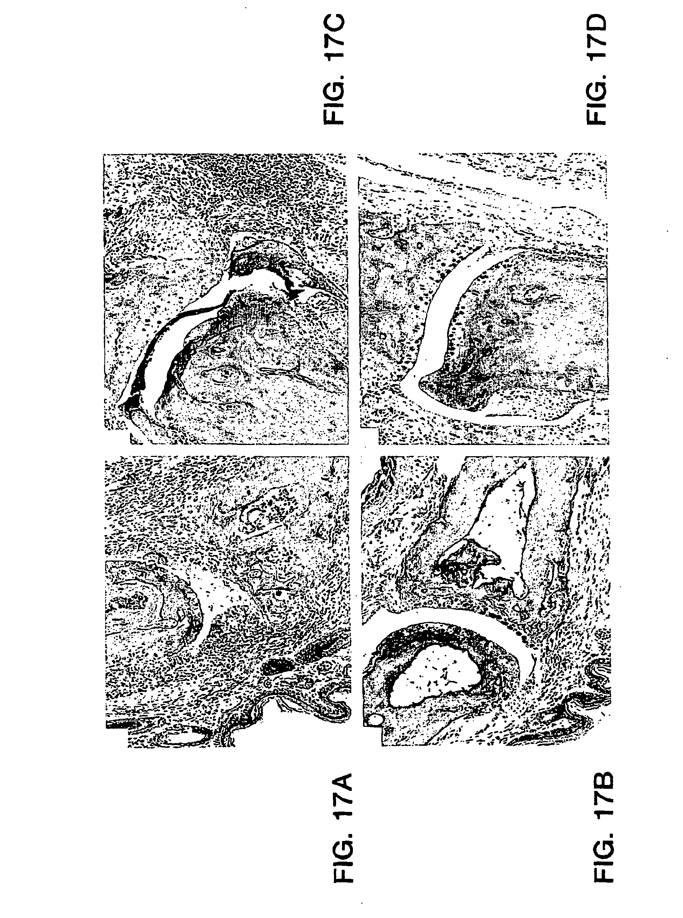

In the control group, a severe arthritic disease was characterized with synovial proliferation, massive leukocyte infiltration, pinnus formation that resulted in articular cartilage and bone erosion as shown in the proximal interphalangeal joint (Fig. 17A). Fig. 17A shows a phalangeal joint of a control mouse indicating severe synovitis, hyperplasia, and cartilage and bone destruction. Synovial thickening, leukocyte infiltration, articular cartilage degeneration and periarticular erosion was also seen in the metacarpal joint (Fig. 17B). Fig. 17B shows a phalangeal joint of a TACI-Fc-treated mouse with no signs of synovitis or disease pathology. In the TACI-Fc treated mice, there was no evidence of histo-pathological symptoms, indicating that TACI-Fc not only blocks clinical symptoms of CIA but also inhibits histo-pathological symptoms (Fig. 17C,D). Fig. 17C shows a metacarpal of a control mouse having disease pathology with massive signs of synovitis, and Fig. 17D shows a metacarpal joint of a TACI-Fc-treated mouse with no disease pathology.

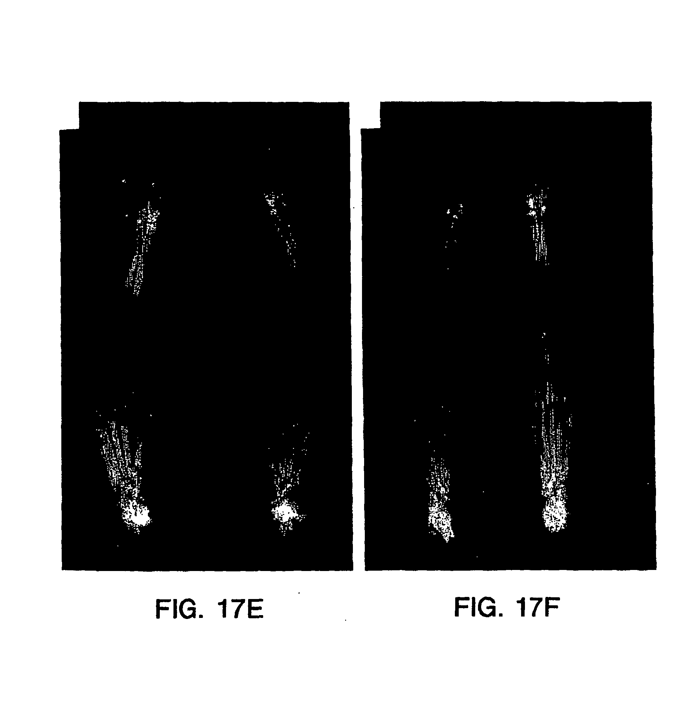

At the termination of the study, both fore- and hind- paws of the animals were also X-rayed and analyzed for bone structures as described above. In the control group, signs of massive bone destruction and disfiguration was apparent, whereas in the TACI-Fc treated mice, no significant signs of bone loss or disfiguration was seen (Fig. 17E,F). Fig. 17E shows a radiograph from a control mouse showing signs of massive bone destruction and disfiguration. Fig. 17F shows a radiograph from a TACI-Fc treated mouse showing no significant signs of bone loss or disfiguration. When radiographs from the TACI-Fc treated mice were compared with those from naïve mice; no apparent differences were revealed, indicating TACI-Fc treatment completely protected mice from bone and cartilage damage (Data not shown).

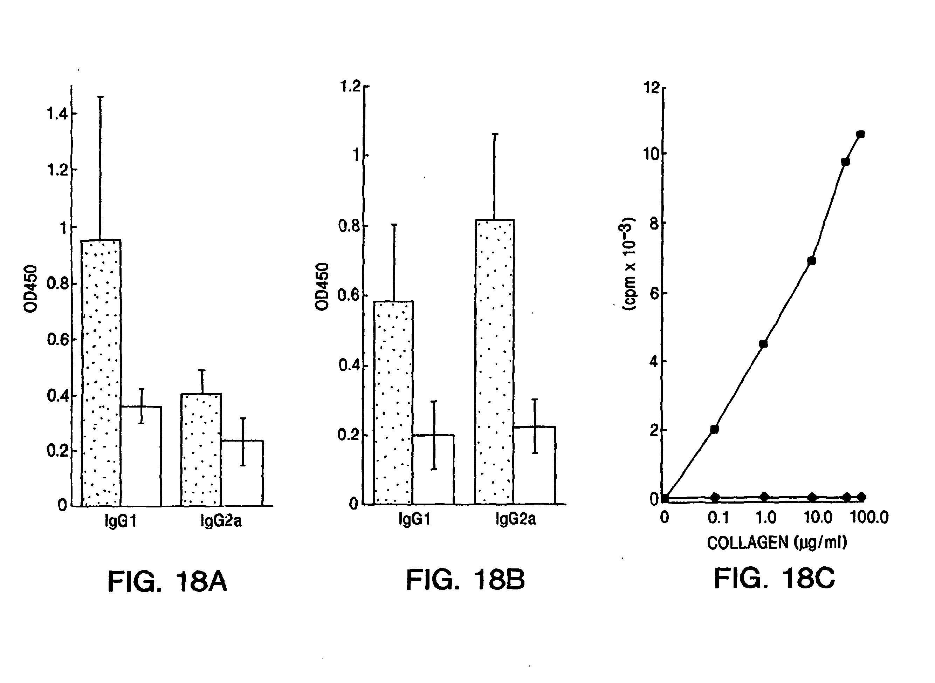

Since anti-collagen antibodies are believed to play an important role in the development of arthritis, serum samples of the mice were also analyzed to determine whether TACI-Fc treatment of the mice resulted in inhibition of an anti-collagen humoral immune response. To test humoral immune responses, the mice were bled retroorbitally 14 days (Fig. 18A) and 47 days (Fig. 18B) after the second immunization and analyzed for the presence of anti-collagen antibodies.

Serum levels of anti-BCII IgG1 and IgG2a isotypes were measured by an ELISA using BCII collagen as antigen. In brief, microtiter plates were coated with 10 µg/ml native bovine CII, blocked, and incubated with serially diluted test sera. Bound IgG was detected by incubation with alkaline phosphatase-conjugated goat anti-mouse IgG (Pharmingen), followed by substrate (dinitrophenyl phosphate). Optical densities were measured at 450 nm in an ELISA plate reader (Molecular Devices).

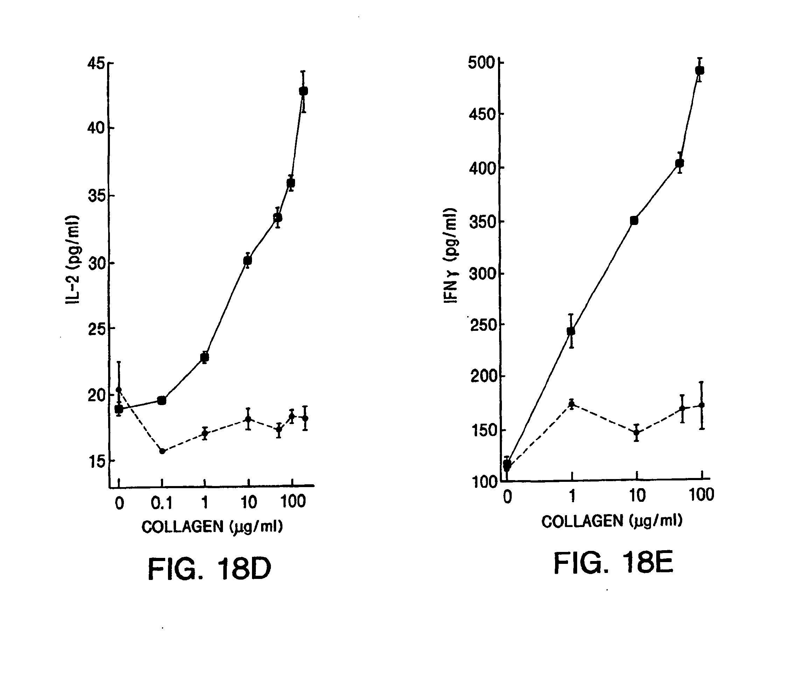

The results are shown in Fig. 18. Each data point represents a mean ± SD from five mice in each group. Serum from the mice in the control group showed presence of high levels of anti-collagen IgG1 and IgG2a, whereas in the TACI-Fc treated group, a considerable inhibition of both anti-collagen IgG1 and Ig2a was seen on days 14 and 47 after the second immunization. (Fig. 18A, B). Fig. 18A shows anticollagen IgG1 and IgG2a levels 14 days after the second immunization, and Fig. 18B shows anticollagen IgG1 and IgG2a levels 47 days after the second immunization (White bars, mice treated with BSA; black bars, mice treated with TACI-Fc). These results suggest that TACI-Fc treatment may attune the development of CIA, at least partially, by blocking anti-collagen antibodies.

Since both collagen-specific B and T cells can initiate CIA, an assay was further conducted to examine whether prevention of TACI-Fc mediated CIA was also associated with inhibition of T cell effector functions. Lymph nodes and spleens from both the control and TACI-Fc treated mice were collected at the end of the study and in vitro recall responses of T cells against collagen and production of effector cytokines was examined. BCII immunized mice were sacrificed 47 days after the second immunization, and their inguinal lymph nodes and spleen were collected. Single cell suspensions were prepared, and cells were cultured in 96-well plates at a density of 1 x 10⁶ cells/ml (200 µl/well) in DMEM containing 5% heat-inactivated FCS, 2 mM glutamine, 100 U/ml penicillin, 100 µg/ml streptomycin, and 2 x 10⁻⁵ M 2-ME. Cells were cultured in medium alone, or in the presence of various concentrations BCII. To test lymphocyte proliferation (Fig. 18C), lymph node cells (1 x 10⁶ cells per well) were first cultured for 72 hours, followed by addition of 1 µCi of [³H] thymidine (International Chemical and Nuclear, Irvine, CA) for the last 18 hours of a 5-day culture, and incorporation of radioactivity was assayed by liquid scintillation counting (represented as cpm) using a Wallac β-plate counter.

Proliferative T cell responses against collagen from TACI-Fc treated mice were almost negligible as compared to that of control mice (Fig. 18C; control mice -boxes; TACI-Fc treated mice- circles).

For the cytokine assays, the lymph node and splenic cells were cultured in 0.2 ml of medium with or without BCII; 1 x 10⁶ cells/ml (200 µl/well) were cultured in the above-mentioned medium alone, or in the presence BCII. Supernatants were collected after 24 hours to test for for IL-2 secretion and 72 hours later to test for for IFN-gamma production, which was found to be the optimal incubation time for cytokine determination, and stored at -20°C until analyzed. Levels of IL-2 and IFN-gamma were detected by ELISA using a kit from Pharmingen (San Deigo, California). Standard curves were generated using mouse recombinant IL-2 and IFN-gamma. When IL-2 and IFN-gamma production by T cells from these mice was measured, the TACI-Fc treated group (shown in Figs. 18D and 18E by circles) showed very little production of these cytokines, whereas T cells from the control group (shown in Figs. 18D and 18E by boxes) secreted significant levels of both IL-2 and IFN-gamma (Fig. 18D (IL-2 production), 18E (IFN-gamma production)).

These data suggest that TACI-Fc treatment of mice not only inhibited anti-collagen antibody production but also regulated functions of effector T cells. Thus, TACI interactions with its ligand(s) are also believed to be important in T cell mediated immune responses.

Since TACI receptor is also shown to be expressed on T cells and is involved in activation NF-AT associated with activation of T cells [von Bulow et al., Science, 278:138 (1997)], it is believed that blocking of TACI interaction with its ligand(s) may directly impair T cell activation and its effector functions that are required, for instance, for the progression of CIA in mice.

To determine the direct role of TACI in T cell activation, an in vitro assay of antigen-specific activation of T cells was performed. Activation of T cells by anti-CD3 antibody in vitro in the presence of TACI-Fc was examined by measuring proliferation and IL-2 production by these T cells. Splenic cells from adult C57BL/6 mice (Jackson Laboratory) were cultured (1 X 10⁶ per well) in various concentrations of 10 µg/ml anti-CD3 monoclonal antibody (Pharmingen) with or without different concentrations of TACI-Fc in medium as described above. Proliferation was measured by uptake of ³H-thymidine as stated above. Parallel assays were also set up to measure the effects of TACI-Fc on production of anti-CD3 antibody induced IL-2 production in a 24 hour culture system as mentioned above. An ELISA was used to determine IL-2 levels in supernatants, using antibodies from Pharmingen, and using their recommended protocols. To study the effects of TACI-Fc on *in vitro* stimulation of TCR transgenic cells, 1 X 10⁶ cells from adult MBP-TCR transgenic mice (bred from animal breeding pair obtained from Dr. Richard Flavell, Howard Hughes Medical Institute, Yale University) were cultured in the presence of 10 µg/ml MBP-Acl-11 (a synthetic NH2-terminal peptide of Myelin Basic Protein having amino acid sequence ASQKRPSQRSK (SEQ ID NO:10) with the first amino acid acetylated) with or without different concentrations of TACI-Fc in 96-well plates in DMEM medium supplemented with 5% FCS, 2 mM glutamine, 100 U/ml penicillin, 100 µg/ml streptomycin. Proliferation was measured by addition of 1 µCi of [³H] thymidine (International Chemical and Nuclear, Irvine, CA) for the last 18 hours of a 5-day culture, and incorporation of radioactivity was assayed by liquid scintillation counting.

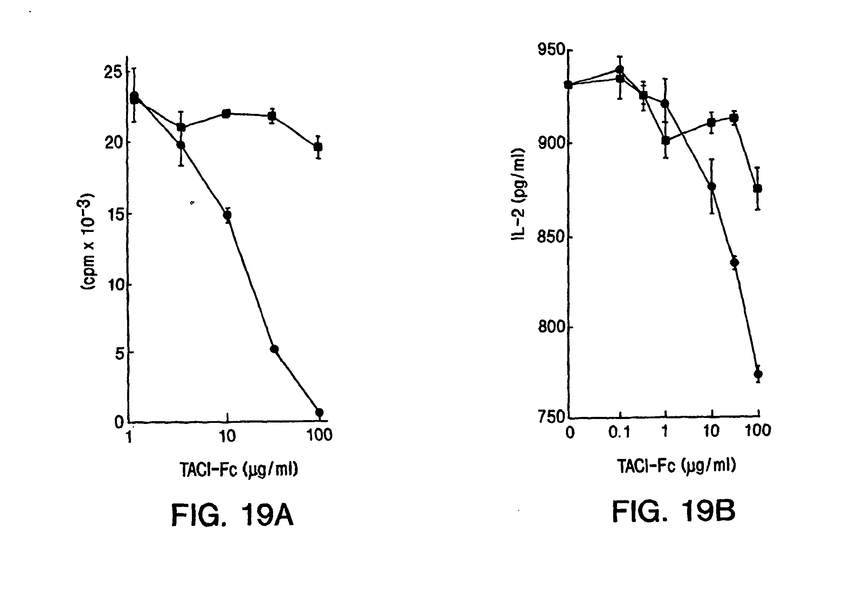

Fig. 19A shows the inhibition of anti-CD3 antibody-induced proliferation of naive T cells by TACI-Fc in a dose dependent manner, while Fig. 19B shows the inhibition of anti-CD3 antibody-induced IL-2 production by naive T cells, as affected by TACI-Fc in a dose dependent manner. (In Figs. 19A and 19B, TACI-Fc treatment is shown by circles; controls are shown by boxes).

Activation of myelin basic protein (MBP)-TCR transgenic T cells by antigen *in vitro* in the presence of TACI-Fc were also examined by measuring proliferation and IL-2 production by these T cells (as described above). Again, TACI-Fc inhibited both proliferation and IL-2 production by MBP-TCR transgenic T cells in a dose dependent manner (data not shown). These results demonstrate that TACI receptor is involved in T cell activation, and that this function can be blocked with TACI-Fc.

### EXAMPLE 11

### Effects of TACI Immunoadhesin in Murine EAE Model

The EAE murine model been described in the literature as a model for human multiple sclerosis [Grewal et al., Science, 273:1864-1867 (1996).

In Applicants' study, two groups of 10 mice each (10 to 15 week old male and female MBP-TCR transgenic mice (described in Example 10) were immunized subcutaneously with 10 µg MBP Acl-11 (described in Example 10 above) in 100 µl complete Freund's adjuvant (CFA) (Difco). Following the initial immunization with Acl-11, 200 ng Pertussis toxin (List Biologicals, Campbell, CA) in 100 µl saline was injected intraperitoneally in each mouse at 24 hours and 48 hours. Starting on day 2 through day 24, one group of mice was injected with 100 µg of TACI-Fc (described in Example 10) in 100 µl sterile saline 5 intraperitoneally daily, and a second group received 100 µg of murine IgG in 100 µl sterile saline intraperitoneally each day. Animals were then monitored daily for the onset of disease. Clinical signs of experimental allergic encephalomyelitis (EAE) were assessed daily and a score of 1 to 5 was given to each mouse based on the established EAE index system: 0= normal appearance; 1= tail droop; 2= abnormal gait; 3= limb weakness; 4= paralysis involving one limb (partial hindlimb paralysis); 5= paralysis involving two limbs (total hindlimb paralysis). This is a modified scoring system from that previously described in Grewal et al., 273:1864-1867 (1996).

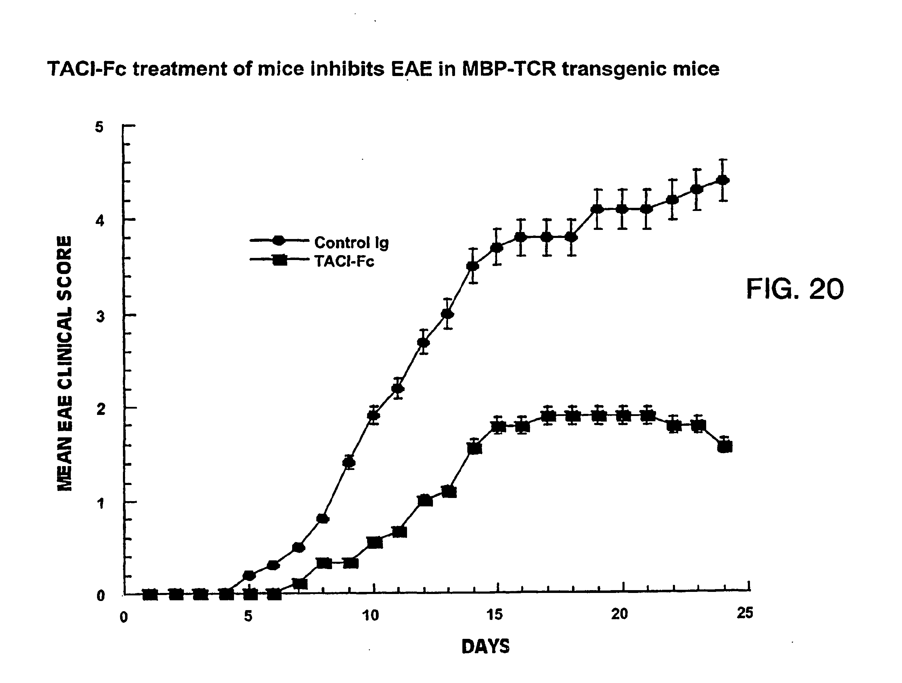

The results are shown in Figure 20. The data shown in Figure 20 indicate that animals receiving the control IgG developed expected clinical symptoms of EAE; the disease onset in the control treated mice started on day 5 and reached the peak levels within 10 days. In contrast, the TACI-Ig treated mice did not develop severe forms of the EAE symptoms. The disease score was much lower than the control group, only reaching clinical scores of 2 (which did not progress to higher scores during the study). The results thus suggest that the TACI-Ig treatment protected the mice from developing overt EAE.

### Deposit of Material

The following materials have been deposited with the American Type Culture Collection, 10801 University Blvd., Manassas, VA 20110-2209, USA (ATCC):

| Material | ATCC Dep. No. | Deposit Date |
|---|---|---|
| 3C6.4.2 | PTA-1347 | Feb. 15, 2000 |
| 5E11.1.2 | PTA-1346 | Feb. 15, 2000 |
| 5G8.2.2 | PTA-1345 | Feb. 15, 2000 |
| 5E8.7.4 | PTA-1344 | Feb. 15, 2000 |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC '122 and the Commissioner's rules pursuant thereto (including 37 CFR '1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written description is considered to be sufficient to enable one skilled in the art to practice the invention. The present invention is not to be limited in scope by the example presented herein. Indeed, various modifications of the invention in addition to those shown and described herein will become apparent to those skilled in the art from the foregoing description.

## Claims

1. An APRIL polypeptide antagonist which is a monoclonal anti-APRIL. antibody wherein APRIL polypeptide comprises the amino acid sequence shown in SEQ ID NO: 8 or a fragment thereof, wherein said APRIL antagonist blocks, inhibits or neutralizes the binding of APRIL polypeptide to TACI receptor or both TACI and BCMA receptors for use in a method of treatment of an immune related disease or cancer, wherein said immune related disease or cancer is rheumatoid arthritis, multiple sclerosis, psoriasis, Sjogren's syndrome or lupus, or leukaemia, lymphoma or myeloma.

2. Use of an APRIL polypeptide antagonist which is a monoclonal anti-APRIL antibody wherein APRIL polypeptide comprises the amino acid sequence shown in SEQ ID NO: 8 or a fragment thereof, wherein said APRIL antagonist blocks, inhibits or neutralizes the binding of APRIL polypeptide to TACI receptor or both TACI and BCMA receptors in the preparation of a medicament for the treatment of an immune related disease or cancer, wherein said immune related disease or cancer is rheumatoid arthritis, multiple sclerosis, psoriasis, Sjogren's syndrome or lupus, or leukaemia, lymphoma or myeloma.

3. The APRIL polypeptide antagonist for use of claim 1 or the use of claim 2, wherein the monoclonal antibody is a chimeric antibody, a humanized antibody or a human antibody.

4. The APRIL polypeptide antagonist for use or the use of claim 3, wherein the anti-APRIL antibody is a humanized antibody that comprises a sequence from the 3C6.4.2 antibody secreted by the hybridoma deposited with ATCC as accession number PTA-1347 or comprises a sequence from the 5E11.1.2 antibody secreted by the hybridoma deposited with ATCC as accession number PTA-1346.

5. The APRIL polypeptide antagonist for use of claim 1 or the use of claim 2, wherein the antibody is monoclonal antibody 3C6.4.2 deposited as ATCC accession number PTA-1347 or monoclonal antibody 5E11.1.2 deposited as ATCC accession number PTA-1346.

6. The APRIL polypeptide antagonist for use or the use of any one of the preceding claims, wherein APRIL polypeptide has the amino acid sequence shown in SEQ ID NO: 8, TACI receptor has the amino acid sequence shown in SEQ ID NO: 2, and BCMA receptor has the amino acid sequence shown in SEQ ID NO: 4.

7. The APRIL polypeptide antagonist for use or the use of any one of the preceding claims, wherein the cancer is Burkitt's lymphoma, multiple myeloma, acute lymphoblastic or lymphocytic leukaemia, non-Hodgkin's lymphoma, Hodgkin's lymphoma, or acute myeloid leukaemia.

8. The APRIL polypeptide antagonist for use or the use of any one of the preceding claims, wherein the APRIL polypeptide antagonist or the medicament is for administration with one or more cytotoxic agents, chemotherapeutic agents, cytokines, growth inhibitory agents, antibodies, or radiation therapy.

9. The APRIL polypeptide antagonist for use or the use of claim 8, wherein the antibody is an antibody that binds to an antigen selected from the group consisting of CD20, CD11a, CD18, CD40, ErbB2, EGFR, ErbB3, ErbB4, and VEGF.

10. The APRIL polypeptide antagonist for use or the use of claim 9, wherein the antibody that binds CD20 is rituximab.

11. The APRIL polypeptide antagonist for use or the use of claim 9, wherein the antibody that binds ErbB2 is trastuzumab.

12. A composition comprising an APRIL polypeptide antagonist which is a monoclonal anti-APRIL antibody wherein APRIL polypeptide comprises the amino acid sequence shown in SEQ ID NO: 8 or a fragment thereof, which blocks, inhibits or neutralizes the binding of APRIL polypeptide to TACI receptor or both TACI and BCMA receptors and a carrier.

13. The composition of claim 12, wherein the antibody is a chimeric antibody, a humanized antibody or a human antibody.

14. The composition of claim 12, wherein the antibody is a humanized antibody that comprises a sequence from the 3C6.4.2 antibody secreted by the hybridoma deposited with ATCC as accession number PTA-1347 or comprises a sequence from the 5E11.1.2 antibody secreted by the hybridoma deposited with ATCC as accession number PTA-1346.

15. The composition of claim 12, wherein the antibody is monoclonal antibody 3C6.4.2 deposited as ATCC accession number PTA-1347 or monoclonal antibody 5E11.1.2 deposited as ATCC accession number PTA-1346.

16. The composition of any one of claims 12 to 15, wherein the carrier is a pharmaceutically acceptable carrier.

17. An APRIL polypeptide antagonist which is a monoclonal anti-APRIL antibody wherein APRIL polypeptide comprises the amino acid sequence shown in SEQ ID NO: 8 or a fragment thereof, which blocks, inhibits or neutralizes the binding of APRIL polypeptide to TACI receptor or both TACI and BCMA receptors.

18. A method for identifying an antagonist or agonist of APRIL polypeptide interaction with TACI receptor or both TACI and BCMA receptors, comprising contacting a candidate compound with said APRIL polypeptide or a TACI receptor or both TACI and BCMA receptors, and detecting interaction of said components.

19. The method of claim 18, wherein the candidate compound binds to, or complexes with, an APRIL polypeptide, or TACI or BCMA receptor, or otherwise interferes with the interaction of these polypeptides with other cellular proteins.

20. An in vitro method of inhibiting or neutralizing an APRIL polypeptide biological activity in a mammalian cell, comprising exposing said cell to an effective amount of a monoclonal anti-APRIL antibody as defined in any one of claims 12 to 15, wherein the biological activity is (i) binding of APRIL polypeptide to TACI receptor, or (ii) binding of APRIL polypeptide to TACI and BCMA receptors.

21. The method of any one of claims 18 to 20, wherein the APRIL polypeptide, TACI receptor, or BCMA receptor is recombinant.

22. The method of any one of claims 18 to 20, wherein APRIL polypeptide has the amino acid sequence shown in SEQ ID NO: 8, TACI receptor has the amino acid sequence shown in SEQ ID NO: 2, and BCMA receptor has the amino acid sequence shown in SEQ ID NO: 4.

## Patentansprüche

1. APRIL-Polypeptid-Antagonist, der ein monoklonaler Anti-APRIL-Antikörper ist, worin APRIL-Polypeptid die in Seq.-ID Nr. 8 angeführte Aminosäuresequenz oder ein Fragment davon umfasst, worin der APRIL-Antagonist die Bindung von APRIL-Polypeptid an den TACI-Rezeptor oder an den TACI- und den BCMA-Rezeptor blockiert, hemmt oder neutralisiert, zur Verwendung in einem Verfahren zur Behandlung einer Immunerkrankung oder von Krebs, worin die Immunerkrankung bzw. der Krebs rheumatoide Arthritis, multiple Sklerose, Psoriasis, das Sjögren-Syndrom oder Lupus erythematodes oder Leukämie, ein Lymphom oder ein Mylom ist.

2. Verwendung eines APRIL-Polypeptid-Antagonisten, der ein monoklonaler Anti-APRIL-Antikörper ist, worin APRIL-Polypeptid die in Seq.-ID Nr. 8 angeführte Aminosäuresequenz oder ein Fragment davon umfasst, worin der APRIL-Antagonist die Bindung von APRIL-Polypeptid an den TACI-Rezeptor oder an den TACI- und den BCMA-Rezeptor blockiert, hemmt oder neutralisiert, zur Herstellung eines Medikaments zur Behandlung einer Immunerkrankung oder von Krebs, worin die Immunerkrankung bzw. der Krebs rheumatoide Arthritis, multiple Sklerose, Psoriasis, das Sjögren-Syndrom oder Lupus erythematodes oder Leukämie, ein Lymphom oder ein Mylom ist.

3. APRIL-Polypeptid-Antagonist zur Verwendung nach Anspruch 1 oder Verwendung nach Anspruch 2, worin der monoklonale Antikörper ein chimärer Antikörper, ein humanisierter Antikörper oder ein menschlicher Antikörper ist.

4. APRIL-Polypeptid-Antagonist zur Verwendung oder Verwendung nach Anspruch 3, worin der Anti-APRIL-Antikörper ein humanisierter Antikörper ist, der eine Sequenz des 3C6.4.2-Antikörpers umfasst, der von dem bei der ATTCC unter der Zugriffsnummer PTA-1347 hinterlegten Hybridom sekretiert wird, oder eine Sequenz des 5E.11.1.2-Antikörpers umfasst, der von dem bei der ATCC unter der Zugriffsnummer PTA-1346 hinterlegten Hybridom sekretiert wird.

5. APRIL-Polypeptid-Antagonist zur Verwendung nach Anspruch 1 oder Verwendung nach Anspruch 2, worin der Antikörper der monoklonale Antikörper 3C6.4.2, der unter der ATCC-Zugriffsnr. PTA-1347 hinterlegt ist, oder der monoklonale Antikörper 5E11.1.2 ist, der unter der ATCC-Zugriffsnr. PTA-1346 hinterlegt ist.

6. APRIL-Polypeptid-Antagonist zur Verwendung oder Verwendung nach einem der vorangegangenen Ansprüche, worin das APRIL-Polypeptid die in Seq.-ID Nr. 8 angeführte Aminosäuresequenz aufweist, der TACI-Rezeptor die in Seq.-ID Nr. 2 angeführte Aminosäuresequenz aufweist und der BCMA-Rezeptor die in Seq.-ID Nr. 4 angeführte Aminosäuresequenz aufweist.

7. APRIL-Polypeptid-Antagonist zur Verwendung oder Verwendung nach einem der vorangegangenen Ansprüche, worin der Krebs ein Burkitt-Lymphom, ein multiples Myelom, akute Lymphoblasten oder Lymphozytenleukämie, ein Non-Hodgkin-Lymphom, ein Hodgkin-Lymphom oder akute myeloische Leukämie ist.

8. APRIL-Polypeptid-Antagonist zur Verwendung oder Verwendung nach einem der vorangegangenen Ansprüche, worin der APRIL-Polypeptid-Antagonist oder das Medikament zur Verabreichung mit einem oder mehreren zytotoxischen Mitteln, chemotherapeutischen Mitteln, Zytokinen, wachstumshemmenden Mitteln, Antikörpern oder in Verbindung mit Strahlentherapie dient.

9. APRIL-Polypeptid-Antagonist zur Verwendung oder Verwendung nach Anspruch 8, worin der Antikörper ein Antikörper ist, der an ein Antigen bindet, das aus der aus CD20, CD11a, CD18, CD40,ErbB2, ECGF, ErbB3, ErbB4 und VEGF bestehenden Gruppe ausgewählt ist.

10. APRIL-Polypeptid-Antagonist zur Verwendung oder Verwendung nach Anspruch 9, worin der Antikörper, der CD20 bindet, Rituximab ist.

11. APRIL-Polypeptid-Antagonist zur Verwendung oder Verwendung nach Anspruch 9, worin der Antikörper, der ErbB2 bindet, Trastuzumab ist.

12. Zusammensetzung, die einen APRIL-Polypeptid-Antagonisten, der ein monoklonaler Anti-APRIL-Antikörper ist, worin das APRIL-Polypeptid die in Seq.-ID Nr. 8 angeführte Aminosäuresequenz oder ein Fragment davon umfasst, und der die Bindung von APRIL-Polypeptid an den TACI-Rezeptor oder an den TACI-Rezeptor und den BCMA-Rezeptor blockiert, hemmt oder neutralisiert, sowie einen Träger umfasst.

13. Zusammensetzung nach Anspruch 12, worin der Antikörper ein chimärer Antikörper, ein humanisierter Antikörper oder ein menschlicher Antikörper ist.

14. Zusammensetzung nach Anspruch 12, worin der Antikörper ein humanisierter Antikörper ist, der eine Sequenz des 3C6.4.2-Antikörpers umfasst, der von dem bei der ATCC unter der Zugriffsnummer PTA-1347 hinterlegten Hybridom sekretiert wird, oder eine Sequenz des 5E.11.1.2-Antikörper umfasst, der von dem bei der ATCC unter der Zugriffsnummer PTA-1346 hinterlegten Hybridom sekretiert wird.

15. Zusammensetzung nach Anspruch 12, worin der Antikörper der monoklonale Antikörper 3C6.4.2, der unter der ATCC-Zugriffsnr. PTA-1347 hinterlegt ist, oder der monoklonale Antikörper 5E11.1.2 ist, der unter der ATCC-Zugriffsnr. PTA-1346 hinterlegt ist.

16. Zusammensetzung nach einem der Ansprüche 12 bis 15, worin der Träger ein pharmazeutisch annehmbarer Träger ist.

17. APRIL-Polypeptid-Antagonist, der ein monoklonaler Anti-APRIL-Antikörper ist, worin das APRIL-Polypeptid die in Seq.-ID Nr. 8 angeführte Aminosäuresequenz oder ein Fragment davon umfasst, und der die Bindung von APRIL-Polypeptid an den TACI-Rezeptor oder an den TACI-Rezeptor und den BCMA-Rezeptor blockiert, hemmt oder neutralisiert.

18. Verfahren zur Identifizierung eines Antagonisten oder Agonisten der Wechselwirkung von APRIL-Polypeptid mit dem TACI-Rezeptor oder dem TACI-Rezeptor und dem BCMA-Rezeptor, das das Kontaktieren einer Kandidatenverbindung mit dem APRIL-Polypeptid oder dem TACI-Rezeptor oder dem TACI- und dem BCMA-Rezeptor und das Detektieren der Wechselwirkung der Komponenten umfasst.

19. Verfahren nach Anspruch 18, worin die Kandidatenverbindung an APRIL-Polypeptid oder den TACI-Rezeptor oder den BCMA-Rezeptor bindet oder damit Komplexe bildet oder die Wechselwirkung dieser Polypeptide mit anderen Zellproteinen anderweitig stört.

20. In-vitro-Verfahren zur Hemmung oder Neutralisierung einer biologischen Aktivität von APRIL-Polypeptid in einer Säugetierzelle, das das Aussetzen der Zelle gegenüber einer wirksamen Menge eines monoklonalen Anti-APRIL-Antikörpers, wie in einem der Ansprüche 12 bis 15 definiert, umfasst, worin es sich bei der biologischen Aktivität um (i) das Binden von APRIL-Polypeptid an den TACI-Rezeptor oder (ii) das Binden von APRIL-Polypeptid an den TACI- und den BCMA-Rezeptor handelt.

21. Verfahren nach einem der Ansprüche 18 bis 20, worin das APRIL-Polypeptid, der TACI-Rezeptor oder der BCMA-Rezeptor rekombinant ist.

22. Verfahren nach einem der Ansprüche 18 bis 20, worin das APRIL-Polypeptid die in Seq.-ID Nr. 8 angeführte Aminosäuresequenz aufweist, der TACI-Rezeptor die in Seq.-ID Nr. 2 angeführte Aminosäuresequenz aufweist und der BCMA-Rezeptor die in Seq.-ID Nr. 4 angeführte Aminosäuresequenz aufweist.

## Revendications

1. Antagoniste du polypeptide APRIL qui est un anticorps monoclonal anti-APRIL, ledit polypeptide APRIL comprenant la séquence d'amino-acides représentée dans la SEQ ID NO:8 ou un de ses fragments, ledit antagoniste d'APRIL bloquant, inhibant ou neutralisant la liaison du polypeptide APRIL au récepteur TACI ou bien à la fois au récepteur TACI et au récepteur BCMA, destiné à être utilisé dans une méthode de traitement d'une maladie en rapport avec l'immunité ou d'un cancer, où ladite maladie en rapport avec l'immunité ou ledit cancer est la polyarthrite rhumatoïde, la sclérose en plaques, le psoriasis, le syndrome de Sjögren ou le lupus, une leucémie, un lymphome ou un myélome.

2. Utilisation d'un antagoniste du polypeptide APRIL qui est un anticorps monoclonal anti-APRIL, ledit polypeptide APRIL comprenant la séquence d'amino-acides représentée dans la SEQ ID NO:8 ou un de ses fragments, ledit antagoniste d'APRIL bloquant, inhibant ou neutralisant la liaison du polypeptide APRIL au récepteur TACI ou bien à la fois au récepteur TACI et au récepteur BCMA, dans la préparation d'un médicament destiné au traitement d'une maladie en rapport avec l'immunité ou d'un cancer, dans laquelle ladite maladie en rapport avec l'immunité ou ledit cancer est la polyarthrite rhumatoïde, la sclérose en plaques, le psoriasis, le syndrome de Sjögren ou le lupus, ou une leucémie, un lymphome ou un myélome.

3. Antagoniste du polypeptide APRIL destiné à être utilisé suivant la revendication 1 ou utilisation suivant la revendication 2, où l'anticorps monoclonal est un anticorps chimère, un anticorps humanisé ou un anticorps humain.

4. Antagoniste du polypeptide APRIL destiné à être utilisé ou utilisation suivant la revendication 3, où l'anticorps anti-APRIL est un anticorps humanisé qui comprend une séquence de l'anticorps 3C6.4.2 sécrété par l'hybridome déposé dans l'ATCC sous le numéro de dépôt PTA-1347 ou bien comprend une séquence de l'anticorps 5E11.1.2 sécrété par l'hybridome déposé dans l'ATCC sous le numéro de dépôt PTA-1346.

5. Antagoniste du polypeptide APRIL destiné à être utilisé suivant la revendication 1 ou utilisation suivant la revendication 2, où l'anticorps est l'anticorps monoclonal 3C6.4.2 déposé sous le numéro de dépôt ATCC PTA-1347 ou l'anticorps monoclonal 5E11.1.2 déposé sous le numéro de dépôt ATCC PTA-1346.

6. Antagoniste du polypeptide APRIL destiné à être utilisé ou utilisation suivant l'une quelconque des revendications précédentes, ledit polypeptide APRIL ayant la séquence d'amino-acides représentée dans la SEQ ID NO:8, le récepteur TACI ayant la séquence d'amino-acides représentée dans la SEQ ID NO:2 et le récepteur BCMA ayant la séquence d'amino-acides représentée dans la SEQ ID NO:4.

7. Antagoniste du polypeptide APRIL destiné à être utilisé ou utilisation suivant l'une quelconque des revendications précédentes, où le cancer est le lymphome de Burkitt, le myélome multiple, la leucémie aiguë, lymphoblastique ou lymphocytaire, le lymphome non hodgkinien, le lymphome de Hodgkin ou la leucémie myéloïde aiguë.

8. Antagoniste du polypeptide APRIL destiné à être utilisé ou utilisation suivant l'une quelconque des revendications précédentes, ledit antagoniste du polypeptide APRIL ou ledit médicament étant destiné à l'administration avec un ou plusieurs agents cytotoxiques ou agents chimiothérapeutiques, une ou plusieurs cytokines, un ou plusieurs agents inhibiteurs de croissance ou anticorps, ou bien une radiothérapie.

9. Antagoniste du polypeptide APRIL destiné à être utilisé ou utilisation suivant la revendication 8, l'anticorps étant un anticorps qui se lie à un antigène choisi dans le groupe consistant en CD20, CD11a, CD18, CD40, ErbB2, EGFR, ErbB3, ErbB4 et VEGF.

10. Antagoniste du polypeptide APRIL destiné à être utilisé ou utilisation suivant la revendication 9, l'anticorps qui se lie à CD20 étant le rituximab.

11. Antagoniste du polypeptide APRIL destiné à être utilisé ou utilisation suivant la revendication 9, l'anticorps qui se lie à Erb2 étant le trastuzumab.

12. Composition comprenant l'antagoniste du polypeptide APRIL qui est un anticorps monoclonal anti-APRIL, dans laquelle le polypeptide APRIL comprend la séquence d'amino-acides représentée dans la SEQ ID NO:8 ou un de ses fragments, qui bloque, inhibe ou neutralise la liaison du polypeptide APRIL au récepteur TACI ou bien à la fois au récepteur TACI ou au récepteur BCMA, et un support.

13. Composition suivant la revendication 12, dans laquelle l'anticorps est un anticorps chimère, un anticorps humanisé ou un anticorps humain.

14. Composition suivant la revendication 12, dans laquelle l'anticorps est un anticorps humanisé qui comprend une séquence de l'anticorps 3C6.4.2 sécrété par l'hybridome déposé dans l'ATCC sous le numéro de dépôt PTA-1347 ou comprend une séquence de l'anticorps 5E11.1.2 sécrété par l'hydridome déposé dans l'ATCC sous le numéro de dépôt PTA-1346.

15. Composition suivant la revendication 12, dans laquelle l'anticorps est l'anticorps monoclonal 3C6.4.2 déposé sous le numéro de dépôt ATCC PTA-1347 ou l'anticorps monoclonal 5E11.1.2 déposé sous le numéro de dépôt ATCC PTA-1346.

16. Composition suivant l'une quelconque des revendications 12 à 15, dans laquelle le support est un support pharmaceutiquement acceptable.

17. Antagoniste du polypeptide APRIL qui est un anticorps monoclonal anti-APRIL, ledit polypeptide APRIL comprenant la séquence d'amino-acides représentée dans la SEQ ID NO:8 ou un de ses fragments, qui bloque, inhibe ou neutralise la liaison du polypeptide APRIL au récepteur TACI ou bien à la fois au récepteur TACI et au récepteur BCMA.

18. Méthode pour identifier un antagoniste ou agoniste de l'interaction du polypeptide APRIL avec le récepteur TACI ou bien à la fois avec le récepteur TACI et le récepteur BCMA, comprenant la mise en contact d'un composé candidat avec ledit polypeptide APRIL ou un récepteur TACI ou bien à la fois un récepteur TACI et un récepteur BCMA, et la détection de l'interaction desdits constituants.

19. Méthode suivant la revendication 18, dans laquelle le composé candidat se lie à, ou se complexe avec, un polypeptide APRIL, ou un récepteur TACI ou BCMA, ou bien interfère avec l'interaction de ces polypeptides avec d'autres protéines cellulaires.

20. Méthode in vitro d'inhibition ou de neutralisation d'une activité biologique d'un polypeptide APRIL dans une cellule de mammifère, comprenant l'exposition de ladite cellule à une quantité efficace d'un anticorps monoclonal anti-APRIL répondant à la définition figurant dans l'une quelconque des revendications 12 à 15, dans laquelle l'activité biologique est (i) la liaison du polypeptide APRIL au récepteur TACI ou (ii) la liaison du polypeptide APRIL aux récepteurs TACI et BCMA.

21. Méthode suivant l'une quelconque des revendications 18 à 20, dans laquelle le polypeptide APRIL, le récepteur TACI ou le récepteur BCMA est recombinant.

22. Méthode suivant l'une quelconque des revendications 18 à 20, dans laquelle le polypeptide APRIL a la séquence d'amino-acides représentée dans la SEQ ID NO:8, le récepteur TACI a la séquence d'amino-acides représentée dans la SEQ ID NO:2 et le récepteur BCMA a la séquence d'amino-acides représentée dans la SEQ ID NO:4.
